(19) Europäisches Patentamt
European Patent Office
Office européen des brevets

(11) **EP 3 739 065 A1**

(12) **EUROPEAN PATENT APPLICATION**

(43) Date of publication:
**18.11.2020 Bulletin 2020/47**

(51) Int Cl.:
*C12Q 1/6869* (2018.01)    *C12Q 1/6886* (2018.01)
*G16B 20/20* (2019.01)    *G16B 40/00* (2019.01)

(21) Application number: **19382385.3**

(22) Date of filing: **16.05.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Fundació Centre de Regulació
Genòmica
08003 Barcelona (ES)**

(72) Inventors:
• **GUT, Ivo Glynne
08003 Barcelona (ES)**

• **STOBBE, Miranda Danielle
08003 Barcelona (ES)**
• **THUN, Gian-Andri
08003 Barcelona (ES)**

(74) Representative: **Mewburn Ellis LLP
Aurora Building
Counterslip
Bristol BS1 6BX (GB)**

Remarks:
Claims 16 to 21 are deemed to be abandoned due to non-payment of the claims fees (Rule 45(3) EPC).

(54) **SOMATIC MUTATION-BASED CLASSIFICATION OF CANCERS**

(57)    The present invention provides a computer-implemented method for classifying a test sample obtained from a tumour of a test subject, comprising: (a) providing a whole genome sequence of the tumour and of a non-tumour sample from the test subject; (b) analysing the genome sequence of the tumour to compute at least five general features and at least five recurrence features selected from the general and recurrence features shown in Table SF2-1;
(c) providing at least five reference clusters that have been obtained by: (i) analysing a training data set comprising tumour genomes of at least 1000 subjects to compute said at least five general features and at least five recurrence features defined in step (b) for each of said at least 1000 subjects; (ii) performing principal component analysis (PCA) using the features of step (i) as input, optionally after scaling and/or centring the features to account for their different scales, to obtain a plurality of principal components that together account for at least 50% of the variance of the training data set; and (iii) performing hierarchical clustering using said plurality of principal components to divide the samples of the training data set into at least five reference clusters, each reference cluster having a centre that is defined by computing the mean of the plurality of principal components of all samples in that cluster; (d) determining the Euclidean distance between said plurality of principal components of the test sample and the centre of each of said reference clusters; and (e) classifying the tumour sample obtained from the test subject as belonging to the reference cluster to which it is found to be nearest in step (d). Also provided a related methods and systems.

Figure 7

**Description**

**Field of the invention**

The present invention relates in part to methods for classifying and grouping cancers based on analysis of somatic mutations, including recurrent somatic mutations. The classification into particular clusters provides actionable clinical phenotypes for the management and treatment of cancer patients.

**Background to the invention**

Mutational processes induced by exogenous sources and/or endogenous mechanisms determine the mutational burden of a cell. They each leave their own genomic fingerprint that differs in terms of the number, types and distribution of mutations. Cancer cells usually show higher mutation rates than normal cells due to elevated cell proliferation and lack of proper DNA repair. The mutations accumulated before, during and after the oncogenic transformation may result in a mutational load exceeding several thousand per cancer genome (1). Even with such a high burden, the sheer size of the human genome with over three billion bp still makes it improbable that by chance alone identical somatic mutations are found at exactly the same genomic location in two or more cancer patients. Positive selection is one possible explanation for such mutations, henceforth called recurrent. Recurrent mutations or often more general, recurrently mutated genes and regulatory elements, are used in the prediction of cancer drivers that provide a growth advantage to the cell (2). However, the number of mutations per cancer genome that so far has been identified as being under positive selection is very small (3,4) and the discussion on what are sufficient conditions for driver mutations to cause cancer is on-going (5,6).

While analysis of cancer genomes shows promise in the field of cancer care, there remains an unmet need for methods and systems that provide actionable clinical phenotypes for the management and treatment of cancer patients. The present invention seeks to provide solutions to these needs and provides further related advantages.

**Brief Description of the Invention**

The present inventors hypothesised that recurrent mutations may be highly informative of the non-randomness of mutagenesis and provide a different way to group cancer genomes. In support of this, at both megabase as well as local scale several cancer-specific patterns of the non-random distribution of mutations have been well described [7]. For instance, mutation rate is influenced by replication time [8], is linked to epigenomic features [9], shows a periodic pattern around nucleosomes [10], and can depend strongly on the 5' and 3' flanking base as shown in mutational signatures for several mutational processes [11]. This enrichment of mutations in specific genomic regions or sequence contexts increases the probability of recurrence as does the number of mutations per sample, which also varies across mutagenic processes. As described in detail herein, the present inventors have used recurrence as a starting point for a systematic analysis of cancer genomes from the Pan-Cancer Analysis of Whole Genomes (PCAWG) consortium (10). This cohort study, brought together by an initiative of the International Cancer Genome Consortium (ICGC) and The Cancer Genome Atlas (TCGA), covers 37 tumour types from 2,583 donors and is the largest publicly available dataset of its kind. It allows a comprehensive pan-cancer analysis of recurrence in particular since the somatic mutation calling pipeline was identical across all genomes. Moreover, the whole-genome sequencing data that is available for all donors provides a more complete view than whole-exome sequencing data that so far has been used for large-scale pan-cancer analyses (11). To make full use of the whole-genome sequencing data and analyse recurrence in an unbiased way, the present inventors have taken a purely data driven approach that is independent of the completeness and correctness of current genome annotations. Focussing on Somatic Single-base Mutations (SSMs) and Somatic Insertion/deletion Mutations (SIMs), the inventors first confirmed that the number of recurrent mutations is far higher than expected by chance alone and shed light on the relationship between recurrence and the number of samples. Recurrence was then analysed in the context of general mutational characteristics that capture the effect of mutational processes on the genome. These general features together with recurrence-related features were used to form the base for clustering cancer genomes in a novel way. To help interpret the recurrence observed in the 16 identified clusters, link clusters to potential mutational processes and provide further details of each cluster, the present inventors used various types of metadata, including tumour type information, driver predictions, and replication time. As a result, the present inventors were not only able to refine the mutational consequences of many exposure-specific processes, but also capture clinically relevant phenotypes by using hitherto unused, but easily obtainable mutational features from whole genome sequences.

Accordingly, in a first aspect the present invention provides a computer-implemented method for classifying a test sample obtained from a tumour of a test subject, comprising:

(a) providing a whole genome sequence of the tumour and of a non-tumour sample from the test subject;

(b) analysing the genome sequence of the tumour to compute at least 5, 10, 15, 20 or at least 25 general features selected from the general features shown in Table SF2-1 and at least 5, 6, 7, 8, 9, 10, 11 or at least 12 recurrence features selected from the recurrence features shown in Table SF2-1;

(c) providing at least 5, 6, 7, 8, 9, 10, 11, 12, 13, 14, or at least 15 reference clusters that have been obtained by:

(i) analysing a training data set comprising tumour genomes of at least 1000, 1500, 2000 or at least 2500 subjects to compute said at least 5 general features and at least 5 recurrence features defined in step (b) for each of said at least 1000 subjects;

(ii) performing principal component analysis (PCA) using the features of step (i) as input, optionally after scaling and/or centring the features to account for their different scales, to obtain a plurality of (e.g. 2, 3, 4, 5, 6, 7, 8, 9, 10, 11, 12, 13, 14, 15, 16, 17, 18 or more) principal components that together account for at least 50%, 60%, 70% or at least 80% of the variance of the training data set; and

(iii) performing hierarchical clustering using said plurality of principal components, optionally further performing k-means clustering, to divide the samples of the training data set into at least 5, 6, 7, 8, 9, 10, 11, 12, 13, 14 or at least 15 reference clusters, each reference cluster having a centre that is defined by computing the mean of the plurality of principal components of all samples in that cluster;

(d) determining the Euclidean distance between said plurality of principal components of the test sample and the centre of each of said reference clusters; and

(e) classifying the tumour sample obtained from the test subject as belonging to the reference cluster to which it is found to be nearest in step (d).

The plurality of (e.g. 2, 3, 4, 5, 6, 7, 8, 9, 10, 11, 12, 13, 14, 15, 16, 17, 18 or more) principal components of said general and recurrence features of the test sample are derived using the same PCA as performed on the training data set. In this way, the principal components representing the mutational features of the test sample can be directly compared with the principal components representing the training data set and the clusters derived from the training data set. In some embodiments, the general and recurrence features of the test sample are projected into the existing PCA derived from the training data set. In some embodiments, the PCA is recomputed by including the general and recurrence features of the test sample in the training data set.

Preferably, said at least five general features comprise all 29 general features shown in Table SF2-1. Preferably, said at least five recurrence features comprise all 13 recurrence features shown in Table SF2-1.

Somatic mutation calling may be performed using variant calling pipelines known in the art. Preferably, the somatic mutations are identified in a consistent manner across the training data set and the test sample. In particular embodiments, somatic mutations are identified as described in (10), the entire contents of which is expressly incorporated herein by reference. Briefly, each tumour and normal sample (i.e. matched pair from a subject) is aligned to the human genome (e.g. human reference build hs37d50) and somatic mutations identified in the aligned sequence data using one or more of the computations pipelines "Sanger", "EMBL/DKFZ" and "Broad" - see reference (10). The pipelines used for calling SSMs were MuSE [45] and three in-house pipelines developed at the Deutsches Krebsforschungszentrum (DKFZ) in collaboration with the European Molecular Biology Laboratory (EMBL), Wellcome Sanger Institute and Broad Institute, respectively. A consensus set was built by keeping those calls on which two or more callers agreed. SIMs were called by SMuFIN [46] and three pipelines developed by the same institutes as mentioned for SSMs. The consensus for SIMs was determined by stacked logistic regression, as the level of agreement between the callers was lower than for SSMs. Furthermore, the SIM calls were left aligned to make them comparable across samples.

Somatic mutation calling may comprise a filtering step to remove germline variants. In some embodiments mutation calling may comprise a filtering step to remove oxidative damage due to sample preparation (see, e.g., as described in [47]). Further filters may be one or more filters as described in [48], incorporated herein by reference.

Once the somatic mutations have been identified, the general and recurrence features set forth in Table SF2-1 are determined for each sample.

The 29 general features shown in Table SF2-1 are:

1. Number of somatic single-base mutations (SSMs)
2. Number of somatic insertion/deletion mutations (SIMs)
3. % of somatic mutations that are of the type SIM
4. % of C>A SSMs
5. % of C>G SSMs
6. % of C>T SSMs
7. % of T>A SSMs
8. % of T>C SSMs

9. % of T>G SSMs

10.% of 1 bp A/T deletions

11.% of 1 bp C/G deletions

12.% of 1 bp A/T insertions

13.% of 1 bp C/G insertions

14.% of 1 bp A/T deletions that have no homopolymer context (i.e. the based next to the one that is deleted is not of the same type)

15.% of 1 bp A/T deletions that have a short homopolymer context (i.e. a 2-4 bp mononucleotide repeat of the same base as the 1 bp SIM)

16.% of 1 bp A/T deletions that have a midsize homopolymer context (i.e. a 5-7 bp mononucleotide repeat of the same base as the 1 bp SIM)

17.% of 1 bp A/T deletions that have a long homopolymer context (i.e. a $\geq$ 8 bp mononucleotide repeat of the same base as the 1 bp SIM)

18.% of 1 bp C/G deletions that have no homopolymer context (i.e. the based next to the one that is deleted is not of the same type)

19.% of 1 bp C/G deletions that have a short homopolymer context (i.e. a 2-4 bp mononucleotide repeat of the same base as the 1 bp SIM)

20.% of 1 bp C/G deletions that have a midsize homopolymer context (i.e. a 5-7 bp mononucleotide repeat of the same base as the 1 bp SIM)

21.% of 1 bp C/G deletions that have a long homopolymer context (i.e. a $\geq$ 8 bp mononucleotide repeat of the same base as the 1 bp SIM)

22.% of 1 bp A/T insertions that have no homopolymer context (i.e. the based next to the one that is deleted is not of the same type)

23.% of 1 bp A/T insertions that have a short homopolymer context (i.e. a 2-4 bp mononucleotide repeat of the same base as the 1 bp SIM)

24.% of 1 bp A/T insertions that have a midsize homopolymer context (i.e. a 5-7 bp mononucleotide repeat of the same base as the 1 bp SIM)

25.% of 1 bp A/T insertions that have a long homopolymer context (i.e. a $\geq$ 8 bp mononucleotide repeat of the same base as the 1 bp SIM)

26.% of 1 bp C/G insertions that have no homopolymer context (i.e. the based next to the one that is deleted is not of the same type)

27.% of 1 bp C/G insertions that have a short homopolymer context (i.e. a 2-4 bp mononucleotide repeat of the same base as the 1 bp SIM)

28.% of 1 bp C/G insertions that have a midsize homopolymer context (i.e. a 5-7 bp mononucleotide repeat of the same base as the 1 bp SIM)

29.% of 1 bp C/G insertions that have a long homopolymer context (i.e. a $\geq$ 8 bp mononucleotide repeat of the same base as the 1 bp SIM)

The 13 recurrence features shown in Table SF2-1 are:

1. % of recurrent SSMs

2. % of recurrent SIMs

3. % of recurrent mutations that are of the type SIM

4. % of recurrent C>A SSMs

5. % of recurrent C>G SSMs

6. % of recurrent C>T SSMs

7. % of recurrent T>A SSMs

8. % of recurrent T>C SSMs

9. % of recurrent T>G SSMs

10.% of recurrent 1 bp A/T deletions

11.% of recurrent 1 bp C/G deletions

12.% of recurrent 1 bp A/T insertions

13.% of recurrent 1 bp C/G insertions

In accordance with this and other aspects of the invention, mutations may, in some embodiments, be considered recurrent if they are recurrent in the Pan-Cancer Analysis of Whole Genomes (PCAWG) consortium cohort as of the finalized PCAWG mutation call of 14 September 2016.- see reference (10). In some embodiments, a mutation (SSM or SIM) is considered recurrent if it occurs at the same genomic position in more than one sample across the entire training set (e.g. the entire PCAWG cohort). In some embodiments, a mutation (SSM or SIM) may be considered recurrent if it occurs at the same genomic position in more than one sample across all the samples of a particular cancer type in the training set (e.g. all the samples of Liver-HCC of the PCAWG cohort).

In some embodiments in accordance with this and other aspects of the invention the training data set comprises 2583 cancer genomes of the PCAWG consortium cohort as of 14 September 2016 - see reference (10), incorporated herein by reference in its entirety. The training data set may comprise the cancer genomes of the PCAWG consortium cohort after applying quality controls to filter samples of low quality. The quality control framework may be as described in [49], incorporated herein by reference.

In some embodiments the plurality of principal components comprises the first 18 principal components. As described in detail herein, it was found that the first 18 principal components explain around 80% of the variance of the PCAWG data set, with the remaining components likely representing noise in the data. Therefore, using the first 18 principal components of the data comprising the general and recurrence features captures the majority of the informative signal in the data set.

In some embodiments the distance from the test sample to the closest cluster centre in step (d) is found to be within 0.5 of the distance to the next closest cluster centre. As described in detail herein, the PCA of the PCAWG cohort general and recurrence features, that is to say, the first 18 principal components of the PCA using all 42 features found that the distance between all 2,583 samples ranged from 0.6 to 49.5. The median distance between samples was 7.0. Therefore, a difference in Euclidean distance of 0.5 is, in this context, considered close. That is, the test sample is almost equidistant to two or more clusters resulting in ambiguous classification. In such cases the method may classify the sample as belonging to one of two or more (e.g. 3) potential clusters, i.e. the highest-ranking or closest clusters to the sample. In some embodiments the method further comprise a step of adding employing further statistics and/or metadata to confirm the classification. These may include, e.g., one or more of: replication time; drivers (where available), tumour type (where known from the sample); and sequence motif(s). The method of deriving sequence motifs may be as described in detail below.

*Sequence motifs*

We collected for clusters A, E, G, H and L all SSMs of the subtype that is the most characteristic. This is C>A for clusters A and H, C>G for cluster E, C>T for cluster G and T>G for cluster L. Next, we extracted from the reference genome (GRCh37/h19) the ten adjacent bases in 5' and 3' direction of the mutation. We used them as input to construct two sequence logos per cluster: one for the non-recurrent mutations and one for the recurrent ones (pan-cancer definition of recurrence). As a measure of information content we used the relative entropy [50, 51], which is defined for position i by:

$$RE_i = \sum_{b \,\in\{A,C,G,T\}} f(b_i) \, log_2 \frac{f(b_i)}{P(b)}$$

Here, $f(b_i)$ stands for the frequency of base $b$ (A, C, G or T) in position $i$ and $P(b)$ stands for the prior probability of base $b$ as determined by the frequency in the human genome (GRCh37/h19). The height of each base in the sequence plot is proportional to $f(b_i) log_2 \frac{f(b_i)}{P(b)}$. A positive value corresponds to an enrichment of the base with respect to the prior probability and a negative value to a depletion. The relative entropy ($RE_i$) is zero, if all four bases are observed with the same frequency as the prior in position $i$. We set 0.25 as a threshold for $RE_i$ to define the enriched motif. Furthermore, we computed per cluster the percentages of all, non-recurrent and recurrent SSMs that were in the sequence context that was found to be enriched in the recurrent SSMs. To estimate the percentage of the respective motifs in the human genome, we first slid a window of the same size (k) as the motif across the genome with a shift equal to the length of the motif and counted all possible k-mers. Next, we added to this the counts retrieved in the same way for the reverse complement of the reference sequence (corresponding to the opposing strand), since we also combined the reverse complements for each of the SSM subtypes. From this we computed the percentage of the enriched motif with respect to all k-mers and to the k-mer with the base that is mutated in the enriched motif at the same position.

In some embodiments the at least five reference clusters comprise 16 reference clusters. As described in detail herein, 16 clusters were chosen as a trade-off between too many clusters, which would each be specific to just a handful of samples, and too few, which would result in loss of meaningful information.

In particular, said 16 reference clusters may be defined by the following centroids (i.e. means of the first 18 principal components) as set forth in Table 2 or Table 3):

**Table 2** - Centroids rounded to 2 decimal places

| cluster | Principal component means - centroid | | | | | | | | | | | | | | | | | |
|---|---|---|---|---|---|---|---|---|---|---|---|---|---|---|---|---|---|---|
| | PC1 | PC2 | PC3 | PC4 | PC5 | PC6 | PC7 | PC8 | PC9 | PC10 | PC11 | PC12 | PC13 | PC14 | PC15 | PC16 | PC17 | PC18 |
| A | -4.21 | 1.48 | 0.2 | 0.23 | 0.93 | 0.44 | 1.44 | 2.47 | 0.19 | -0.4 | 1.15 | 0.64 | -0.67 | -0.51 | 1.11 | 0.09 | 0.05 | -1.52 |
| B | -2.31 | 1.72 | 0.11 | -0.16 | 1.05 | -1.17 | -0.05 | -0.76 | -0.32 | 0.24 | -0.25 | 0.03 | -0.23 | 0.01 | 0.19 | -0.1 | 0.29 | -0.03 |
| C | -4.64 | -0.2 | 1.87 | 1.17 | -0.39 | 0.51 | -0.66 | 0.59 | 0.59 | -0.74 | 0.31 | -0.3 | 0.8 | -0.06 | -0.5 | -0.21 | -0.03 | 0.6 |
| D | -1.71 | -0.19 | 0.08 | -0.49 | -0.57 | 0.64 | 0.24 | -0.49 | -0.31 | 0.15 | 0.06 | 0.15 | -0.12 | 0.04 | -0.11 | 0.27 | -0.24 | -0.15 |
| E | -0.35 | -0.34 | -2.03 | -0.47 | -1.48 | 1.82 | 0.84 | 1.03 | 0.26 | 0.63 | -0.98 | -0.28 | -1.25 | 0.2 | 0.66 | -1.16 | -0.44 | 1.22 |
| F | 0.31 | 0.44 | -1.47 | 0.48 | 0.32 | -0.24 | -1.72 | 0.09 | 1.85 | 2.22 | 0.81 | 0.06 | -0.15 | 0.83 | -0.17 | -0.03 | -0.13 | -0.42 |
| G | -1.18 | -6.56 | -1.41 | 2.01 | -1.52 | -1.7 | 0 | -0.97 | 0.19 | -0.01 | -0.07 | -0.03 | -0.23 | -0.13 | 0.2 | 0.23 | 0.82 | -0.15 |
| H | 3.76 | -2.83 | 0.91 | -1.39 | -1.73 | -6.74 | 6.19 | 4.59 | -1.73 | -0.55 | 6.33 | 5.13 | 2.12 | 4.14 | 2.45 | -1.38 | 1.02 | 0.35 |
| I | 0.71 | -1.8 | 0.52 | -0.47 | 1.17 | -0.46 | 1.07 | 3.08 | 2.86 | -0.46 | -5.26 | 0.06 | 2.7 | 1.59 | 0.56 | 3.62 | 1 | -0.98 |
| J | 7.76 | 4.98 | 6.64 | 8.57 | -3.91 | -1.7 | -0.73 | 0.66 | -2.84 | 1.78 | -2.46 | 2.45 | -1.37 | 0.75 | -0.54 | 0.35 | -1.3 | -0.12 |
| K | 0.85 | 0.35 | -1.22 | 0.01 | 0.2 | -0.14 | 0.1 | 0.08 | -0.4 | -0.25 | -0.23 | -0.07 | 0.25 | -0.02 | -0.22 | 0.03 | -0.05 | 0.04 |
| L | 3.21 | -1.18 | 3.34 | -2.8 | -0.12 | -1.15 | 0.57 | 0.01 | 1.01 | 0.21 | -0.85 | -0.6 | 0.19 | -0.1 | -0.03 | -1.05 | -0.36 | -0.26 |
| M | 3.22 | -0.01 | 1.37 | -1.18 | -0.3 | 0.14 | -1.02 | -0.14 | -0.38 | -0.09 | 0.48 | -0.27 | 0.13 | -0.07 | 0.57 | 0.28 | 0.38 | 0.26 |
| N | 3.85 | 0.51 | -0.65 | 0.75 | -0.09 | 0.17 | 0.06 | 0.17 | 0.48 | -0.59 | 0.38 | -0.09 | 0.05 | -0.39 | -0.2 | 0.08 | 0.05 | -0.12 |
| O | 2.56 | -4.32 | 2.01 | -0.69 | 4.85 | 1.78 | -1.38 | 1.09 | -0.84 | -0.14 | -0.1 | 2.08 | -1.51 | 0.21 | -0.17 | -0.04 | -0.65 | 0.36 |
| P | 5.87 | -2.22 | 4.27 | 5.97 | 5.67 | 5.36 | 7.74 | -3.62 | -1.15 | 4.74 | 2.04 | -4.3 | 2.38 | 0.96 | 1.29 | -0.56 | 1.79 | -0.16 |

**Table 3** - Centroids partially rounded

| Cluster | Principal component means - centroids | | | | | | | | | | | | | | | | | |
|---|---|---|---|---|---|---|---|---|---|---|---|---|---|---|---|---|---|---|
| | PC1 | PC2 | PC3 | PC4 | PC5 | PC6 | PC7 | PC8 | PC9 | PC10 | PC11 | PC12 | PC13 | PC14 | PC15 | PC16 | PC17 | PC18 |
| A | -4.20897 | 1.480967 | 0.199653 | 0.22953 | 0.927902 | 0.438706 | 1.443725 | 2.473314 | 0.18633 | -0.40449 | 1.150209 | 0.640848 | 0.66865 | -0.50873 | 1.106113 | 0.08597 | 0.046066 | -1.51563 |
| B | -2.30605 | 1.716011 | 0.106674 | -0.1623 | 1.05152 | -1.17199 | 0.04888 | -0.7616 | -0.31773 | 0.236173 | -0.24927 | 0.026353 | 0.23327 | -0.013184 | 0.194005 | 0.09671 | -0.294698 | 0.02696 |
| C | -4.64379 | -0.2004 | 1.867407 | 1.166544 | -0.39014 | 0.507795 | -0.66085 | 0.593924 | 0.592668 | -0.74412 | 0.311173 | 0.29701 | 0.79859 | -0.06348 | -0.50095 | -0.20809 | -0.02893 | 0.60444 |
| D | -1.71309 | 0.19489 | -0.075795 | 0.49212 | 0.56627 | -0.639946 | 0.237284 | -0.48909 | 0.30781 | -0.151445 | 0.061267 | 0.145104 | 0.11914 | -0.039326 | 0.10898 | -0.268878 | -0.243 | -0.15284 |
| E | -0.34545 | -0.33794 | -2.02658 | -0.47455 | -1.48215 | 1.822368 | 0.838869 | 1.03076 | 0.259737 | 0.628583 | -0.98395 | -0.28191 | -1.2543 | 0.201229 | 0.656366 | -1.16045 | 0.44197 | -1.221533 |
| F | 0.312851 | 0.444151 | -1.4723 | 0.480967 | 0.322668 | -0.24223 | -1.72182 | -0.092504 | 1.845398 | 2.224746 | 0.809566 | 0.063692 | -0.15429 | -0.833825 | -0.16599 | -0.02676 | -0.13051 | -0.41861 |
| G | 1.17791 | -6.5558 | 1.41295 | -2.01417 | 0.322668 | -1.69704 | -0.00369 | -0.96832 | 0.186023 | -0.00808 | -0.07343 | -0.02641 | -0.22587 | -0.13451 | 0.202438 | 0.234102 | 0.822119 | -0.1479 |
| H | 3.764501 | -2.82548 | 0.91399 | -1.39343 | -1.72795 | 6.73866 | 6.19405 | 4.587034 | -1.73262 | 0.55011 | 6.329088 | 5.127124 | 2.122616 | 4.139913 | 2.448706 | 1.37904 | -1.023289 | 0.349038 |
| I | 0.709432 | -1.79707 | 0.516468 | -0.46744 | 1.169193 | -0.46207 | 1.072478 | 3.081309 | 2.862926 | -0.4572 | 5.26267 | 0.056944 | 2.695535 | 1.591904 | 0.563427 | 3.616428 | 1.002831 | -0.97887 |
| J | 7.759114 | 4.976233 | 6.641945 | 8.57196 | -3.9121 | 1.70009 | -0.73148 | -0.658961 | 2.83751 | -1.776132 | 2.45866 | -2.44877 | 1.36803 | -0.750801 | 0.53713 | -0.349761 | -1.3043 | -0.1227 |
| K | 0.852349 | 0.348819 | -1.22342 | 0.01112 | 0.196838 | -0.14018 | 0.10366 | 0.081599 | -0.39977 | 0.24994 | -0.22947 | 0.07075 | 0.249521 | -0.01739 | 0.21984 | -0.027232 | -0.04788 | 0.043366 |
| L | 3.212082 | -1.18064 | 3.335321 | -2.79643 | -0.12145 | -1.15072 | 0.570131 | 0.006534 | 1.011482 | 0.205807 | -0.85003 | 0.60487 | 0.19336 | -0.09801 | -0.02562 | 1.04718 | -0.36172 | -0.26407 |
| M | 3.216516 | -0.0136 | 1.366802 | -1.18187 | -0.30219 | 0.139308 | -1.01706 | -0.13902 | -0.38254 | -0.0852 | 0.475884 | -0.26946 | 0.131181 | -0.07366 | -0.567388 | 0.281186 | 0.379693 | 0.258594 |
| N | 3.853626 | 0.505979 | -0.64945 | 0.751259 | -0.09393 | -0.174972 | 0.058984 | 0.172313 | 0.478314 | -0.58738 | 0.38276 | -0.09075 | 0.04747 | -0.38758 | -0.20135 | -0.076343 | 0.050549 | -0.12328 |

EP 3 739 065 A1

(continued)

| Cluster | Principal component means - centroids | | | | | | | | | | | | | | | | | |
|---|---|---|---|---|---|---|---|---|---|---|---|---|---|---|---|---|---|---|
| | PC1 | PC2 | PC3 | PC4 | PC5 | PC6 | PC7 | PC8 | PC9 | PC10 | PC11 | PC12 | PC13 | PC14 | PC15 | PC16 | PC17 | PC18 |
| O | 2.557231 | -4.32277 | 2.008867 | -0.69398 | 4.854016 | 1.778267 | -1.38255 | 1.088338 | -0.83623 | -0.13657 | -0.10098 | 2.08496 | -1.51481 | -0.211366 | -0.16855 | -0.03655 | -0.65044 | -0.361973 |
| P | 5.873468 | -2.22379 | 4.270804 | 5.96871 | 5.673558 | 5.357843 | 7.740246 | -3.61762 | 1.15059 | -4.73614 | 2.035072 | -4.304049 | 2.37596 | 0.960771 | 1.285512 | -0.55624 | 1.793306 | -0.16075 |

For example, the centroids may be:

[In the format: Cluster, PC1,PC2,PC3,PC4,PC5,PC6,PC7,PC8,PC9,PC10,PC11,PC12,PC13,PC14,PC15,PC 16,PC17,PC18]

A,-4.208970706,1.480966874,0.199652999,0.229529713,0.927901504,0.438705643,1.4 43724984,2.473314152,0.186330465,-0.404485401,1.150208507,0.640848376,-0.668649541,- 0.508734156,1.106113054,0.085969532,0.046066213,-1.515628453

B,-2.306046902,1.716011158,0.106674173,-0.162303114,1.051519868,-1.171989824,-0.048879304,- 0.761602622,-0.317731986,0.236172646,-0.249273813,0.026352635,- 0.233267798,0.013184195,0.194005069,-0.096708342,0.294697987,-0.026957532

C,-4.643792107,-0.200402568,1.867406508,1.166544017,-0.390138425,0.507794637,- 0.660847837,0.59392375,0.592667686,-0.744122446,0.31117277,-0.297006507,0.798590395,-0.063476752,- 0.500953911,-0.20808781,-0.028926907,0.604440384

D,-1.713094529,-0.194894149,0.075795277,-0.49211805,-0.566272478,0.639946101,0.237283539,- 0.489088899,-0.307813243,0.151445002,0.061266573,0.145103801,-0.119139937,0.039326318,- 0.108983337,0.268878095,-0.243001859,-0.152835078

E,-0.3454474,-0.337940236,-2.02657792,-0.474554851,- 1.482146613,1.822368376,0.838868539,1.030760204,0.259737086,0.628582546,-0.983953636,-0.281913801,- 1.254300555,0.201229236,0.656366169,-1.160453083,-0.441971807,1.221533091

F,0.312850817,0.444150851,-1.472304929,0.480967035,0.322667766,-0.24223234,- 1.721823997,0.092504359,1.845398269,2.22474571,0.809565914,0.063691634,-0.154287977,0.833824984,- 0.165988333,-0.026761116,-0.130505168,-0.418609328

G,-1.177908341,-6.555799464,-1.412951832,2.014170013,0.322667766,-1.697040297,-0.003691908,- 0.968324221,0.186022554,-0.008083605,-0.073428286,-0.026412841,-0.225865983,- 0.134512043,0.202438276,0.234102267,0.822118909,-0.147896161

H,3.764500775,-2.825480888,0.913990155,-1.393431761,-1.727946191,- 6.738661846,6.194050014,4.587033547,-1.732615267,- 0.550114173,6.329087958,5.127124157,2.122616324,4.139912686,2.448706197,- 1.379036886,1.023288837,0.349037965

I,0.709432499,-1.797074357,0.516467731,-0.467442551,1.169192687,- 0.462074949,1.072478126,3.081308523,2.862925542,-0.45719614,- 5.26267264,0.056944295,2.695535447,1.591903745,0.563426758,3.616428465,1.00 2830973,-0.97887243

J,7.75911447,4.976233044,6.641945153,8.571960377,-3.912096015,-1.70008563,-0.73147868,0.65896144,- 2.837511691,1.776131588,-2.45866386,2.448771559,-1.368033695,0.750801401,-0.537129746,0.349760664,- 1.304303044,-0.122696289

K,0.852348754,0.348819265,-1.223418116,0.01111988,0.196837532,- 0.140180606,0.103659583,0.081599071,-0.399771887,-0.249943075,-0.229466492,- 0.070746743,0.249520659,-0.017392256,-0.219838299,0.027231666,-0.047881957,0.043366443

L,3.212081895,-1.180642364,3.33532086,-2.796428277,-0.121453116,- 1.150716382,0.570131335,0.006534163,1.011482315,0.205806908,-0.850025908,-0.604867322,0.193360023,- 0.098008859,-0.025622683,-1.047181582,-0.361724002,-0.264067397

M,3.216515997,-0.013596013,1.366802338,-1.181872997,-0.3021884,0.139308006,-1.017062675,- 0.139021486,-0.382543609,-0.085195739,0.47588391,-0.269458936,0.131181113,- 0.073664827,0.56738807,0.281186188,0.379692855,0.258593619

N,3.853625868,0.505979224,-0.649452198,0.751259384,-0.093933066,0.174972031,0.058983602,0.172312888,0.478313951,-0.58737571,0.382760055,-0.090754723,0.047469806,-0.387575384,-0.20134712,0.076342926,0.050549107,-0.123277041

0,2.557230663,-4.322771559,2.008866532,-0.693980679,4.85401617,1.778266655,-1.382546735,1.088338194,-0.836233151,-0.136565925,-0.100981243,2.084960167,-1.514814637,0.211365975,-0.16855322,-0.036547995,-0.650438949,0.361973123

P,5.873468414,-2.223789306,4.270804106,5.968709638,5.67355777,5.357843161,7.740245972,-3.617615304,-1.150589294,4.73614174,2.035078515,-4.304039974,2.375961485,0.960770741,1.285512489,-0.556241839,1.793305898,-0.160752597

In some embodiments, the test sample is classified as belonging to one of the following medium or most stable clusters:

Cluster A (lung cancers - smoking)
Cluster B (liver cancer)
Cluster C (kidney cancers)
Cluster E (increased activity of cytidine deaminases)
Cluster G (skin melanoma - UV light)
Cluster J (MSI)
Cluster F (unknown)
Cluster H (deregulated activity of POLE)
Cluster I (unknown)
Cluster L (Eso-AdenoCA and Stomach-AdenoCA - gastric acid) and
Cluster O (unknown).

In some embodiments, the test sample is classified as belonging to one of the following most stable clusters:

Cluster A;
Cluster B;
Cluster C;
Cluster E;
Cluster G; and
Cluster J.
As described in detail herein, the leave-one-out validation identified clusters A, B, C, E, G and J and particularly robust. It is therefore concluded that classification of a sample to one of these clusters may have a higher degree of confidence.

In a second aspect, the present invention provides a method for classifying a test sample obtained from a tumour of a test subject, comprising:

sequencing DNA obtained from the test sample to provide a whole genome sequence of the tumour, optionally also sequencing a non-tumour originating sample from the same subject; and
performing the method of the first aspect of the invention using the whole genome sequence thus obtained.

In some embodiments sequencing comprises extracting and/or amplifying DNA from the sample.
In some cases, the sequencing step may comprise Next-generation Sequencing (NGS), including Illumina® sequencing, or Sanger sequencing. NGS offers the speed and accuracy required to detect mutations, e.g. through whole-genome sequencing (WGS). Examples of NGS techniques include methods employing sequencing by synthesis, sequencing by hybridisation, sequencing by ligation, pyrosequencing, nanopore sequencing, or electrochemical sequencing.
In some cases, the method of this aspect of the present invention further comprises a step, prior to sequencing, of preparing a DNA library from a sample (e.g. a tumour sample, a blood or plasma sample) obtained from the patient or from more than one patient. Optionally, the library may be barcoded. In some cases, the method of this aspect of the present invention further comprises a step prior to sequencing of obtaining a sample from the patient. For example, a blood sample or tissue biopsy may be collected from a patient who has been diagnosed as having, or being likely to have, a cancer. Additionally or alternatively a sample comprising circulating tumour DNA (ctDNA) (so-called liquid biopsy) may be obtained and used to provide a whole genome of the tumour for somatic mutation calling. A liquid biopsy approach may be preferred where a tissue biopsy is considered too invasive or where the location of the tumour is unknown or wherein the tumour is only suspected or in the context of tumour monitoring, including after surgical resection. The sample may be subjected to one or more extraction or purification steps, such as centrifugation, in order to obtain substantially cell-free DNA source (e.g. to obtain a plasma sample). It is specifically contemplated that the sample may be transported and/or stored (optionally after freezing). The sample collection may take place at a location remote from the sequencing location and/or the computer-implemented method steps may take place at a location remote from the sample collection location and/or remote from the sequencing location (e.g. the computer-implemented method steps may be performed by means of a networked computer, such as by means of a "cloud" provider). Nevertheless, the entire method may in some cases be performed at single location, which may be advantageous for "on-site" classification or monitoring of cancer.

In a third aspect, the present invention provides a system for classifying a test sample obtained from a tumour of a test subject, comprising:

at least one processor; and
at least one non-transitory computer readable medium containing instructions that, when executed by the at least one processor, cause the at least one processor to perform operations comprising:

(a) receiving sequence data representing the whole genome sequence of the tumour and of a non-tumour sample from the test subject;
(b) analysing the sequence data to compute the 29 general features shown in Table SF2-1 and the 13 recurrence features shown in Table SF2-1;
(c) receiving principal components data defining the 16 reference clusters as set forth in Table 2 or Table 3;
(d) determining the Euclidean distance between the first 18 principal components of the test sample (projected onto the same PCA as in step (c)) and the centre of each of said reference clusters; and
(e) classifying the tumour sample obtained from the test subject as belonging to the reference cluster to which it is found to be nearest in step (d).

In some embodiments, the system is for use in the method of the first aspect of the invention.

In a fourth aspect, the present invention provides a non-transitory computer readable medium for classifying a test sample obtained from a tumour of a test subject, comprising instructions that, when executed by at least one processor, cause the at least one processor to perform operations comprising:

(a) receiving sequence data representing the whole genome sequence of the tumour and of a non-tumour sample from the test subject;
(b) analysing the sequence data to compute the 29 general features shown in Table SF2-1 and the 13 recurrence features shown in Table SF2-1;
(c) receiving principal components data defining the 16 reference clusters as set forth in Table 2 or Table 3;
(d) determining the Euclidean distance between the first 18 principal components of the test sample and the centre of each of said reference clusters; and
(e) classifying the tumour sample obtained from the test subject as belonging to the reference cluster to which it is found to be nearest in step (d).

In some embodiments, the medium is for use in the method of the first aspect of the invention.

In a fifth aspect, the present invention provides a method for predicting that a tumour of a patient is microsatellite instable (MSI), comprising: performing the method of the first aspect of the invention, wherein the test sample is a sample that has been obtained from the tumour of the patient, and wherein the sample is classified as belonging to cluster J.

As described in detail herein, classification into cluster J provides a reliable indication that the sample is MSI. Indeed, a comparison was made with alternative MSI classification methods MSI-method 1 and MSI-method 2. MSI-method 1 classified 16 samples in the entire data set as MSI of which 15 belong to cluster J. MSI-method 2 classified 14 of the 17 samples in cluster J as MSI, along with an additional five samples from other clusters. These results indicate that classification to cluster J has a high degree of correlation with other methods of identifying MSI. Moreover, use of the method of the present invention may correct MSI misclassifications made by other methods of MSI classification. As will be appreciated by a person skilled in the art, MSI is an important marker for determining prognosis for cancer treatments and is, for example, a relevant criteria for the use of certain immunotherapy (e.g. PD-1 inhibitor pembrolizumab).

In a sixth aspect the present invention provides a method for predicting whether a tumour-burdened patient will benefit from immunotherapy, comprising: performing the method of the first aspect of the invention, wherein the test sample is a sample that has been obtained from the tumour of the patient, and wherein the sample is classified as belonging to cluster H, Cluster J or cluster L.

As described in detail herein, cluster H is associated with ultra-hypermutation, which has also been related to beneficial results from immunotherapy (36,37), incorporated herein by reference. Samples of this cluster have a high mutational load as well, but form a mirror image to the MSI samples in terms of other characteristics like a high absolute and relative number of SSMs, and high levels of recurrent SSMs.

Eso-AdenoCA samples that are assigned to cluster L, which have a high percentage of T>C as well as T>G SSMs and a higher total mutational load than Eso-AdenoCA samples not assigned to this cluster. Eso-AdenoCA samples with the characteristics of cluster L have also been suggested to benefit from immunotherapy (39), incorporated herein by reference. The same treatment option may therefore be prioritized for the 22 Stomach-AdenoCA samples that are also in cluster L.

Once a sample has been classified as belonging to cluster H or L and therefore likely to benefit from immunotherapy, the method of the invention may further comprise:

   selecting the patient for immunotherapy to treat said tumour; and/or
   administering a therapeutically effective amount of an immunotherapy to the patient to treat said tumour.

In some cases, the immunotherapy comprises an immune checkpoint inhibitor, such as an anti-PD-1, anti-PD-LI or anti-CTLA-4 antibody. In particular, the immunotherapy may be selected from the group consisting of: Nivolumab (PD-1), ipilimumab (CTLA-4), pembrolizumab (PD-1), cemiplimab (PD-1); PD-L1: atezolizumab, avelumab and durvalumab. Pembrolizumab is approved for adult and paediatric patients with unresectable or metastatic, microsatellite instability-high (MSI-H) or mismatch repair deficient (dMMR) solid tumours that have progressed following prior treatment and who have no satisfactory alternative treatment options or with MSI-H or dMMR colorectal cancer.

In a seventh aspect, the present invention provides a method for prognosis of a patient having chronic lymphocytic leukaemia (Lymph-CLL), comprising: performing the method of the first aspect of the invention, wherein the test sample is a sample that has been obtained from the tumour of the Lymph-CLL patient, and wherein:

   if the sample is classified as belonging to cluster M, the patient is predicted to have a longer survival time;
   if the sample is classified as belonging to cluster D, the patient is predicted to have a shorter survival time.

As described in detail herein, the clearest difference in patient survival by cluster was observed for Lymph-CLL, which coincides with the known differences in survival for patients with and without hypermutation of the immunoglobulin heavy-chain variable region genes. Cluster M Lymph-CLL patients had a significantly longer survival probability than cluster D Lymph-CLL patients (p = 0.0022). Thus, the classification of Lymph-CLL patients into cluster M or D represents an alternative to classification based on detecting hypermutation of the immunoglobulin heavy-chain variable region genes despite the fact that the method of the present invention does not explicitly single-out the analysis of immunoglobulin genes. The more favourable prognosis of the less aggressive form of Lymph-CLL classified to cluster M may in turn influence treatment selection. For example, a patient whose Lymph-CLL is classified to cluster M may be selected for less aggressive treatment, whereas a patient whose Lymph-CLL is classified to cluster D may be selected for more aggressive treatment.

In an eighth aspect, the present invention provides a method for predicting the primary origin of a metastatic tumour in a subject, comprising performing the method of the first aspect of the invention, wherein the test sample is a sample that has been obtained from the metastatic tumour of the patient. A number of the clusters are dominated by a single cancer type and therefore the classification to a cluster may shed light on the possible tissue of origin of a metastatic sample of unknown primary site. In particular,

   if the sample is classified as belonging to cluster A, the primary origin of the metastatic tumour may be predicted to be lung (e.g. lung-adenoCA or lung-SCC);
   if the sample is classified as belonging to cluster B, the primary origin of the metastatic tumour may be predicted to be liver (e.g. liver-HCC);
   if the sample is classified as belonging to cluster C, the primary origin of the metastatic tumour may be predicted to be kidney (e.g. kidney-RCC or kidney-ChRCC);
   if the sample is classified as belonging to cluster G, the primary origin of the metastatic tumour may be predicted to be skin (e.g. melanoma); and, optionally,
   if the sample is classified as belonging to cluster H, the primary origin of the metastatic tumour may be predicted to be colorectal or uterus adenocarcinoma.

Clusters A and E were found to have an elevated percentage of C>G SSMs combined with elevated percentage of C>T SSMs corresponds to a signature previously described for increased activity of cytidine deaminases. The enriched motif uncovered for recurrent C>G SSMs suggests a link to deamination by APOBEC3. The apolipoprotein B mRNA-editing enzyme, catalytic polypeptide-like (APOBEC) mutational signature has only recently been detected in a multitude of cancers through next-generation sequencing. As reported in Ann Oncol. 2018 Mar; 29(3): 563-572, doi: 10.1093/annonc/mdy003, Science Advances 07 Oct 2016: Vol. 2, no. 10, e1601737 DOI: 10.1126/sciadv.1601737, and Mol Ther Oncolytics. 2018 Dec 21; 11: 1-13 doi: 10.1016/j.omto.2018.08.003, there are two general strategies for targeting APOBEC in cancer. The first strategy is therapy by hypermutation, by enhancing the mutagenic effects of APOBEC to the point where cancer cells suffer catastrophic levels of DNA damage and selectively die. Indeed, recent studies have suggested that DDR inhibitors, such as PARP and ATR inhibitors, may sensitize tumour cells with high levels of APOBEC to an APOBEC-dependent death. The second strategy is therapy by hypomutation, by inhibiting APOBEC-dependent tumour evolution and potentially suppressing adverse outcomes including recurrence, metastasis, and drug resistance. Constraining cancer evolvability may be accomplished with drugs to inhibit APOBEC gene expression or with chemical inhibitors of DNA deaminase activity. Proof of principle has been achieved with a genetic knockdown of A3B causing an improvement in the durability of tamoxifen treatment of ER+ xenograft tumours in mice. Besides harnessing APOBEC through a cancer cell intrinsic mechanism, a complementary strategy may be provoking immune responses to cancer cells with high levels of APOBEC-induced neoantigens. It is important to consider whether neoantigens are present in the trunk of the tumour (clonal) or in the branches (subclonal), since clonal neoantigens may improve response to immune checkpoint blockade in contrast to subclonal neoantigens. Interestingly, one APOBEC-mediated mutational signature (signature 2) is primarily found in the branches of multiple different cancer types. In contrast, the other APOBEC-mediated mutational signature (signature 13) is primarily found in the trunk of bladder cancer. This could potentially explain paradoxical effects of APOBEC mutagenesis on immune surveillance and patient outcome. For example, in breast cancer, A3B expression is reported to worsen overall patient survival. In contrast, APOBEC mutagenesis within bladder cancer has been correlated with an improved patient outcome, although the influence of APOBEC on survival in bladder cancer is still a matter of debate. Therefore, it is conceivable that patients with tumours containing extensive clonal APOBEC mutagenesis, in contrast to subclonal APOBEC mutagenesis, are more suitable for immune checkpoint blockade.

Accordingly, in a ninth aspect, the present invention provides a method for predicting whether a tumour-burdened patient will benefit from apolipoprotein B mRNA-editing enzyme, catalytic polypeptide-like (APOBEC)-targeting therapy, comprising: performing the method of the first aspect of the invention, wherein the test sample is a sample that has been obtained from the tumour of the patient, and wherein the sample is classified as belonging to cluster A or cluster E.

Embodiments of the present invention will now be described by way of examples and not limited thereby, with reference to the accompanying figures. However, various further aspects and embodiments of the present invention will be apparent to those skilled in the art in view of the present disclosure.

The present invention includes the combination of the aspects and preferred features described except where such a combination is clearly impermissible or is stated to be expressly avoided. These and further aspects and embodiments of the invention are described in further detail below and with reference to the accompanying examples and figures.

**Brief Description of the figures**

*Figure 1*

Table SF5-1: Cluster-specific overview of total and recurrent SSMs per subtype. The number of SSMs refers to the number all samples of a cluster have combined, counting recurrent SSMs once. The number (and percentage) of recurrent SSMs is computed based on only the samples from the particular cluster (within cluster) and on the entire cohort. In dark red is indicated per SSM subtype the cluster with the lowest median number of SSMs or the lowest percentage of recurrence for 'within cluster' and 'entire cohort'. In light red the second lowest is indicated. The highest and second highest are indicated in dark and light green, respectively

*Figure 2*

Table SF5-2: Cluster-specific overview of total and recurrent 1 bp SIMs per subtype. The number of 1 bp SIMs refers to the number all samples of a cluster have combined, counting recurrent 1 bp SIMs once. The number (and percentage) of recurrent 1 bp SIMs is computed based on only the samples from the particular cluster (within cluster) and on the entire cohort. In dark red is indicated per SIM subtype the cluster with the lowest median number of 1 bp SIMs or the lowest percentage of recurrence for 'within cluster' and 'entire cohort'. In light red the second lowest is indicated. The highest and second highest are indicated in dark and light green, respectively.

*Figure 3*

Table SF5-3: Overview of the median values across samples for the various layers of annotation. For each annotation the dark and light green boxes indicate the top- and second ranking cluster, respectively. Dark and light red indicate the lowest and second lowest cluster, respectively. For the three medians related to SIMs, two samples without any SIMs are left out. For the median percentage of synonymous SSMs (versus all in CDS), samples without any SSMs in CDS are not included. For the median number of predicted drivers, samples without any driver predicted are excluded.

*Figure 4*

Table SF5-4: Top 3 predicted drivers per cluster. The top 3 are based on the percentage of samples in a cluster in which the gene or locus is considered a driver. Drivers that are found in the top 3 of multiple clusters are coloured.

*Figure 5*

Table SF5-5: Top 3 mutational signatures of type SBS, DBS and ID per cluster. The top 3 are based on the percentage of samples in which the signature is present. The median contribution of the signatures is computed based on only those samples in which the signature is found. If there are multiple signatures present in the same percentage of samples, the one with the highest median contribution is selected. Signatures that are found in the top 3 of multiple clusters are coloured.

The acronyms used in this Figure are as follows: SBS signatures (1): SBS1: spontaneous or enzymatic deamination of 5- methylcytosine to thymine; SBS4: tobacco-smoke exposure; SBS7a/b/d: UV-light exposure; SBS10a/b: polymerase $\varepsilon$ exonuclease domain mutations; SBS13: activity of the AID/APOBEC family of cytidine deaminases; SBS44: defective DNA MMR; SBS5, SBS12, SBS17a, SBS17b, SBS28, SBS40: unknown. DBS signatures (1): DBS1: UV-light exposure; DBS2: tobacco-smoke exposure and other endogenous and/or exogenous muta-gens;DBS3: polymerase $\varepsilon$ exonuclease domain mutations; DBS5: prior chemotherapy treatment with platinum drugs; DBS7, DBS10: defective DNA MMR; DBS11: possibly related to activity of the AID/APOBEC family of cytidine deaminases DBS4, DBS6, DBS9: unknown. ID signatures (1): ID1, ID2: slippage during DNA replication of the replicated DNA strand; ID3: tobacco-smoke exposure; ID8: repair of DNA double strand breaks by non-homologous DNA end-joining mechanisms; ID13: UV-light exposure; ID5, ID9, ID14: unknown.

*Figure 6*

Table 1: Key Characteristics of the 16 Clusters. Tumour types that form together ≥50% of the cluster are listed. The key characteristics are those features with the strongest significantly negative or positive association with the cluster. Only if the association with overall recurrence is significant, the respective direction is indicated. [1]Cluster has a low median number of SSMs (<200) and SIMs (<20).

*Figure 7*

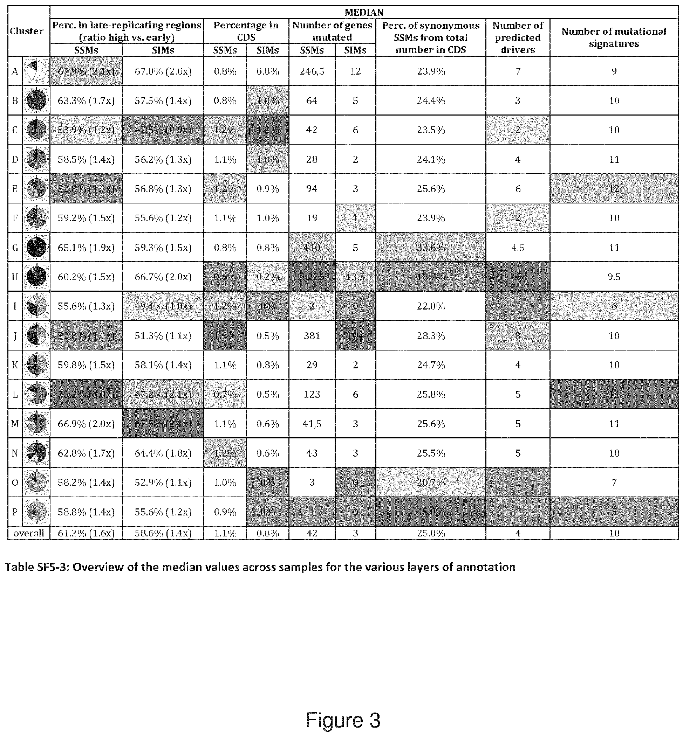

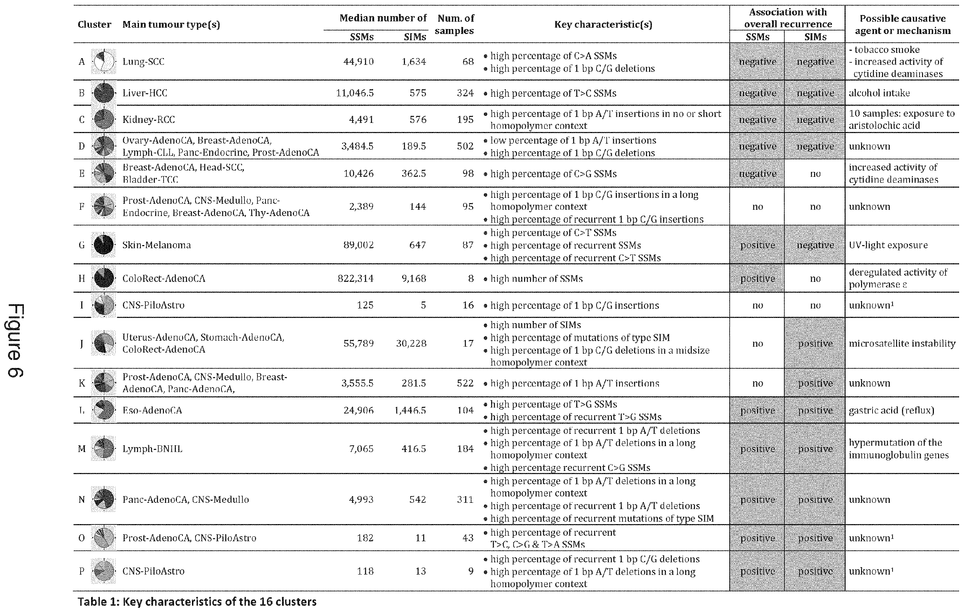

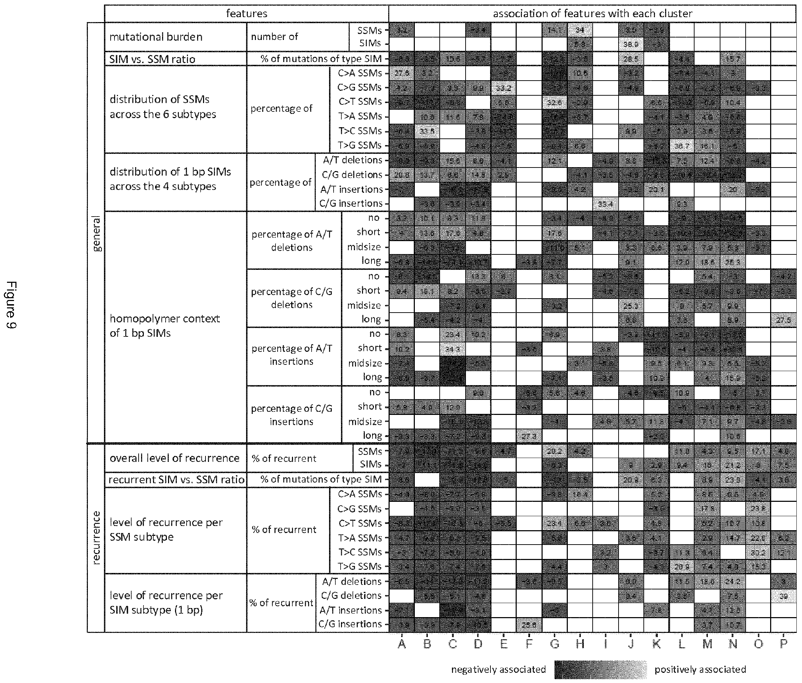

None of the three key factors (middle panel) that impact on recurrence individually explain the observed level of recurrence in the clusters. Whether a cluster has a relatively high or a comparatively lower mutational load is based on the median number of SSMs/SIMs across its samples (Figure 6: Table 1). For cluster M there is a specific sequence context as well, similar to cluster L, but one bp shorter (T>G SSMs, AC̲TT) (not shown). For SIMs a homopolymer of A/T's is used to represent any type of homopolymer. Clusters A and C̄ have a positive association to no and/or short homopolymer context for all types of 1 bp SIMs (red), while for clusters J, L and M this is the case for midsize and/or long homopolymer context (green) (Figure 9). For the replication time region we compute the percentage of SSMs/SIMs that are in late-replicating regions (Figure 3: Table SF5-3). If this percentage is between 45-55%, then we consider the mutations to be nearly equally spread between early- and late-replicating regions of the genome. The specific region that is enriched in cluster M refers to the immunoglobulin genes. The recurrence in clusters A and G is also likely to be positively impacted by an increased mutation rate in a specific region as the majority of their samples are from a particular tumour type for which this has been reported. For lung cancer (cluster A) the mutation rate is increased in linker DNA (Cell. 2018;175(4):1074-87.e18. doi: 10.1016/j.cell.2018.10.004) and for Skin-Melanoma (cluster G) at active transcription factor binding sites (Nature. 2016;532(7598):264-7. doi: 10.1038/nature17661).

*Figure 8*

Survival data for Lymph-CLL samples in cluster D and M. The bar plots show the number (A) and percentage (B) of samples in the indicated clusters that are marked as alive or deceased. The survival curves without (C) and with (D) confidence interval. The hazard ratio of cluster M is lower than for cluster D (E).

*Figure 9*

Black and light grey squares indicate statistically significant negative and positive associations, respectively, where the gradient indicates the strength of the association. White coloured squares indicate no significant association (adjusted p-value > 0.05). Numbers are the results of a 'v test', included in the FactoMineR package, used to determine which features were significantly associated with each cluster. This test compares the mean of a particular feature in a cluster to the overall mean in the dataset. We corrected the p-values of all 'v tests' for multiple testing using the Benjamini-Yekutieli method. A feature is considered to be significantly associated to a cluster if the adjusted p-value < 0.05. For deletions a 'no homopolymer context' means that the base next to the deleted one is not of the same type. For insertions this refers to a base inserted 5' to either a base of a different type or a single base of the same type. Note that we do not have to consider preceding bases as all SIM calls were left aligned. A short homopolymer context is defined as a 2-4 bp mononucleotide repeat of the same type of base as the 1 bp SIM, midsize is 5-7 bp in length and long $\geq$ 8 bp.

## Detailed description of the invention

In describing the present invention, the following terms will be employed, and are intended to be defined as indicated below.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

"Computer-implemented method" where used herein is to be taken as meaning a method whose implementation involves the use of a computer, computer network or other programmable apparatus, wherein one or more features of the method are realised wholly or partly by means of a computer program.

"Patient" as used herein in accordance with any aspect of the present invention is intended to be equivalent to "subject" and specifically includes both healthy individuals and individuals having a disease or disorder (*e.g.* a proliferative disorder such as a cancer). The patient may be a human, a companion animal (*e.g.* a dog or cat), a laboratory animal (e.g. a mouse, rat, rabbit, pig or non-human primate), an animal having a xenografted or xenotransplanted tumour or tumour tissue (e.g. from a human tumour), a domestic or farm animal (*e.g.* a pig, cow, horse or sheep). Preferably, the patient is a human patient. In some cases, the patient is a human patient who has been diagnosed with, is suspected of having or has been classified as at risk of developing, a cancer.

A "sample" as used herein may be a biological sample, such as a cell-free DNA sample, a cell (including a circulating tumour cell) or tissue sample (e.g. a biopsy), a biological fluid, an extract (*e.g.* a protein or DNA extract obtained from the subject). In particular, the sample may be a tumour sample, a biological fluid sample containing DNA, a blood sample (including plasma or serum sample), a urine sample, a cervical smear, a cerebrospinal fluid (CSF) sample, or a non-tumour tissue sample. It has been found that urine and cervical smears contains cells, and so may provide a suitable sample for use in accordance with the present invention. Other sample types suitable for use in accordance with the present invention include fine needle aspirates, lymph nodes, surgical margins, bone marrow or other tissue from a tumour microenvironment, where traces of tumour DNA may be found or expected to be found. The sample may be one which has been freshly obtained from the subject (e.g. a blood draw) or may be one which has been processed and/or stored prior to making a determination (e.g. frozen, fixed or subjected to one or more purification, enrichment or extractions steps, including centrifugation). The sample may be derived from one or more of the above biological samples via a process of enrichment or amplification. For example, the sample may comprise a DNA library generated from the biological sample and may optionally be a barcoded or otherwise tagged DNA library. A plurality of samples may be taken from a single patient, e.g. serially during a course of treatment. Moreover, a plurality of samples may be taken from a plurality of patients.

The following is presented by way of example and is not to be construed as a limitation to the scope of the claims.

## Examples

## Materials and methods

## 1 PCAWG cohort

We used the cohort of cancer genomes assembled by the PCAWG project (10) of the ICGC and TCGA. For every donor, whole-genome sequencing data was available for a normal-tumour pair and all samples were analysed uniformly. A detailed description of the quality control and mutation calling procedure is provided in the PCAWG marker paper (10). After quality control we are left with 2,583 cancer genomes, covering 37 different tumour types.

## 2. Definition of mutations

For SSMs there are 16 possible subtypes. However, we can neither detect substitutions with a base of the same type (e.g. A>A) nor do we usually know on which strand the (pre-) mutagenic event happened first (e.g. A>C is equivalent to T>G on the other strand). Therefore, we combined the substitutions that are each other's reverse complement and refer to them by the pyrimidine of the mutated base pair: C>A, C>G, C>T, T>A, T>C and T>G. We regarded substitutions directly next to each other (median number across samples: 25) as separate single base events since, aside from the very limited numbers, in several cases the individual callers only supported one single base event, and only the consensus resulted in a multiple base substitution call. For 1 bp SIMs, these are the four subtypes A/T deletions, C/G deletions, A/T insertions and C/G insertions, as analogously to SSMs, we cannot determine on which strand the (pre-) mutagenic event happened first.

## 3. Features describing each cancer genome

We computed 29 general features and 13 related to recurrence (Table A) to characterize different aspects of the somatic mutations in a cancer genome. The general ones comprised the number of SSMs and SIMs (two features), the percentage of SIMs with respect to the total number of mutations (one feature), the distribution of SSMs and SIMs across the different subtypes (six and four features, respectively), and the homopolymer context of 1 bp SIMs for each of the four subtypes (four times four features). Recurrence was captured by the overall level of recurrence of SSMs and SIMs (two features), percentage of recurrent mutations of type SIM (one feature) and recurrence per SSM and SIM subtype (six and four features, respectively). The homopolymer context is not included in the recurrence features, as the number of recurrent SIMs is too low to stratify into 16 additional features. Except for the number of SSMs and SIMs, all other 40 features were in percentages to account for the wide range of absolute mutation numbers across the dataset.

## 4. Principal Component Analysis and Hierarchical Clustering on Principal Components

The R package FactoMineR (v1.41) was used for the PCA followed by the hierarchical clustering on the PCs and k-means clustering (12). All input features for the PCA were scaled and centred to account for their different scales. The first 18 PCs, which together explained over 80% of the variance of the data, were used for hierarchical clustering. The remaining components were assumed to mostly represent noise in the data. As the measure of dissimilarity the Euclidean distance was used and for linkage the Ward criterion. We cut the hierarchical clustering tree at various heights to see a more global down to a more specific division of the samples. Resulting clusters were then used as input for k-means clustering to further refine them. As a consequence, part of the samples were finally assigned to a different cluster than after the hierarchical clustering. A 'v test', included in the FactoMineR package, was used to determine which features were significantly associated with each cluster. This test compares the mean of a particular feature in a cluster to the overall mean in the dataset. We corrected the p-values of all 'v tests' for multiple testing using the Benjamini-Yekutieli method. A feature is considered to be significantly associated to a cluster if the adjusted p-value < 0.05.

## 5. Statistical tests

The correlation between every possible pair of the 42 features was measured by the Spearman's rank correlation coefficient using the R package Hmisc (v4.1-1). Multiple testing correction of the p-values of all correlation tests was done by the Benjamini-Yekutieli method. We used the Wilcoxon rank-sum test with continuity correction as the test of significance for differences in features observed between clusters. The different proportions of sequence motifs between recurrent and non-recurrent SSMs were assessed by using $\chi^2$ tests.

## Method for classifying a new sample:

### 6.1 Step 1 - compute the 42 features

We compute the 29 general features and 13 features capturing recurrence that are described in Table SF2-1. For the 13 features capturing recurrence there are two possible scenarios, either we determine recurrence based solely on the PCAWG cohort or we also include the new sample in this. In scenario 1 a mutation in the new sample will only be considered recurrent if it was already recurrent in the PCAWG cohort. In scenario 2, the consequence is that the new sample that is added will potentially change the values of the 13 recurrence features for the samples already included. Subsequently, it might also change the PCA and clustering. This option will potentially allow us to further improve the classifications as the size of the dataset increases.

**Table SF2-1**

| General features | | | SIMs |
|---|---|---|---|
| | SIM vs. SSM ratio | % of mutations of type SIM | |
| | distribution of SSMs across the 6 subtypes | percentage of | C>A SSMs |
| | | | C>G SSMs |
| | | | C>T SSMs |
| | | | T>A SSMs |
| | | | T>C SSMs |
| | | | T>G SSMs |
| | distribution of 1 bp SIMs across the 4 subtypes | percentage of | A/T deletions |
| | | | C/G deletions |
| | | | A/T insertions |
| | | | C/G insertions |
| | homopolymer context of 1 bp SIMs | % of A/T deletions | no |
| | | | short |
| | | | midsize |
| | | | long |
| | | % of C/G deletions | no |
| | | | short |
| | | | midsize |
| | | | long |
| | | % of A/T insertions | no |
| | | | short |
| | | | midsize |
| | | | long |
| | | % of C/G insertions | no |
| | | | short |
| | | | midsize |
| | | | long |
| | | | SIMs |

(continued)

| Recrrence features | recurrent SIM vs. SSM ratio | | % of recurrent mutations of type SIM | |
|---|---|---|---|---|
| | level of recurrence per SSM subtype | % of recurrent | C>A SSMs |
| | | | C>G SSMs |
| | | | C>T SSMs |
| | | | T>A SSMs |
| | | | T>C SSMs |
| | | | T>G SSMs |
| | | % of recurrent | A/T deletions |
| | level of recurrence per SIM subtype (1 bp) | | C/G deletions |
| | | | A/T insertions |
| | | | C/G insertions |

**6.2 Step 2** - **compute principal components**

In scenario 1 we project the new sample into the existing PCA that was based on the PCAWG cohort. In scenario 2 the PCA is recomputed, now including the new sample. In both cases we retrieve the first 18 principle components either only for the new sample (scenario 1) or also for the samples of the PCAWG cohort (scenario 2).

**6.3 Step 3** - **clustering**

1.3.1 Scenario 1

We use the 18 principal components of the new sample to compute the Euclidean distance to the centre of each of the 16 clusters that we based on the PCAWG cohort. The centre of a cluster is computed by taking the mean of the 18 principle components of the samples in the cluster. The new sample will, in principle, be classified into the cluster that is closest. However, we will show the distances to the other clusters as well, because there are cases in which the difference in distance to other clusters is nearly the same as the closest cluster. We might need to add an additional step to solve these cases for which there is only a minimal difference in distance to one or more other clusters.

6.3.2 Scenario 2

We re-cluster all samples, i.e. the PCAWG cohort plus the new sample, based on the newly computed principal components and we re-evaluate whether we have to split up the samples into 16 clusters or more. The new sample will now be part of the clustering and we can return the description of the newly formed cluster as an output.

**7. Validating the results**

7.1 Leave-one-out validation

As a means to assess the robustness of the clustering and the classification of a new sample we took a leave-one-out strategy. Splitting the cohort into training and test set instead would have left us with too few data points. We considered two cases. One where we leave out one sample and recompute the features related to recurrence as we no longer consider the mutations of the 'left out sample'. This corresponds to **scenario 1** that we described above. In the second case, we leave out one sample, but we do not recompute any of the features. This would be the closest we can get to **scenario 2** were we include a new sample into the cohort, instead of only classifying it into one of the existing 16 clusters. However, we will not use the 'left out sample' in the PCA and subsequent clustering as we would in **scenario 2,** because that will result in the original PCA and clustering. Therefore, in both cases the sample left out is not included in the computation of the PCA and the subsequent clustering. Instead we project it into the PCA that is now based on 2,583 minus one sample. Next, we redo the clustering as we now have 2,582 samples. Then we, as described above, compute for the sample left out the distance to the centre of the 16 clusters we get from the new clustering. We will repeat this process for all 2,583 samples, leaving out one sample at the time.

To evaluate our approach, we have to look at two things. One is to see how stable the clusters are when leaving out one sample. Second is to see whether the left out sample gets assigned to the correct cluster. This cannot simply be evaluated by checking whether the sample is in the correct cluster or not, as clusters may change composition. We will for both situations compute the Jaccard index. This statistic will tell us the similarity in terms of samples between a new cluster and an original cluster that was based on all samples. This statistic takes into account the number of samples the new and original cluster have in common (true positives), and divides this by the number of samples the two clusters have combined. The latter corresponds to the number of true positives plus the number of false negatives and the false positives. The formula to find the index is: Jaccard Index = (the number in both sets) / (the number in either set) * 100.

7.2 Stability of the clusters

For each of the 2,583 rounds we compute the Jaccard index of each of the original clusters with each of the new clusters. Next, for each of the original clusters we take the highest Jaccard index it has with any of the new clusters. We do this for both the case where we recomputed recurrence and the one in which we take the original recurrence statistics. Note that for this comparison the sample left out is assigned to the new cluster that is closest.

7.3 Classification of the sample left out

For each 'left out sample' we take the Jaccard index between the original cluster the sample was in and the new cluster that the sample is closest too. We do this for the case when we recomputed recurrence and when we take the original recurrence statistics.

Conclusions

In summary we conclude that the most stable clusters in all of these analyses are the following six:

- Cluster A (lung cancers - smoking)
- Cluster B (liver cancer)
- Cluster C (kidney cancers)
- Cluster E (increased activity of cytidine deaminases)
- Cluster G (skin melanoma - UV light)
- Cluster J (MSI)

There are another five clusters that are also reasonably stable, although not in all analyses, which are:

- Cluster F (unknown)
- Cluster H (deregulated activity of POLE)
- Cluster I (unknown)
- Cluster L (Eso-AdenoCA and Stomach-AdenoCA - gastric acid)
- Cluster O (unknown)

The remaining five clusters seem to be dependent on the full set of 2,583 cancer genomes:

- Cluster D (second largest cluster with 502 samples)
- Cluster K (largest cluster with 522 samples)
- Cluster M (lymphoid cancers - hypermutation of the immunoglobulin genes)
- Cluster N (pancreatic adenocarcinoma and medulloblastoma)
- Cluster P (nine samples, low mutational burden)

**Annotations on sample level**

On sample level the annotation includes tumour type, MSI status, IGHV mutation status (Lymph-CLL only) and tobacco smoking history of the corresponding donor.

MSI classification

We used two MSI classifications provided by the PCAWG consortium, one by the Mutational Signatures Working Group (unpublished) and one by the Germline Cancer Genome Working Group [55]. The first method, which we will refer to as **MSI-Method 1,** classified a sample MSI if the proportion of mutated microsatellites versus all testable microsatellites was >0.01. The second (**MSI-Method 2**) used the MSIsensor tool [56]. to determine the percentage of mutated microsatellites and adjusted this percentage with a linear model for factors like age at diagnosis, sex and sample purity [55]. Cancer genomes with an adjusted percentage of >3.5% were considered MSI.

**MSI-Method 1** classified 16 samples in the entire dataset as MSI of which 15 belong to cluster J. The MSI-marked sample not assigned to this cluster is a Skin-Melanoma sample, which is assigned to cluster N instead. It has a clearly lower proportion of total (11.0%) and recurrent (33.0%) SIMs compared to samples that are assigned to cluster J (median of 35.1% and 87.5%, respectively), but higher proportions than other Skin-Melanoma samples (median of 1% and 0.4%). The sample does have the characteristic peak of 1 bp C/G deletions in a midsize homopolymer context. The two samples in this cluster **MSI-Method 1** did not identify as MSI, one from ColoRect-AdenoCA and one from Uterus-AdenoCA, have a high degree of correlation with the MSI-annotated samples for all 42 features. This suggests that they could have been misclassified and actually be MSI. In fact, MSI assignment is not always straightforward. **MSI-Method 2** labelled 14 of the 17 samples in this cluster as MSI and an additional five samples from other clusters. Aside from the aforementioned Skin-Melanoma sample, the other four neither have a high percentage of (recurrent) mutations that are SIMs, nor do they show a high percentage of 1 bp C/G deletions in the context of a midsize homopolymer.

IGHV mutation status

The IGHV mutation status of Lymph-CLL samples was retrieved from the original article in which these samples were first described [57].

**Annotations on mutation level**

On mutation level the annotation includes impact classification, functional category, the overlap with predicted drivers linked to the samples, mutational signatures and replication time

The functional impact of a mutation was predicted by Oncotator (5), which was made available by the consortium for 99.9% of the SSMs and 97.3% of the SIMs. We used GENCODE v19 (6) as annotation of the human reference to get the functional category of a mutation. The complete and 'per-patient' list of predicted cancer driver mutations together with the affected gene or regulatory element were provided by the PCAWG Drivers and Functional Interpretation Group (7,8). The mutational signatures and their proposed aetiology were provided by the corresponding working group (1). We downloaded the replication time data in the form of wavelet-smoothed signals of the following five cancer cell lines from the ENCODE project (9): HeLa-S3 (cervical adenocarcinoma), HepG2 (hepatocellular carcinoma), K562 (chronic myelogenous leukaemia), MCF-7 (breast adenocarcinoma) and SK-N-SH (neuroblastoma). The scores were defined for regions of 1 kb in length, but were unavailable for chromosome Y and the chromosomal end regions. Mutations in these regions were left out. Furthermore, samples with less than ten SSMs/SIMs were left out of the density plots. As there was no matching cell line for several tumour types in the PCAWG cohort, we combined the cell lines by taking the median of the scores. To determine the percentage of mutations of a sample that falls into late-replicating regions, we took as a threshold the median score across the summarized scores of the five cell lines

**Identification of peaks of recurrent mutations**

Specifically for the Lymph-BNHL and Lymph-CLL samples in cluster M, we identified peaks of recurrent mutations by sliding a window across the genome and computing per window the number of recurrent mutations, using the entire cohort to define recurrence. The size of a window was set to 11,415 bp such that it would contain on average ten recurrent mutations if all recurrent mutations (SSMs and SIMs) were equally distributed along the genome. At each step the window was shifted by half its size.

**Examples**

**Overview of findings**

**Recurrence characteristics divide the cohort into clusters**

We used the 42 recurrence-based and general mutational features all together to see if we could uncover meaningful clusters of cancer genomes. As there are strong correlations between some of these features, we first performed a principal component analysis (PCA) to obtain independent features and reduce dimensionality. As explained in the methods, we took the first 18 principal components (PCs) of the PCA, which together explain >80% of the variance in the data, as features for hierarchical clustering followed by k-means clustering (first three PCs) (12). To get a pan-cancer perspective we analysed all samples together.

The crude division into two clusters separates the cancer genomes with low relative levels of recurrent SIMs (e.g. Liver-HCC, Kidney-RCC and Lung-SCC) from those with high levels (e.g. ColoRect-AdenoCA, Eso-AdenoCA, Lymph-BNHL and Panc-AdenoCA). At three clusters, the relative level of recurrent SSMs splits off a group of mainly Skin-Melanoma samples from the two other clusters. By further increasing the number of clusters we obtain more detailed patterns as clusters become more specific to a tumour type or a particular mutational process. We will discuss in further detail the division into 16 clusters, chosen as a trade-off between too many clusters, which would each be specific to just a handful of samples, and too few, which would result in loss of meaningful information (Figure 6: Table 1). There are nine clusters (A, B, C, G, H, I, L, M and P) for which at least half of the samples are from the same tumour type. For another two clusters (O and N) samples from two tumour types constitute a majority. In the remaining five clusters (D, E, F, J and K) three or more tumour types are required for this. For each tumour type the percentage of samples in each of the 16 clusters was calculated (not shown). The association of each of the 42 features with the clusters is shown in Figure 9. The key characteristics of each cluster are shown in Figure 6: Table 1. To facilitate a tight linkage of the clusters to mutational processes, we consider, in addition to the mutational features of a cancer genome, also tumour type assignment, microsatellite instability (MSI) status, immunoglobulin heavy-chain variable region gene (IGHV) mutation status (Lymph-CLL only) and tobacco smoking history of the donor (where available). To provide further details on each cluster we integrate annotation based on GENCODE (13), Oncotator (14), driver predictions (3,15), replication time (16) and mutational signatures (17). In the following examples we will show how the level of recurrence can be indicative of the mutational processes, often in combination with the general features. Moreover, we show that our recurrence-based approach groups cancer genomes in a novel way that complements current classification approaches and captures clinically actionable cancer phenotypes.

**Overview of clinically relevant findings: The present method enables the identification of clinically relevant clusters**

Several of our clusters are linked to cancer phenotypes that are relevant for treatment and/or have prognostic value. Our division into 16 clusters and their characteristics may therefore be valuable for complementing current classification schemes, which are mainly based on histology and organ of origin.

One example illustrating the value of our clusters towards this goal is the MSI phenotype linked to cluster J (Example 1). For these patients, immunotherapy may be beneficial (32) while adjuvant chemotherapy may not be needed (33). Briefly, a sample would be classified as MSI in our approach if we observe, among others, a high percentage of 1 bp C/G deletions in a midsize homopolymer context plus a strong shift towards the presence of SIMs compared to SSMs resulting in a high absolute and relative number of SIMs. Also a high percentage of recurrent SIMs and a relatively equal distribution of mutations between early- and late-replicating regions are typical. These characteristics of cluster J form a possible alternative to either explicitly identifying all microsatellite alterations between tumour and normal tissue (34) or using specific markers to detect alterations in five or seven of them like the Bethesda markers (35).

A second example of an actionable phenotype is ultra-hypermutation (cluster H, Example 2), which has also been related to beneficial results from immunotherapy (36,37). Samples of this cluster have a high mutational load as well, but form a mirror image to the MSI samples in terms of other characteristics like a high absolute and relative number of SSMs, and high levels of recurrent SSMs. Also there is a significantly higher percentage of mutations in late-replicating regions than for cluster J (SSMs: 60.2% vs. 52.8%, p=0.0011, SIMs: 66.7% vs. 51.3%, p=1.8e-06).

A third example of a relevant phenotype captured with our clustering approach is the somatic hypermutation of the immunoglobulin genes, which identifies memory B-cells as the cell of origin in the case of lymphomas (Example 3). This has been linked to a less aggressive form of Lymph-CLL and more favourable prognosis (28), which may in turn influence treatment selection. Without explicitly analysing the immunoglobulin genes (38), we were largely able to separate the Lymph-CLL samples with somatic hypermutation (cluster M) from those without (cluster D). The characteristics of the former group include a high percentage of recurrent C>G SSMs and 1 bp A/T deletions. A final example relates to those Eso-AdenoCA samples that are assigned to cluster L, which have a high percentage of T>C as well as T>G SSMs and a higher total mutational load than Eso-AdenoCA samples not assigned to this cluster. Eso-AdenoCA samples with the characteristics of cluster L have also been suggested to benefit from immunotherapy (39). The same treatment option may therefore be prioritized for the 22 Stomach-AdenoCA samples that are also in cluster L. Similarly, a refined investigation of tumour samples that do not cluster with the vast majority of its own kind may ideally point to differences in disease prognosis or treatment response and even has the potential to define novel subtypes or reveal misclassification. Such an analysis would be especially worthwhile for the ~20% or less samples from Kidney-RCC, Liver-HCC, Lung-SCC or Lymph-BNHL that are not assigned to the main cluster.

Based on these findings it is clear that a further application of our classification scheme is to assign a metastatic sample with unknown primary site to a cluster to shed light on the possible tissue of origin or pan-cancer characteristics like MSI.

**Example 1: High levels of recurrent SIMs characterize microsatellite instability (MSI)**

The highest level of recurrent SIMs across all clusters is observed for cluster J, which could be linked to a defective mismatch repair (MMR) pathway resulting in MSI. Of the 179,691 recurrent 1 bp SIMs in the entire cohort, almost half of them are recurrent when only considering this cluster. The very high median number of SIMs (30,228) in this cluster may play a role in the high level of recurrence. The key factor, however, is most likely the mutational process behind MSI, which is slipping of the DNA polymerase during replication of repetitive sequences and the lack of repair by the MMR pathway (21). This not only explains the elevated number of SIMs (22), but also the association of this cluster with all SIM subtypes in the context of midsize-to-long homopolymers. As such homopolymers are scarce in the genome, the shift towards specifically altering them increases the probability of recurrence. Especially striking in this cluster is the proportion of 1 bp C/G deletions that are in the context of a midsize homopolymer (median: 73.2% vs. 8.4% for the other clusters combined, p=1.2e-12). This translates to 6.0% recurrent 1 bp C/G deletions within this cluster versus <0.7% for any other cluster. Further details of this cluster are provided in the detailed cluster-specific descriptions, below.

**Example 2: High levels of recurrent SSMs characterize deregulated activity of POLE**

A high level of recurrent SSMs also characterizes cluster H, specifically C>T and C>A SSMs. This cluster captures samples that can be considered ultra-hypermutators and their mutations are mainly caused by deregulated activity of POLE. These samples have a very high total number of C>A SSMs (median: 297,750) and the median percentage of recurrent C>A SSMs across the samples is 7.7%. Two thirds of all recurrent C>A SSMs in the entire cohort are also recurrent within only this cluster. The recurrent C>A mutations of this cluster are enriched for the motif TTCTTT, when considering only ungapped motifs. Of the recurrent C>A SSMs 29.6% are at this motif, while for non-recurrent ones this is true for only 13.7% ($\chi^2$ test: p<2.2e-16). In the genome, the estimated percentage of this motif of all corresponding 6-mers (NNCNNN) is far smaller (0.6%), suggesting that effects of deregulated activity of POLE are most likely dependent on a sequence context exceeding a single neighbouring base on each side.
Further details of this cluster are provided in the detailed cluster-specific descriptions, below.

**Example 3: Gastric acid-exposure and hypermutation of immunoglobulin genes have a positive association with recurrence of SSMs and SIMs.**

Clusters L, M and N all positively associate with recurrence of both SSMs and SIMs.

Cluster L, which for >80% consists of Eso-AdenoCA and Stomach-AdenoCA samples, can potentially be linked to gastric acid-exposure.

The T>G and T>C SSMs that are recurrent within this cluster cover 45% and ~20%, respectively, of the total observed in the whole cohort. The median percentage of SSMs falling in late-replicating regions is significantly higher than in the rest of the clusters combined (75.2% vs. 61.0%, p<2.2e-16). In general, the mutational load is expected to be higher in late-replicating regions as the MMR pathway is said to be less efficient there (23). However, the question is why the effect is so strong in cluster L compared to the others. It could be that transient single strand-DNA at stalled replication forks, whose formation has been suggested to be more prevalent in late-replicating regions (24), is particularly vulnerable to the mutagenicity of acid-exposure. Alternatively, if the oxidative stress induced by gastric acid-exposure leads to the oxidation of dG in the dNTP pool (25), the use of error-prone DNA polymerases that incorporate the oxidized dG into the DNA (26) may be more frequent in late-replicating regions (27). The strong shift towards late-replicating regions favours higher levels of recurrence. The same holds for the enrichment of the specific sequence context 'AACTT' that we observe for recurrent T>G mutations in this cluster. Nearly 36% of the recurrent T>G SSMs are confined to this motif and ~12% of the non-recurrent ones (p<2.2e-16), which is still far higher than the estimated percentage of this motif in the genome (0.5% of all NNNTN 5-mers). For SIMs, the cluster has a positive association with recurrence for three out of the four SIM subtypes as well as with the same subtypes in a midsize and/or long homopolymer context. This suggests similar mechanisms as for cluster J. Finally, as observed for SSMs in this cluster, SIMs also show a tendency to fall into late-replicating regions (67.2%, Figure 3: Table SF5-3). This may further add to the high level of recurrence for SIMs.

Cluster M, with mainly Lymph-BNHL and Lymph-CLL samples, is linked to the somatic hypermutation of the immunoglobulin genes.

In the aforementioned tumour types, this process is indicative of memory B cells being the cell of origin as opposed to naive B cells (28). The cluster has positive associations with the level of recurrence for all six SSM subtypes. The association is particularly strong for C>G. Of all recurrent C>G SSMs, 10.7% can be found in this cluster alone. The high level of recurrence may partially be explained by the hypermutation observed in the limited area of the genome where the immunoglobulin genes are located. For SIMs, the cluster has positive associations with the level of recurrence for all four subtypes as well as with those subtypes in general when in a midsize and/or long homopolymer context. This cluster has the highest median percentage of SIMs in late-replicating regions (67.5% vs. 57.8% for the other cluster combined, p<2.2e-16, Figure 3: Table SF5-3), which may contribute to the high level of recurrence.

In cluster N, which consists of ~47% Panc-AdenoCA samples, the sources of mutagenesis are less clear, even after the inclusion of all annotation layers. Except for C>G and T>C SSMs, the cluster has positive associations with the recurrence of all other subtypes of SSMs and every SIM subtype. This is especially noticeable as the median of the total number of mutations across samples is intermediate. A high percentage of the recurrent mutations are SIMs in this cluster with a median of 35.0%. This is far higher than for samples of the other clusters combined (median: 15.5%, p<2.2e-16). The positive associations with all SIM subtypes when in a midsize-to-long homopolymer context may point to a slippage-related mechanism (see also cluster J).

Further details of these clusters are provided in the detailed cluster-specific descriptions, below.

**Example 4: Tobacco-smoke exposure, alcohol use and increased activity of cytidine deaminases all have a negative association with recurrence.**

There are also several mutagenic processes that are associated with low levels of recurrence including those represented by clusters A, B, C and E. Cluster A, of which 84% are lung cancer samples, is linked to mutational processes induced by tobacco-smoke exposure. This cluster shows a positive association with the total number of SSMs and the percentage of C>A SSMs, the latter is a known consequence of tobacco-smoke exposure (29). However, the high total mutational load together with the high percentage of C>A SSMs does not translate into elevated levels of recurrence. In agreement, there is little tendency to favour a specific sequence context for the C>A SSMs in contrast to the ultra-hypermutators in cluster H. This can also be observed in the 'tobacco smoking signature' (9), which is present in nearly 90% of the samples in this cluster. There are several factors that increase the probability of recurrence in this cluster, including the high total mutational load together with the high percentage of C>A SSMs and the enrichment of mutations in late-replicating regions. Also, tobacco-smoke induced mutations have been shown to be enriched in linker DNA (i.e. DNA not wrapped around a nucleosome) [52], which constitute only between 10% and 25% of the genome in eukaryotes [53]. The key to explaining the lack of recurrence seems to be that there is little tendency to favour a specific sequence context for the C>A SSMs.

Unlike for several clusters mentioned above, there is a positive association with SIMs in short homopolymer contexts, which are more frequent in the genome than longer homopolymers, and the resulting distribution is therefore also more random. Note that cluster A also has a strong association with the percentage of total 1 bp C/G deletions, which has not been described previously as a possible consequence of tobacco-smoke exposure [54].

Cluster B, consisting of 85% Liver-HCC samples, is likely to be linked to mutational processes indirectly induced by excessive alcohol use. The level of recurrence is low despite the high number of samples of the same tumour type (277) and the consistent pattern of a high percentage of T>C SSMs (median: 31.7% vs. 14.6% in the other cluster combined, p<2.2e-16). With regard to 1 bp SIMs, there is a positive association with a short homopolymer context, as for cluster A, with the exception of 1 bp A/T insertions.

In cluster C, in which ~82% are Kidney-RCC and Kidney-ChRCC samples, the mutational processes remain largely obscure except for a few samples that can be connected to aristolochic acid exposure. Unlike for clusters A and B, the median number of SSMs across samples is relatively low. Furthermore, mutations are nearly equally spread between early- and late-replicating regions as only 53.9% of the SSMs and 47.5% of SIMs are in late (Figure 3: Table SF5-3). SIMs are preferentially located in no or short homopolymer context, similar to clusters A and B.

In cluster E nearly one third are Breast-AdenoCA samples and key mutational characteristics point to the endogenous mutational process of increased activity of cytidine deaminases. There is a general paucity of associations with characteristics of recurrence. In line with this, the mutations in this cluster are nearly equally spread between early- and late-replicating regions of the genome (Figure 3: Table SF5-3). The most outstanding feature of this cluster is the high percentage of C>G SSMs. This is the rarest substitution type, making the detection of recurrence unlikely, particularly if not confined to specific genomic regions. Interestingly though, the recurrent C>G SSMs are enriched for the motif CTCW (W = A or T). Very similar motifs have been described as being characteristic for deamination mediated by APOBEC3 (30). The difference between the percentage of recurrent and non-recurrent mutations at this motif (42.1% vs. 32.8%, respectively, $\chi^2$ test: p<2.2e-16) is not as large as for other motifs discussed. The CTCW motif is also shorter, more general and therefore relatively frequent in the genome (5.4% of all NNCN 4-mers), all possible causes for the lacking trend towards recurrence. Further details of these clusters are provided in the detailed cluster-specific descriptions, below.

**Example 5: High levels of recurrent SSMs and low levels of recurrent SIMs characterize exposure to UV light**

A positive association with overall recurrence of SSMs and more specifically with recurrence of C>T SSMs characterizes cluster G that mainly consists of Skin-Melanoma samples (Fig. 9). The association is negative with the recurrence of SIMs. We link this cluster to mutagenesis induced by UV light. The samples assigned to cluster G account by themselves for 60.7% of the total number of recurrent C>T SSMs. The combination of the high total number of SSMs per sample and the high percentage of C>T substitutions in this cluster is what contributes to the high level of recurrence. The mechanisms inherent to UV-light exposure further increase the probability of recurrence as it tends to result in C>T SSMs near energy sinks in the genome. The energy from UV-light-exposed DNA usually travels along the DNA strand until it arrives at the lowest energy point, a dT, particularly when it is next to a dC, which acts as energy barrier (18). In agreement with this, there is a strong enrichment of recurrent C>T mutations in a TTCCT motif (the underlined C is mutated). While the percentage of this motif in the genome is estimated to be only 1.0% of all 5-mers with a C at the central position, 8.4% and 21.9% of the non-recurrent and recurrent C>T SSMs, respectively, of this cluster are at this motif. An enrichment of a CTTCCG motif was found for frequently recurrent mutations in promoters in 38 melanoma samples (19). Both these specific sequence contexts help explain the observed high level of recurrence. Furthermore, a decreased activity level of the nucleotide excision repair pathway was detected in melanoma at active transcription factor binding sites and nucleosome embedded DNA compared to the flanking sites (20). This increases local mutation rates and hence also increases the probability of recurrence.

**Example 6: Patient Survival Differentiated by Cluster**

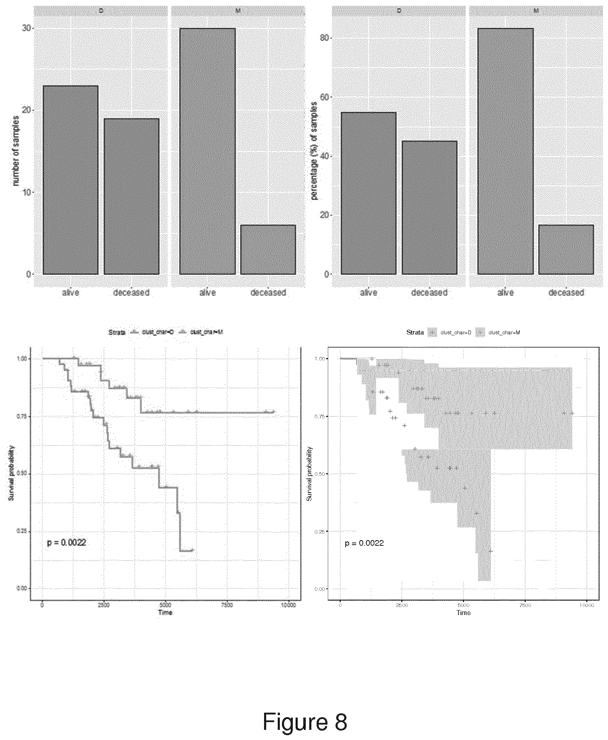

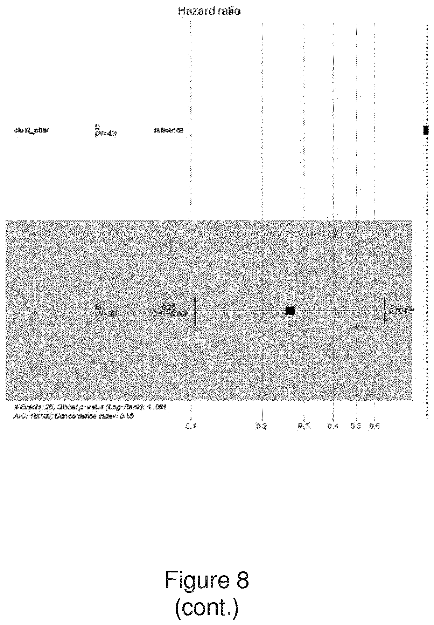

The present inventors also investigated patient survival and/or treatment response by cluster. There is limited information available for the PCAWG cohort. For about 26% of donors we know they received therapy before or after the sample for sequencing was obtained. Treatment response can be obtained for 11% of all donors. In terms of available survival data, two thirds of the donors were alive at the moment of the last follow-up that was recorded and provided to the database. In a typical survival analysis these would all be what is called 'censored' at the time point of the last follow up, which will have an impact on the power we have to detect differences in survival. For the 28% of donors who have passed away, we have for nearly all the survival time. Taken together we have the survival time or time to last follow up for nearly 73% of the donors. One issue is that survival time is possibly more determined by tumour type than anything else and for nine of the sixteen clusters the majority of the samples are from the same tumour type. If we would do a survival analysis on the cluster level, this will likely be dominated by the difference in survival between tumour types. To overcome this, we would have to analyse per tumour type if there is a difference in survival between clusters the samples have been assigned to. However, for 24 of the 37 tumour types more than half the samples are assigned to one single cluster. Moreover, the number of samples and survival data is severely limited when doing the analysis per tumour type. Nevertheless we conducted preliminary analysis of the survival data. For 14 tumour types we have samples in more than one cluster and at least 10 samples in each. We studied survival for these 14 tumour types to see if there are differences between clusters. Here we only take those clusters the tumour type had at least 10 samples in. For four tumour types there is a significant difference in survival between clusters, which are Eso-AdenoCA, Lymph-CLL, Ovary-AdenoCA and Skin-Melanoma. The clearest difference we observed for Lymph-CLL (Cluster M longer survival vs. Cluster D; p = 0.0022; see Figure 8), which coincides with the known differences in survival for patients with and without hypermutation of the immunoglobulin heavy-chain variable region genes.

Further details of this cluster are provided in the detailed cluster-specific descriptions, below.

**References**

1. Fredriksson NJ, Ny L, Nilsson JA, Larsson E. Systematic analysis of noncoding somatic mutations and gene expression alterations across 14 tumor types. Nat Genet 2014;46:1258 doi 10.1038/ng.3141.

2. Ostrow SL, Barshir R, DeGregori J, Yeger-Lotem E, Hershberg R. Cancer Evolution Is Associated with Pervasive Positive Selection on Globally Expressed Genes. PLoS Genet 2014;10(3):e1004239 doi 10.1371/journal.pgen.1004239.

3. Sabarinathan R, Pich O, Martincorena I, Rubio-Perez C, Juul M, Wala J, et al. The whole-genome panorama of cancer drivers. bioRxiv 2017 doi 10.1101/190330.

4. Tomasetti C, Marchionni L, Nowak MA, Parmigiani G, Vogelstein B. Only three driver gene mutations are required for the development of lung and colorectal cancers. Proc Natl Acad Sci USA 2015;112(1):118-23 doi 10.1073/pnas.1421839112.

5. Martincorena I, Fowler JC, Wabik A, Lawson ARJ, Abascal F, Hall MWJ, et al. Somatic mutant clones colonize the human esophagus with age. Science 2018;362(6417):911-7 doi 10.1126/science.aau3879.

6. Ciccarelli FD. Mutations differ in normal and cancer cells of the oesophagus. Nature 2019;565(7739):301-3 doi 10.1038/d41586-018-07737-8.

7. Woo YH, Li W-H. DNA replication timing and selection shape the landscape of nucleotide variation in cancer genomes. Nat Commun 2012;3:1004 doi 10.1038/ncomms1982.

8. Polak P, Karlic R, Koren A, Thurman R, Sandstrom R, Lawrence MS, et al. Cell-of-origin chromatin organization shapes the mutational landscape of cancer. Nature 2015;518(7539):360-4 doi 10.1038/nature14221.

9. Alexandrov LB, Nik-Zainal S, Wedge DC, Aparicio SAJR, Behjati S, Biankin AV, et al. Signatures of mutational processes in human cancer. Nature 2013;500(7463):415-21 doi 10.1038/nature12477.

10. Campbell PJ, Getz G, Stuart JM, Korbel JO, Stein LD. Pan-cancer analysis of whole genomes. bioRxiv 2017 doi 10.1101/162784.

11. Ding L, Bailey MH, Porta-Pardo E, Thorsson V, Colaprico A, Bertrand D, et al. Perspective on Oncogenic Processes at the End of the Beginning of Cancer Genomics. Cell 2018;173(2):305-20.e10 doi 10.1016/j.cell.2018.03.033.

12. Lê S, Josse J, Husson F. FactoMineR: An R Package for Multivariate Analysis. Journal of Statistical Software 2008;25(1):18 doi 10.18637/jss.v025.i01.

13. Harrow J, Frankish A, Gonzalez JM, Tapanari E, Diekhans M, Kokocinski F, et al. GENCODE: the reference human genome annotation for The ENCODE Project. Genome research 2012;22(9):1760-74 doi 10.1101/gr.135350.111.

14. Ramos AH, Lichtenstein L, Gupta M, Lawrence MS, Pugh TJ, Saksena G, et al. Oncotator: Cancer Variant Annotation Tool. Hum Mutat 2015;36(4):E2423-E9 doi 10.1002/humu.22771.

15. Rheinbay E, Nielsen MM, Abascal F, Tiao G, Hornshøj H, Hess JM, et al. Discovery and characterization of coding and non-coding driver mutations in more than 2,500 whole cancer genomes. bioRxiv 2017 doi 10.1101/237313.

16. Hansen RS, Thomas S, Sandstrom R, Canfield TK, Thurman RE, Weaver M, et al. Sequencing newly replicated DNA reveals widespread plasticity in human replication timing. Proc Natl Acad Sci USA 2010;107(1):139-44 doi 10.1073/pnas.0912402107.

17. Alexandrov L, Kim J, Haradhvala NJ, Huang MN, Ng AWT, Boot A, et al. The Repertoire of Mutational Signatures in Human Cancer. bioRxiv 2018:322859 doi 10.1101/322859.

18. Gut IG, Wood PD, Redmond RW. Interaction of Triplet Photosensitizers with Nucleotides and DNA in Aqueous Solution at Room Temperature. J Am Chem Soc 1996;118(10):2366-73 doi 10.1021/ja9519344.

19. Fredriksson NJ, Elliott K, Filges S, Van den Eynden J, Ståhlberg A, Larsson E. Recurrent promoter mutations in melanoma are defined by an extended context-specific mutational signature. PLoS Genet 2017;13(5):e1006773 doi 10.1371/journal.pgen.1006773.

20. Sabarinathan R, Mularoni L, Deu-Pons J, Gonzalez-Perez A, López-Bigas N. Nucleotide excision repair is impaired by binding of transcription factors to DNA. Nature 2016;532(7598):264-7 doi 10.1038/nature17661.

21. Ellegren H. Microsatellites: simple sequences with complex evolution. Nat Rev Genet 2004;5:435 doi 10.1038/nrg1348.

22. Sun JX, Helgason A, Masson G, Ebenesersdóttir SS, Li H, Mallick S, et al. A direct characterization of human mutation based on microsatellites. Nat Genet 2012;44:1161 doi 10.1038/ng.2398.

23. Supek F, Lehner B. Differential DNA mismatch repair underlies mutation rate variation across the human genome. Nature 2015;521(7550):81-4 doi 10.1038/nature14173.

24. Yang Y, Sterling J, Storici F, Resnick MA, Gordenin DA. Hypermutability of Damaged Single-Strand DNA Formed at Double-Strand Breaks and Uncapped Telomeres in Yeast Saccharomyces cerevisiae. PLoS Genet 2008;4(11):e1000264 doi 10.1371/journal.pgen.1000264.

25. Tomkova M, Tomek J, Kriaucionis S, Schuster-Böckler B. Mutational signature distribution varies with DNA replication timing and strand asymmetry. Genome Biology 2018;19(1):129 doi 10.1186/s13059-018-1509-y.

26. Kamiya H. Mutations Induced by Oxidized DNA Precursors and Their Prevention by Nucleotide Pool Sanitization Enzymes. Genes and Environment 2007;29(4):133-40 doi 10.3123/jemsge.29.133.

27. Seplyarskiy VB, Bazykin GA, Soldatov RA. Polymerase ζ Activity Is Linked to Replication Timing in Humans: Evidence from Mutational Signatures. Molecular Biology and Evolution 2015;32(12):3158-72 doi 10.1093/molbev/msv184.

28. Hamblin TJ, Davis Z, Gardiner A, Oscier DG, Stevenson FK. Unmutated Ig VH Genes Are Associated With a More Aggressive Form of Chronic Lymphocytic Leukemia. Blood 1999;94(6):1848-54.

29. Pfeifer GP, Denissenko MF, Olivier M, Tretyakova N, Hecht SS, Hainaut P. Tobacco smoke carcinogens, DNA damage and p53 mutations in smoking-associated cancers. Oncogene 2002;21:7435 doi 10.1038/sj.onc.1205803.

30. Chan K, Roberts SA, Klimczak LJ, Sterling JF, Saini N, Malc EP, et al. An APOBEC3A hypermutation signature is distinguishable from the signature of background mutagenesis by APOBEC3B in human cancers. Nat Genet 2015;47:1067 doi 10.1038/ng.3378.

31. Lang GI, Murray AW. Mutation Rates across Budding Yeast Chromosome VI Are Correlated with Replication Timing. Genome Biology and Evolution 2011;3:799-811 doi 10.1093/gbe/evr054.

32. Xiao Y, Freeman GJ. The Microsatellite Instable (MSI) Subset of Colorectal Cancer is a particularly good candidate for checkpoint blockade immunotherapy. Cancer discov 2015;5(1):16-8 doi 10.1158/2159-8290.CD-14-1397.

33. Saridaki Z, Souglakos J, Georgoulias V. Prognostic and predictive significance of MSI in stages II/III colon cancer. World J Gastroenterol 2014;20(22):6809-14 doi 10.3748/wjg.v20.i22.6809.

34. Niu B, Ye K, Zhang Q, Lu C, Xie M, McLellan MD, et al. MSIsensor: microsatellite instability detection using paired tumor-normal sequence data. Bioinformatics 2014;30(7):1015-6 doi 10.1093/bioinformatics/btt755.

35. Umar A, Boland CR, Terdiman JP, Syngal S, Chapelle Adl, Ruschoff J, et al. Revised Bethesda Guidelines for Hereditary Nonpolyposis Colorectal Cancer (Lynch Syndrome) and Microsatellite Instability. J Natl Cancer Inst 2004;96(4):261-8 doi 10.1093/jnci/djh034.

36. Schlesner M, Eils R. Hypermutation takes the driver's seat. Genome Med 2015;7(1):31 doi 10.1186/s13073-015-0159-x.

37. Heong V, Ngoi N, Tan DSP. Update on immune checkpoint inhibitors in gynecological cancers. J Gynecol Oncol 2017;28(2):e20 doi 10.3802/jgo.2017.28.e20.

38. Puente XS, Bea S, Valdes-Mas R, Villamor N, Gutierrez-Abril J, Martin-Subero JI, et al. Non-coding recurrent mutations in chronic lymphocytic leukaemia. Nature 2015;526(7574):519-24 doi 10.1038/nature14666.

39. Secrier M, Li X, de Silva N, Eldridge MD, Contino G, Bornschein J, et al. Mutational signatures in esophageal adenocarcinoma define etiologically distinct subgroups with therapeutic relevance. Nat Genet 2016;48:1131 doi 10.1038/ng.3659.

40. Wagih O. ggseqlogo: a versatile R package for drawing sequence logos. Bioinformatics 2017;33(22):3645-7 doi 10.1093/bioinformatics/btx469.

45: Fan Y, Xi L, Hughes DST, Zhang J, Zhang J, Futreal PA, et al. MuSE: accounting for tumor heterogeneity using a sample-specific error model improves sensitivity and specificity in mutation calling from sequencing data. Genome Biol. 2016;17(1):178. doi: 10.1186/s13059-016-1029-6.

46: Moncunill V, Gonzalez S, Bea S, Andrieux LO, Salaverria I, Royo C, et al. Comprehensive characterization of complex structural variations in cancer by directly comparing genome sequence reads. Nat Biotechnol. 2014;32:1106. doi: 10.1038/nbt.3027.

47: Costello M, Pugh TJ, Fennell TJ, Stewart C, Lichtenstein L, Meldrim JC, et al. Discovery and characterization of artifactual mutations in deep coverage targeted capture sequencing data due to oxidative DNA damage during sample preparation. Nucleic Acids Res. 2013;41(6):e67-e.doi:10.1093/nar/gks1443.

48: Campbell PJ, Getz G, Stuart JM, Korbel JO, Stein LD. Pan-cancer analysis of whole genomes. bioRxiv. 2017. doi: 10.1101/162784.

49: Whalley JP, Buchhalter I, Rheinbay E, Raine KM, Kleinheinz K, Stobbe MD, et al. Framework For Quality Assessment Of Whole Genome, Cancer Sequences. bioRxiv. 2017. doi: 10.1101/140921

50. Schneider TD, Stormo GD, Gold L, Ehrenfeucht A. Information content of binding sites on nucleotide sequences. J Mol Biol. 1986;188(3):415-31. doi: 10.1016/0022-2836(86)90165-8.

51. Kullback S, Leibler RA. On Information and Sufficiency. Ann Math Statist. 1951;22(1):79-86.

52. Pich O, Muiños F, Sabarinathan R, Reyes-Salazar I, Gonzalez-Perez A, Lopez-Bigas N. Somatic and Germline Mutation Periodicity Follow the Orientation of the DNA Minor Groove around Nucleosomes. Cell. 2018;175(4):1074-87.e18. doi: 10.1016/j.cell.2018.10.004.

53. Segal E, Fondufe-Mittendorf Y, Chen L, Thåström A, Field Y, Moore IK, et al. A genomic code for nucleosome positioning. Nature. 2006;442(7104):772-8. doi: 10.1038/nature04979

54. Alexandrov L, Kim J, Haradhvala NJ, Huang MN, Ng AWT, Boot A, et al. The Repertoire of Mutational Signatures in Human Cancer. bioRxiv. 2018:322859. doi: 10.1101/322859.

55. Waszak SM, et al. Germline determinants of the somatic mutation landscape in 2,642 cancer genomes. bioRxiv 2017

56. Niu B, et al. MSIsensor: microsatellite instability detection using paired tumor-normal sequence data. Bioinformatics 2014 ;30(7):1015-6

57. Puente XS, et al. Non-coding recurrent mutations in chronic lymphocytic leukaemia. Nature 2015;526(7574):519-24

**Detailed cluster-specific descriptions.**

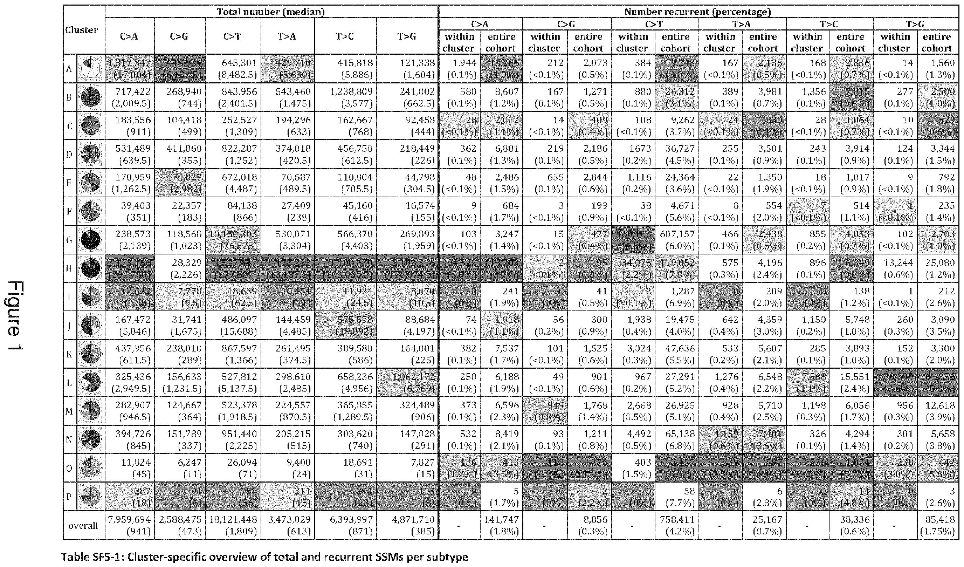

Here we describe in more detail the level of recurrence, the mutational pattern observed and possible mechanisms associated with each cluster (Figure 6: Table 1). We will describe only the most characteristic features for each cluster. An overview of the levels of recurrence within each cluster and across the entire cohort is shown in Figure 1:

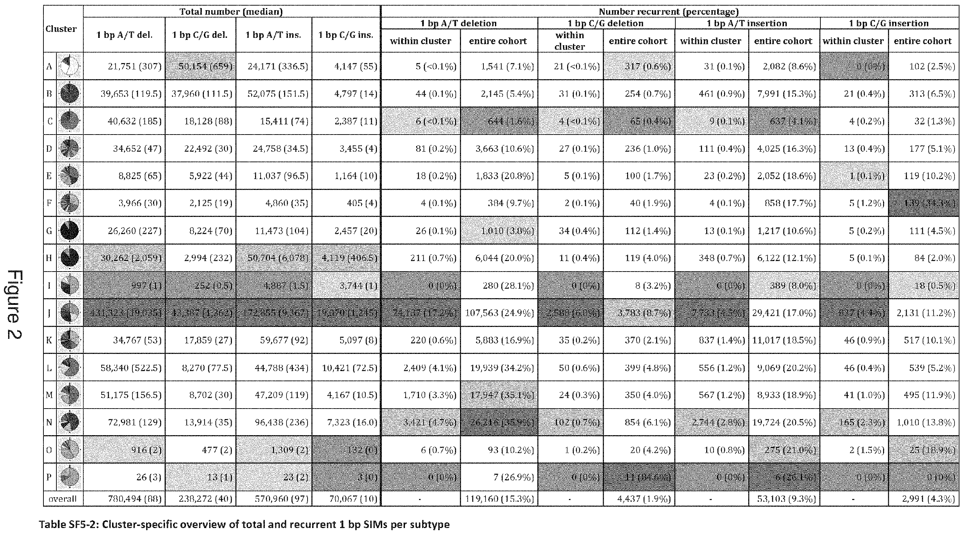

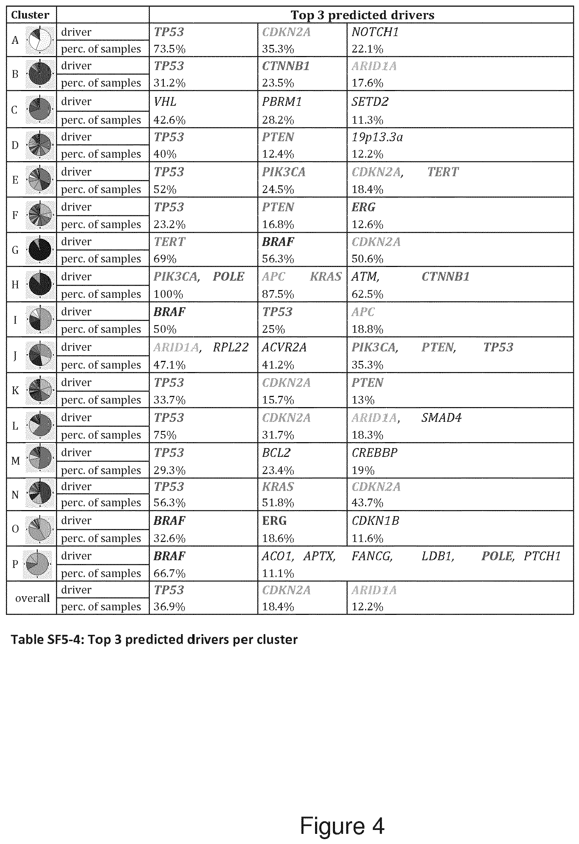

Table SF5-1 for the six SSM subtypes and in Figure 2: Table SF5-2 for the 1 bp SIM subtypes. Furthermore, there were several types of annotation available, provided either on sample or mutation level, which we overlay onto each cluster. An overview of the median values of these statistics is provided in Figure 3: Table SF5-3 as a summary. A more detailed view of the distribution of the mutations in terms of replication time on a sample level in each cluster was also calculated for SSMs and SIMs. Figure 4: Table SF5-4 shows the top three predicted drivers for each cluster. Finally, there are three types of mutational signatures based on different types of mutations: single base substitutions (SBS), doublet base substitutions (DBS), and insertions/deletions (ID). Note that doublet base substitutions were considered as single events in the PCAWG Mutational Signatures Working Group. We will use the aforementioned abbreviations when referring to the different types of signatures. For each cluster, the top three for each type of signatures are provided in Table SF5-5, including a short description of the proposed aetiology that has been linked to the signatures (1).

Cluster A - high percentage of C>A SSMs and 1 bp C/G deletions

Cluster A is dominated by samples from the two subtypes of lung cancer, Lung-AdenoCA and Lung-SCC. Together they make up nearly 84% of the 68 samples in this cluster. The two key features that are positively associated are the percentage of C>A SSMs and 1 bp C/G deletions. For close to 80% of the 58 samples in this cluster with collected smoking history, the donor was either a current smoker at diagnosis or had quit less than 15 years before. C>A transversions have been linked to the effects of tobacco smoke before, suggesting a preferential incorporation of dA opposite some of the tobacco-related guanine adducts (10). The 1 bp C/G deletions have not yet been associated with smoking related DNA damage. Interestingly, we do not observe the same association between smoking and the high percentage of C>A SSMs and 1 bp C/G deletions as in the two lung cancer subtypes for ten other tumour types for which information on smoking history of a sufficient number of the respective donors is available. Only three samples of Head-SCC reach similar percentages for both mutation types as the lung cancer samples. These donors also smoked at the time of diagnosis or had quit less than 15 years before. This supports the idea that direct tobacco-smoke exposure is essential for the 1 bp C/G deletions as well as the C>A SSMs. Several mechanisms for the deletions are conceivable including that the incoming dNTP pairs to the next template base and is stabilized by stacking interactions with the guanine adduct (10). Alternatively, if base excision repair removes the modified guanine, the abasic site in the template strand could loop out in a way that a 1 bp deletion manifests in the newly synthesized strand (11). Another interesting observation for this cluster is that it has the highest median number of C>G SSMs of all clusters (Figure 1: Table SF5-1), which is the least common SSM subtype overall. Cytosine deamination followed by base excision repair results in abasic sites, which combined with the activity of the REV1 polymerase has been suggested as one possible explanation for this SSM subtype (12). Finally, there is a significant negative association with all features reflecting recurrence. The cluster ranks in the bottom two in terms of relative recurrence (across the cohort) for four SSM subtypes and two SIM subtypes (Figure 1: Table SF5-1 and Figure 2: SF5-2) despite having the second highest median number for two of these four SSM subtypes (C>A and T>A, Table SF5-1) and one of the two SIM subtypes (1 bp C/G deletions, Table SF5-2).

**Annotation**

There are 2.1 times more SSMs (median across the cluster) in late-replicating regions compared to early, which is the second highest ratio of all clusters. The most frequently predicted driver in cluster A is the tumour suppressor gene *TP53* (73.5% of samples). This gene also ranks first in eight other clusters, but is only in one other cluster as often predicted a driver as in cluster A. *CDKN2A,* encoding an essential tumour suppressor, ranks second and is predicted to be a driver in 35.3% of the samples. Third is *NOTCH1,* a gene encoding a transmembrane protein relevant for interactions among adjacent cells (22.1% of samples). Another interesting observation is that the *RYR2,* although not considered a driver in any sample, has non-synonymous mutations in as many as 39.7% of the samples. This gene is involved in calcium signalling and has been found with elevated mutation frequency in air pollution-related lung cancer (13).

In line with the clinical data on smoking history, the 'tobacco smoking' signature based on single base substitutions (SBS4) is present in 89.7% of the samples and explains nearly half of the SBSs in these samples. For ~84% of the samples signature DBS2 explains all doublet base substitutions. This signature is potentially linked to exposure to tobacco smoking as well, but also to other exogenous and endogenous mutagens (1). Finally, tobacco-smoke exposure was also proposed to underlie the main ID signature in this cluster (ID3, median contribution: 71.9%).

Cluster B - high percentage of T>C SSMs

This cluster contains 324 samples of which just over 85% are Liver-HCC, which account for ~88% of the total number of samples of this tumour type. The strongest positively associated feature is the percentage of T>C transitions. A recent study of liver cancer with a high number of samples in common with the PCAWG cohort, uncovered two mutational signatures with high levels of T>C SSMs (14). While both signatures were linked to mutations in the *TERT* promoter, one was associated with alcohol intake and the other with age at cancer diagnosis. Based on whole exome sequencing data of liver cancer, Totoki et *al.* earlier suggested that the high percentage of T>C SSMs is more prevalent in Japanese males than in donors from the USA and that transcription-coupled repair pathways are crucial for the formation of this mutation type (15). In line with this, ~93% of Liver-HCC samples of male donors originating from the two Japanese studies are in cluster B versus ~81% of female Japanese donors and donors from studies not originating from Japan combined (Fisher Exact Test, p=0.0022). Within cluster B the male Japanese donors have a higher percentage of T>C SSMs than the other donors (34.3% vs. 28.3%, Wilcoxon rank-sum test, p=1.9e-10). With respect to a possible mechanism, in liver cells from individuals with diseases related to alcohol abuse increased levels of 1,N6-ethenodeoxyadenosine DNA adducts were detected (16). This type of adduct is likely a product of alcohol-induced oxidative stress and lipid peroxidation (16) and could lead to T>C transitions. Alcohol can also induce error-prone repair involving polymerase η, which leads to elevated levels of T>C transitions as well (17).

There is significant negative association with features capturing recurrence. Intuitively, one would expect the contrary given the large number of samples from the same tumour type and the fact that this cluster contains the highest total number of T>C SSMs (1,238,809). However, only 0.1% of the T>C SSMs are recurrent within the cluster.

**Annotation**

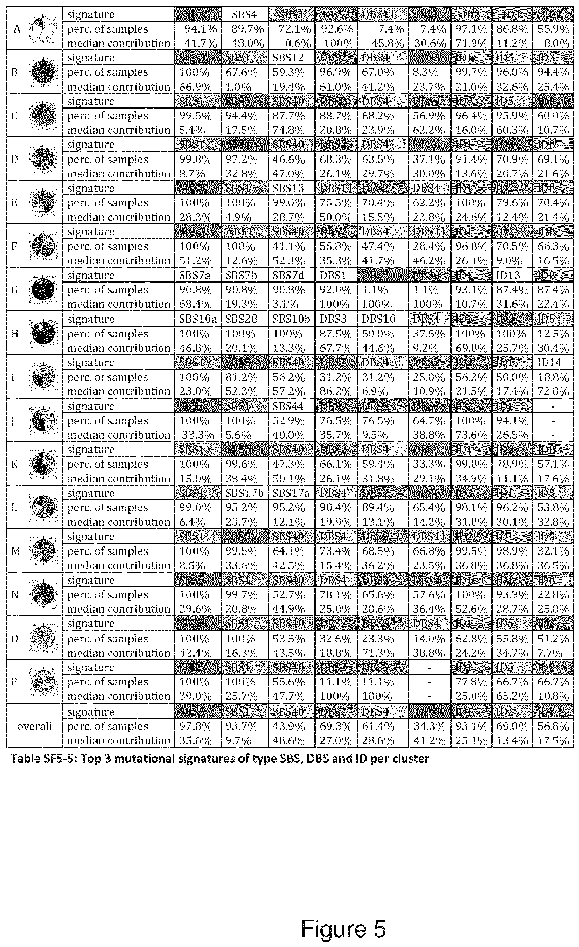

Around 1% of SIMs (median across samples) are in CDS, which is a higher proportion than in any other cluster with the exception of cluster C and D. *TP53* is the most frequently predicted driver (31.2% of samples), followed by the gene encoding the cell adhesion factor *CTNNB1* (23.5%), which has been related to alcohol-related liver cancer before (18). Ranking third is *ARID1A* (17.6%), a ubiquitously expressed tumour suppressor gene exerting its function by contributing to chromatin remodelling and transcriptional activation. In terms of mutational signatures, SBS5 stands out as it is present in all samples with a median contribution of 66.9% (Figure 5: Table SF5-5). No specific aetiology has been identified, but the number of mutations this signature explains, was correlated with age in normal as well as tumour cells (1). Another prominent signature is SBS12, as 192 of the 196 samples with a non-zero contribution of this signature belong to this cluster. The transcriptional strand bias observed for this signature (19) is consistent with transcription-coupled repair pathways acting upon dA, as described by Totoki *et al.* SBS12, however, is not an exact match to the signature that was linked to alcohol use by Fujimoto *et al.* and is of unknown aetiology (Figure 5: Table SF5-5). Finally, cluster B has in absolute numbers the most samples (77) with a non-zero contribution of the 'tobacco smoking signature' SBS4, which corresponds to ~24% of the samples in this cluster. However, the median contribution is much smaller than for cluster A (19% vs. 48%). In terms of SIMs the ID3 signature, which has been linked to tobacco-smoke exposure (and other mutagens), is omnipresent in this cluster (94.4% of all samples), but also with a much lower median contribution (25.4%) than for cluster A (71.9%).

Cluster C - high percentage of 1 bp A/T insertions in context of a short homopolymer

Cluster C contains ~96% of the Kidney-RCC and ~51% of the Kidney-ChRCC samples. Together they make up nearly 82% of the 195 samples in this cluster. The most striking feature is a strong positive association with the percentage of 1 bp A/T insertions in no and short homopolymer context. This is in contrast to what is observed overall, where 87.4% of the 2,546 samples with 1 bp A/T insertions have more than half of them in the context of a midsize-to-long homopolymer. Also for the three other SIM subtypes there is a positive association for this cluster with a short homopolymer context. There is a negative association with the percentage of recurrent SIMs, which is what we would expect given that recurrence is correlated with the midsize-to-long homopolymer context. The features related to recurrence of SSMs are also all negatively associated with this cluster. Other characteristics of this cluster include a high percentage of 1 bp A/T deletions and T>A SSMs. There are nine samples in this cluster that are outliers in terms of the percentage of T>A transversions, meaning a percentage that is larger than the third quartile by at least 1.5 times the interquartile range based on the entire cohort. The median percentage is 44.5% across these nine samples versus 11.4% in the rest of the dataset. This could point to an exposure to aristolochic acid, which is a highly mutagenic substance that forms purine adducts leading to misincorporation of dA opposite dA adducts (20,21). The error-prone polymerase ζ (REV3L) may be responsible for this mechanism resulting in T>A SSMs (22).

**Annotation**

In the whole dataset there are 1.6 and 1.4 times (median across all samples) more SSMs and SIMs, respectively, in late- versus early-replicating regions. In contrast, SSMs and SIMs in this cluster are relatively evenly distributed, as the median ratios are 1.2 and 0.9, respectively. The absence of enrichment of mutations in late-replicating regions matches also the relatively high percentage of the SSMs and SIMs that fall in CDS, regions that are typically replicated early (23), with a median across samples of 1.2% for both mutation types. The most frequently predicted driver in this cluster is the tumour-suppressor gene *VHL* (42.6% of the samples). Another tumour-suppressor gene, *PBRM1,* ranks second with 28.2% of the samples affected, and for 11.3% of the samples *SETD2,* a gene encoding a histone methyltransferase, is likely a driver. All three genes have been described before as playing a role in kidney cancer (24-26). Interestingly, for as many as ~24% of the samples no driver was discovered and the median number of suggested drivers for the remaining samples is only two. Finally, the three most prevalent SBS signatures (SBS1, SBS5, SBS40) coincide with the most common ones in the entire cohort. Seven of the nine samples with a particular high percentage of T>A SSMs have a contribution of between 16.3% and 82.6% by signature SBS22. This signature largely consists of T>A SSMs in various trinucleotide contexts and has been linked to aristolochic acid exposure (27). For ID signatures this is the only cluster with neither ID1 nor ID2 within the top three. Both of these signatures have been related to the slippage during DNA replication of the replicated DNA strand (1).

Cluster D - low percentage of 1 bp A/T insertions and high percentage of 1 bp C/G deletions

This clusters contains 502 samples from 31 of the 37 tumour types. The five tumour types that contribute the most samples and together constitute more than half of this cluster are Ovary-AdenoCA (17.7%), Breast-AdenoCA (14.5%), Lymph-CLL (8.8%), Panc-Endocrine (7.8%) and Prost-AdenoCA (6.4%). For Ovary-AdenoCA this corresponds to 80.9% of the samples, while for the other four this fraction is below 50%. Lymph-CLL is classically divided into two groups (28), those with somatic hypermutation in the variable segments of immunoglobulin genes and those without. We retrieved this information from the article in which the Lymph-CLL samples were described first (4). Based on this classification 42 of the 49 samples without hypermutation belong to this cluster and only two of the 40 samples with hypermutation. The two features that have the strongest association with this cluster are in positive direction the percentage of 1 bp C/G deletions and in negative direction the percentage of 1 bp A/T insertions. Interestingly, this cluster has a positive association with the percentages of the two symmetric substitutions, C>G and T>A, which are the least frequent SSM subtypes. The cluster has negative associations with the number of SIMs and SSMs as well as with any recurrence features. In line with this, there is a positive association with 1 bp SIMs in a non-homopolymer context.

**Annotation**

The median percentage of SIMs in CDS is 1.0%, which is the second highest value (together with cluster B) across all clusters. For 40% of the samples *TP53* is a predicted driver. The second most common driver candidate is *PTEN* (12.4%), encoding a protein relevant for the AKT/PKB signalling pathway. Closely behind follows the *19p13.3a* locus (12.2%). At the level of mutational signatures the top three SBS signatures are the most common ones in the entire cohort (SBS1, SBS5, SBS40). Also for the DBS signatures nothing stands out with respect to the other clusters. Finally, signature ID9, present in 70.9% of the samples, is more frequent than in any other cluster. The underlying process for this signature is unknown (1).

Cluster E - high percentage of C>G SSMs

There is not a single tumour type that dominates cluster E. The highest numbers of samples are from Breast-AdenoCA (31.6%), Head-SCC (14.3%) and Bladder-TCC (13.3%), which combined form more than half of this cluster. The majority of the Bladder-TCC samples belong to this cluster. The most striking characteristic of this cluster is a strong positive association with the percentage of C>G SSMs. The median percentage of C>G SSMs across the samples in this cluster is 26.5%, compared to only 7.7% overall. This also translates into a high absolute number, ranking this cluster second in this respect. There is a positive, but weaker association with the percentage of C>T SSMs and 1 bp C/G deletions. The elevated percentage of C>G combined with C>T SSMs has been described before as being due to increased activity of cytidine deaminases (12) (see also cluster A). The enriched motif uncovered for (recurrent) C>G SSMs (Figure 9), suggests a link to deamination mediated by APOBEC3 (29).

**Annotation**

This cluster has the lowest median percentage of SSMs in late-replicating regions (52.8%), together with cluster J. Furthermore, 1.2% of the SSMs are in CDS, which is the second highest value across all clusters. The most frequently predicted driver in this cluster is *TP53* (52% of the samples), followed by *PIK3CA* (24.5%), a gene encoding a kinase implicated in many cancers including breast cancer (30). *CDKN2A* and the telomerase gene *TERT,* whose expression counteracts cellular senescence, are the third most frequently proposed drivers (both with 18.4%). A high median number of 12 mutational signatures are present per sample (Figure 3: Table SF5-3, second highest of all clusters). Signatures SBS13 and SBS2 are both present in 99.0% of the samples with a median contribution of 28.7% and 25.3%, respectively.

The first signature has a high proportion of C>G SSMs and the latter a high proportion of C>T SSMs. Both have been suggested to be linked to the activity of the AID/APOBEC family of cytidine deaminases in combination with the faulty repair by error-prone polymerases (31). Further evidence for APOBEC's role in these signatures and hence in the mutations of the samples of this cluster comes from the observation that germline variations in genes encoding APOBEC3 enzymes are linked to predisposition to bladder and breast cancer as well as to mutational loads of signatures SBS2 and SBS13 (32). Finally, in 75.5% of the samples DBS11 is present, which may have the same aetiology as the two SBS signatures (1).

Cluster F - high percentage of 1 bp C/G insertions in context of a long homopolymer

There are 95 samples from 26 different tumour types in this cluster. The five tumour types that contribute the most samples and constitute more than half of this cluster are Prostate-AdenoCA (21.1%), CNS-Medullo (9.5%), Breast-AdenoCA (8.4%), Panc-Endocrine (8.4%), and Thyroid-AdenoCA (6.3%). The two features that have the strongest association with this cluster are the percentage of 1 bp C/G insertions in context of a long homopolymer and the percentage of recurrent 1 bp C/G insertions. The absolute number of mutations is relatively low in this cluster. The total number of 1 bp C/G insertions per sample ranges from only 1 to 16. Still, of all the 1 bp C/G insertions of this cluster combined, 34.3% of the 405 are recurrent (Figure 2: Table SF5-2), when defining recurrence across the entire cohort. In contrast, there is no significant association with overall recurrence, neither for SSMs nor for SIMs.

**Annotation**

The median number of genes mutated by a SIM is only one per sample. With two, the median number of detected drivers is low as well. The top three predicted driver genes are *TP53* (23.2%), *PTEN* (16.8%) and *ERG* (12.6%). The latter encodes a transcription factor with oncogenic potential. In terms of signatures, the most notable are SBS1 and SBS5, which are present in all samples with a median contribution of 12.6% and 51.2%, respectively

Cluster G - high percentage of recurrent C>T SSMs

This cluster is dominated by Skin-Melanoma samples (79 out of 87) and contains ~74% of all the samples of this tumour type. The high percentage of C>T SSMs, with a median of 84.7% per sample, is the key characteristic of this cluster. The high number of C>T substitutions is likely due to UV-induced DNA damage (33-35). There is a positive association with recurrence of SSMs in general and specifically with recurrent C>T SSMs. The median percentage of recurrent C>T substitutions across samples is 11.1%. Of the 10,150,303 C>T SSMs the samples in this cluster have combined, 4.5% are recurrent within this cluster alone (Figure 1: Table SF5-1). This translates to 460,163 in absolute numbers, which corresponds to 60.7% of the total number of recurrent C>T SSMs in the entire cohort.

**Annotation**

The median ratio of synonymous versus non-synonymous SSMs within CDS is significantly higher (~⅓) than in the remaining clusters (~¼) (p<2.2e-16). Nevertheless, there are still many genes affected by non-synonymous mutations (median: 410, ranked second in this respect). More than two thirds of the samples have *TERT* as a possible driver. Furthermore, over 50% have *BRAF,* a proto-oncogene that plays a crucial role in cell division, and/or *CDKN2A* as a driver. *TP53* is somewhat less frequently altered in this cluster (21.8% of the samples). In terms of the top three of mutational signatures, several signatures are linked to UV-light exposure (SBS7a/b/d, DBS1, ID13) (1) and contribute, as expected, strongly to the observed mutations (between 87.4% and 92.0%, Figure 5: Table SF5-5).

Cluster H - high mutational burden of SSMs

There are seven samples from ColoRect-AdenoCA in this cluster and one from Uterus-AdenoCA. Cluster H has the highest median number of SSMs (822,314) across its samples and the second highest for SIMs (9,168), behind cluster J. Cancer genomes with very high number of SSMs are often referred to as ultra-hypermutators (36). The cluster ranks first for the absolute number of all SSM subtypes with the exception of C>G of which cluster B and F exhibit even more (Figure 1: Table SF5-1). In agreement, the median percentage of C>G SSMs per sample is much lower than for the other clusters combined (0.3% vs. 7.8%, p≈1e-06). The main cause for the ultra-hypermutation is a mutated *POLE* gene, which is considered a driver in all eight samples. It may also explain the low percentage of C>G transversions. Mertz *et al.* showed for yeast that mutations in polymerase $\delta$, the other high-confidence polymerase of the B-family that complements polymerase $\epsilon$'s role in nuclear DNA replication, led to intracellular changes in the dNTP concentrations (37). This in turn further increased the nucleotide misincorporation rate. They showed that the proportionally lower availability of dG coincided with a reduced percentage of C>G transversions, the observation we also made for these ultra-hypermutated samples. In terms of recurrence there is positive association for C>A and C>T SSMs. There is a high level of recurrence for these two subtypes within the cluster, 3% and 2.2%, respectively (Figure 1: Table SF5-1). This translates in absolute numbers into 94,522 C>A SSMs, which corresponds to two thirds of all recurrent C>A SSMs in the entire cohort. For C>T SSMs this corresponds to only 4.5% of all recurrent ones, which is clearly less than in cluster G, despite the higher median number of this SSM subtype in cluster H. This is probably explainable by the low number of samples in this cluster. There is no significant association with recurrence of the other four SSM subtypes despite the high median number across samples. There is also no significant association with recurrence of SIMs, even though the cluster ranks second in terms of median number across samples for all 1 bp SIM subtypes except for C/G deletions (Figure 2: Table SF5-2).

**Annotation**

About two third of the samples in the entire cohort have a higher percentage of SSMs than SIMs in late-replicating regions. However, this cluster has the largest median difference in the opposite direction, 66.7% of SIMs are in late-replicating regions and only 60.2% of SSMs. This cluster has the lowest percentage of SSMs in CDS (0.6%) and the second lowest for SIMs (0.2%). Nevertheless, the high absolute number of mutations results in the top-ranking position in terms of median number of genes affected by non-synonymous SSMs (3,223) and the second position for the median number of genes altered by SIMs (13.5). In line with this, the cluster has the lowest median percentage of synonymous SSMs (18.7% of all SSMs in CDS). Furthermore, samples of no other cluster have such a high number of suggested drivers (median: 15). Beside *POLE,* all samples have *PIK3CA* as predicted driver. In seven out of the eight samples *APC,* a gene involved in apoptosis and cell migrations, which is often found mutated in malignancies, is considered a driver. In the same number of samples *KRAS,* a well-described proto-oncogene and a major regulator of cell proliferation, is a suggested driver. The only gene mutated by a SIM in more than one sample is *TTN,* which does not point to oncogenic potential, but is likely due to the huge size of this gene encoding the largest human protein with over 30k amino acids and relevant for muscle contraction. In contrast to many other clusters, *TP53* is considered a driver in only three of the eight samples. Finally, in all samples between 72.4% and 92.7% of the single base substitutions are explained by a combination of the mutational signatures SBS10a, SBS10b and SBS28. The former two are both linked to mutations in *POLE.* Signature SBS28 is of unknown aetiology although usually found in combination with the two SBS10 signatures (1). Signature DBS3 is also linked to a mutated *POLE* and is found in seven of the eight samples with a contribution of at least 38.9%. For six samples all SIMs are explained by ID1 and ID2 combined. For the other two samples signature ID5 or ID14, both of unknown aetiology, explain the remaining 30.4% and 22.3% of the insertions/deletions, respectively.

Cluster I - high percentage of C/G insertions

This cluster contains 16 samples of which eight are from CNS-PiloAstro. There is a strong positive association with the percentage of 1 bp C/G insertions. Although the median of the total number of 1 bp SIMs for samples in this cluster is only four, there are five outliers (three from ColoRect-AdenoCA and two from Panc-AdenoCA) that have relatively high absolute numbers of 1 bp SIMs ranging from 379 to 5,392. With a median number of 125 SSMs this cluster has the second lowest number of substitutions of all clusters and only the five aforementioned samples have substantially higher numbers of SSMs (5,530 - 21,116).

**Annotation**

The median number of genes mutated by SSMs and SIMs is two and zero, respectively. In terms of suggested drivers, the median number across samples is only one. A driver affecting all the CNS-PiloAstro samples is *BRAF.* Despite the low number of mutations in this cluster, the median number of signatures per sample is still six. The top three SBS signatures are the most common ones in the entire cohort (SBS1, SBS5, SBS40). For the DBS and ID signatures, only ID1 and ID2 are found in at least half of the samples.

Cluster J - high mutational burden of SIMs

This cluster consists of 17 samples from eight different tumour types, including Uterus-, Stomach- and ColoRect-AdenoCA. It is characterized by the highest median number of SIMs of all clusters (30,228). The high number of SIMs also translates into a high proportion of SIMs per sample. The median percentage is 35.1%, which is almost six times more than the percentage for the entire dataset. The SIM-related feature that particularly stands out is the high percentage of 1 bp C/G deletions in the context of a midsize homopolymer. Moreover, there is a high level of recurrence of SIMs. Of the 179,691 recurrent 1 bp SIMs in the entire cohort, 47.5% are also recurrent when only considering the samples in this cluster. The highest level of recurrence is observed for 1 bp A/T deletions. The samples in this cluster combined have 431,323 different 1 bp A/T deletions of which 17.2% are recurrent within this cluster (Figure 2: Table SF5-2). For the 1 bp C/G deletions 6.0% are recurrent within this cluster while for the other clusters this value ranges from 0% to 0.7% (Figure 2: Table SF5-2). For SSMs there is no significant association with recurrence and even though this cluster has the second highest median number of T>C SSMs across samples, only 0.2% are recurrent within this cluster and 1%, when referring to the entire cohort.

**MSI-Method 1** classified 16 samples in the entire dataset as MSI of which 15 belong to this cluster. The MSI-marked sample not assigned to this cluster is a Skin-Melanoma sample, which is assigned to cluster N instead. It has a clearly lower proportion of total (11.0%) and recurrent (33.0%) SIMs compared to samples that are assigned to cluster J (median of 35.1% and 87.5%, respectively), but higher proportions than other Skin-Melanoma samples (median of 1% and 0.4%). The sample does have the characteristic peak of 1 bp C/G deletions in a midsize homopolymer context. The two samples in this cluster **MSI-Method 1** did not identify as MSI, one from ColoRect-AdenoCA and one from Uterus-AdenoCA, have a high degree of correlation with the MSI-annotated samples for all 42 features. This suggests that they could have been misclassified and actually be MSI. In fact, MSI assignment is not always straightforward. **MSI-Method 2** labelled 14 of the 17 samples in this cluster as MSI and an additional five samples from other clusters. Aside from the aforementioned Skin-Melanoma sample, the other four neither have a high percentage of (recurrent) mutations that are SIMs, nor do they show a high percentage of 1 bp C/G deletions in the context of a midsize homopolymer.

## Annotation

The median percentage of SSMs and SIMs in late-replicating regions is only 1.1 times higher than in early-replicating regions. This is in line with what has previously been described, i.e. that mutations arising after the MMR pathway has been rendered dysfunctional, as is the case in MSI samples, are no longer enriched in late-replicating regions (38). In agreement with the very high burden of SIMs, this cluster has the highest median number of genes mutated by SIMs (104), which is far more than the median value for the entire dataset (3). In terms of SSMs, this cluster has the highest median percentage of SSMs in CDS (1.3%), but a relatively large percentage (median of 28.3%) of those SSMs are synonymous. The median number of detected drivers is 8, which is higher than for most clusters, but none of them affect more than half of the samples. The two most common drivers in this cluster (47.1% of the samples) are *RPL22,* which encodes a ribosomal protein often altered in MSI-related tumours (39) and *ARID1A*. Ranking second is *ACVR2A* (41.2%), an ubiquitously expressed transmembrane kinase, which has previously been predicted, based on a dataset of 6,747 cancer exomes from TCGA, to contain recurrently mutated, cancer-driving microsatellites (40). Interestingly, *TP53* is only a driver in roughly one third of the samples. Finally, there are seven SBS signatures (SBS6, SBS14, SBS15, SBS20, SBS21, SBS26 and SBS44) that have been linked to defective DNA MMR, two DBS signatures (DBS7, DBS10), and two ID signatures (ID1 and ID2). The latter two are found in most cancer genomes, but it has been noted that they tend to have a high absolute contribution to MSI samples (1). This is indeed the case in this cluster and combined these two ID signatures fully explain all SIMs of the 17 samples. One MSI-annotated sample in this cluster has zero contribution of all seven SBS signatures combined. The other 16 samples of the cluster, including the two not marked as MSI by the PCAWG consortium, have a combined contribution of these seven signatures of at least 39%. With respect to the two DBS signatures, 11 samples have a combined contribution of at least ~26%, whereas the others have none.

Cluster K - high percentage of 1 bp A/T insertions

This is the largest of all 16 clusters with 522 samples from 33 different tumour types. The four tumour types that contribute the most samples and constitute more than half of this cluster are Prost-AdenoCA (20.1%), CNS-Medullo (14.4%), Breast-AdenoCA (13.2%) and Panc-AdenoCA (11.7%). The strongest positive association in this cluster is with the percentage of 1 bp A/T insertions. For this SIM subtype there is also a positive association in terms of recurrence. There is no significant association with recurrence of SSMs in general, but there are positive associations with three specific subtypes (C>A, C>T and T>A).

## Annotation

This cluster does not show any particular characteristics as far as the annotation layers are concerned. While the median percentages of SSMs and SIMs in CDS for this cluster are around the overall median, corresponding numbers for late-replicating regions are below the overall value. In about one third of the samples *TP53* is likely to be driver, followed by *CDKN2A* (15.7%) and *PTEN* (13%). The top three SBS signatures are the most common ones in the entire cohort (SBS1, SBS5, SBS40). Also for the DBS and ID signatures, this cluster does not stand out from the others.

Cluster L - high percentage of recurrent T>G SSMs

Eso-AdenoCA (62.5%) and Stomach-AdenoCA (20.2%) samples combined constitute the vast majority of the samples in cluster L. The strongest positive association in this cluster is with the percentage of T>G SSMs. There is also a positive association with the percentage of T>C SSMs. As for absolute numbers of T>G and T>C SSMs, the median across samples is 6,769 and 4,956, respectively (Figure 1: Table SF5-1). This ranks the cluster second for T>G SSMs and fourth for T>C SSMs. Accordingly, two mutational signatures have been described to be unique for Eso-AdenoCA, one with a high percentage of T>G SSMs and the other with additionally a relatively high percentage of T>C SSMs. These signatures have been suggested to be linked to gastric reflux (41,42), whereby the low pH as well as bile acids could result in oxidative DNA damage particularly affecting purines (43). Tomkova et al. recently proposed that, in the case of gastric cancers and oesophagus adenocarcinoma, the T>G SSMs may be explained by oxidative damage to dG in the dNTP pool (44). In fact, error-prone DNA polymerases have been shown to incorporate the oxidized dG opposite dA during replication, which would then result in a A>C manifestation after the next replication round (45).

With respect to recurrence of SSMs, the cluster again shows a particularly strong association with T>G SSMs, followed by T>C SSMs. When only considering the samples within this cluster 3.6% of the T>G and 1.1% of the T>C SSMs are recurrent. These numbers correspond to the highest proportions of all clusters with respect to the total number of recurrent T>G and T>C SSMs of the entire cohort (45.0% and 19.7%, respectively). This suggests that the mutational processes behind both SSM subtypes are not random and more complex than only via the production of high levels of oxidative stress. In terms of SIMs, there is a positive association with recurrence of all subtypes except for the 1 bp C/G insertions. The strongest association is with the recurrence of 1 bp A/T deletions, for which there is also a positive association with the long homopolymer context feature.

**Annotation**

There is a strong enrichment of SSMs in late-replicating regions with a median percentage of 75.2%, which is three times higher than in early-replicating regions and the highest percentage of all clusters. Also for SIMs a high median percentage is in late-replicating region (67.2%), only marginally exceeded by cluster M. In line with these observations is that only 0.7% and 0.5% of SSMs and SIMs, respectively, fall in CDS, which are, as mentioned, typically in early-replicating regions. We speculate that mutagenic influences typical for this cluster (e.g. gastric or bile acid) could more severely affect late-replicating regions due to the longer transient exposure of single-stranded DNA (46). Another explanation would be that if oxidative damage to the dNTP pool underlies the T>G SSMs (44), the faulty incorporation of oxidized dG into DNA depends on error-prone polymerases (45) active in late stages of replication (47). One caveat to these interpretations is the approximation of replication times by calculating the median of values from five cancer cell lines, of which none represents the cell types of the main tumour types in this cluster.

In terms of drivers, TP53 stands out as it is considered a driver in 75% of the samples, which is the highest percentage in all clusters. At second place is CDKN2A (31.7%), followed by ARID1A and SMAD4 (18.3%), which is part of the TGFβ signalling pathway. Although not considered a driver, PCLO was altered by non-synonymous mutations in 33.8% of the Eso-AdenoCA samples in this cluster and 29.8% of all samples in this cluster. Mutations in this gene encoding a presynaptic cytoskeletal protein have recently been suggested as a prognostic marker in oesophageal squamous-cell carcinoma (Eso-SCC) (48). Further genes which are affected by non-synonymous mutations in a material number of samples include HMCN1 (28.9%), a cell polarity-related gene previously found mutated in cancers of the gastro-intestinal tract (49), SYNE1 (26%), encoding a nuclear membrane protein and candidate gene for oesophagogastric junctional adenocarcinoma (50), as well as LRP1B (25%), a tumour suppressor gene previously related to Eso-SCC (51). Finally, the median number of mutational signatures present across samples is 14, the highest of all clusters. Signatures SBS17a and SBS17b are present in 95.2% of the samples with a median contribution of 23.7% and 12.1%, respectively. Despite the resemblance to the aforementioned signatures found by Secrier et al., the PCAWG working group did not link the signatures to any aetiology.

Cluster M - high percentage of recurrent 1 bp A/T deletions and C/G SSMs

Two lymphoid cancers, Lymph-BNHL (51.6%) and Lymph-CLL (20.1%), together make up more than 70% of cluster M. Nearly all Lymph-BNHL samples are in this cluster (88.8%). While each of the 13 features capturing recurrence is positively associated with this cluster, the strongest association is observed with recurrent 1 bp A/T deletions. Related to that is the strong association with the general feature of the percentage of 1 bp A/T deletions in a long homopolymer context. With respect to recurrent SSMs the strongest positive association is observed for C>G transversions, for which the median percentage of recurrence per sample is 3.8% (versus 0.6% across the entire dataset). Despite eight clusters having a higher median total number of C>G SSMs across samples, this cluster has the highest absolute number of C>G SSMs that are recurrent within the cluster, which represent 10.7% of the total amount in the cohort. Interestingly, for the general features the cluster shows elevated percentages of the three possible thymidine substitutions (T>A/C/G), while the three possible cytosine substitutions (C>A/G/T) are reduced.

For the samples of Lymph-BNHL and Lymph-CLL in this cluster combined we observe peaks of recurrent mutations at immunoglobulin genes. This points to a large proportion of samples from non-naive B-cells, in which somatic hypermutation has already taken place. This process involves the error-prone polymerase η and results in mutations preferably at A and T bases (52). Polymerase η is further known to efficiently bypass various dT lesions, but with a higher chance of nucleotide misincorporation (53). For the Lymph-CLL samples in PCAWG, annotation according to hypermutation status is available (4). This earlier study classified 40 samples as hypermutated of which 36 samples are in cluster M. Of the 49 Lymph-CLL samples classified as non-hypermutated, only one is assigned to this cluster. The hypermutation of the immunoglobulin genes is likely one explanation of the observed high level of recurrence, as these regions represent only a small part of the genome. In contrast, Lymph-CLL samples without the hypermutation phenotype show negative association with recurrence (cluster D).

**Annotation**

There are twice as many SSMs in late-replicating regions as in early-replicating regions (median across the samples). For SIMs, the imbalance is even more pronounced as the median percentage in late-replicating regions is 67.5%, which is the highest value of all clusters. In terms of drivers, *TP53* (29.3%) is the top-ranking gene, followed by *BCL2* (23.4%), playing a role in apoptosis of lymphocytes (54) and *CREBBP* (19%), encoding a nuclear protein previously associated with leukemia (55). The *IGLL5* gene, located in the immunoglobulin lambda locus, is altered by non-synonymous mutations in ⅓ of the lymphoid cancer samples in this cluster and represents an effect of the somatic hypermutation. In 39.1% of the samples signature SBS9 is present, which has been linked to polymerase η activity (1). With respect to the 36 Lymph-CLL samples that were indicated to be hypermutated in this cluster, 33 of them have a non-zero contribution of this signature. Of the 86 samples in the entire cohort in which this signature is present, ~84% are in this cluster.

Cluster N - high proportion of recurrent SIMs

The majority of the samples in cluster N are from Panc-AdenoCA (46.6%) or CNS-Medullo (16.4%). Also ~56% of all ColoRect-AdenoCA samples are in this cluster. Except for C>G and T>C SSMs, all other features capturing recurrence are positively associated with this cluster. The level of recurrence is one aspect that differentiates the Panc-AdenoCA from tumours belonging to Panc-Endocrine (cluster D). The high level of recurrence is particularly notable for CNS-Medullo where the median total number of mutations (1,359) is much lower than, for example, in Panc-AdenoCA samples of this cluster (5,626). The strongest association is observed with recurrent 1 bp A/T deletions along with, as expected, the same SIM subtype in the context of a long homopolymer. Another feature that particularly stands out in terms of recurrence is the high proportion of SIMs. The median percentage of recurrent mutations that are SIMs is 34.1%, which is twice as high as in the entire dataset. Finally, this cluster is ranked second in terms of the percentage of T>A SSMs that are recurrent within the cluster (Figure 1: Table SF5-1). The same holds for recurrence in the entire cohort for this SSM subtype and in absolute numbers the cluster even ranks first.

**Annotation**

A median of 1.2% of the SSMs falls in CDS, which is, together with clusters A, F and H, the second highest percentage of all clusters. More than half of the samples have *TP53* (56.3%) and/or *KRAS* (51.8%) as predicted drivers. This percentage increases to ~81% and ~95%, respectively, when only considering the Panc-AdenoCA samples in this cluster. *CDKN2A* ranks third with 43.7%, which also increases substantially to ~78% in Panc-AdenCA samples. All three genes are considered relevant for the development of this tumour type (56). In terms of signatures, SBS1, SBS5 and SBS40 show large contributions to the samples of this cluster, which is, however, in general the case across the samples of the entire cohort. A signature more specific to this cluster is SBS18, which is present in ~48% of the samples, including 28 of the 29 ColoRect-AdenoCA samples and 11 of the 12 Stomach-AdenoCA samples. A suggested aetiology for this signature is damage caused by reactive oxygen species (1). For insertions/deletions, the main signatures are ID1 and ID2, which are present in most samples of the cohort.

Cluster O - high percentage of recurrent SSMs for each subtype

Samples from Prost-AdenoCA and CNS-PiloAstro together make up nearly 80% of this cluster, which consists of 43 samples in total. There are positive associations with 9 of the 13 recurrence features, particularly with recurrent SSMs of type T>C, C>G and T>A. However, the absolute number of SSMs and SIMs is very low in this cluster with a median number of 182 and 11, respectively.

**Annotation**

In 12 of the 43 samples no drivers were detected. The remaining samples have a median number of just one driver. Most frequently predicted driver genes are *BRAF* (14 samples), *ERG* (8 samples) and *CDKN1B* (5 samples). The latter plays a role in the cell cycle progression at the G1 phase. The median number of signatures linked to each sample is seven, which is surprisingly high given the low median number of mutations. The top three signatures for SBS, DBS and ID, are all among the most common ones in the entire cohort (SBS1, SBS5, SBS40; DBS2, DBS4, DBS9; ID1, ID2, ID5).

Cluster P - percentage of 1 bp C/G deletions in context of a long homopolymer

This cluster captures only nine samples from four different tumour types (CNS-Medullo, CNS-PiloAstro, Prost-AdenoCA and Thy-AdenoCA). A positive association with the percentage of 1 bp C/G deletions in context of a long homopolymer as well as with the percentage of recurrent 1 bp C/G deletions are the most notable features. The absolute numbers of SSMs and SIMs are quite low per sample and range from 42 to 560 and from 7 to 28, respectively.

**Annotation**

For six out of the nine samples *BRAF* is suggested as a driver. The median number of signatures active in a sample is five, the lowest number of all clusters. The most frequently contributing signatures for SBS, DBS and ID, are all the most common ones in the entire cohort (SBS1, SBS5, SBS40; DBS2, DBS9; ID1, ID2, ID5).

**References for detailed cluster-specific descriptions**

1. Alexandrov L, Kim J, Haradhvala NJ, Huang MN, Ng AWT, Boot A, et al. The Repertoire of Mutational Signatures in Human Cancer. bioRxiv 2018:322859 doi 10.1101/322859.

2. Waszak SM, Tiao G, Zhu B, Rausch T, Muyas F, Rodriguez-Martin B, et al. Germline determinants of the somatic mutation landscape in 2,642 cancer genomes. bioRxiv 2017 doi 10.1101/208330.

3. Niu B, Ye K, Zhang Q, Lu C, Xie M, McLellan MD, et al. MSIsensor: microsatellite instability detection using paired tumor-normal sequence data. Bioinformatics 2014;30(7):1015-6 doi 10.1093/bioinformatics/btt755.

4. Puente XS, Bea S, Valdes-Mas R, Villamor N, Gutierrez-Abril J, Martin-Subero JI, et al. Non-coding recurrent mutations in chronic lymphocytic leukaemia. Nature 2015;526(7574):519-24 doi 10.1038/nature14666.

5. Ramos AH, Lichtenstein L, Gupta M, Lawrence MS, Pugh TJ, Saksena G, et al. Oncotator: Cancer Variant Annotation Tool. Hum Mutat 2015;36(4):E2423-E9 doi 10.1002/humu.22771.

6. Harrow J, Frankish A, Gonzalez JM, Tapanari E, Diekhans M, Kokocinski F, et al. GENCODE: the reference human genome annotation for The ENCODE Project. Genome research 2012;22(9):1760-74 doi 10.1101/gr.135350.111.

7. Rheinbay E, Nielsen MM, Abascal F, Tiao G, Hornshøj H, Hess JM, et al. Discovery and characterization of coding and non-coding driver mutations in more than 2,500 whole cancer genomes. bioRxiv 2017 doi 10.1101/237313.

8. Sabarinathan R, Pich O, Martincorena I, Rubio-Perez C, Juul M, Wala J, et al. The whole-genome panorama of cancer drivers. bioRxiv 2017 doi 10.1101/190330.

9. Hansen RS, Thomas S, Sandstrom R, Canfield TK, Thurman RE, Weaver M, et al. Sequencing newly replicated DNA reveals widespread plasticity in human replication timing. Proc Natl Acad Sci USA 2010;107(1):139-44 doi 10.1073/pnas.0912402107.

10. Avkin S, Livneh Z. Efficiency, specificity and DNA polymerase-dependence of translesion replication across the oxidative DNA lesion 8-oxoguanine in human cells. Mutation Research/Fundamental and Molecular Mechanisms of Mutagenesis 2002;510(1-2):81-90 doi 10.1016/S0027-5107(02)00254-3.

11. Liu B, Xue Q, Tang Y, Cao J, Guengerich FP, Zhang H. Mechanisms of mutagenesis: DNA replication in the presence of DNA damage. Mutation Research/Reviews in Mutation Research 2016;768:53-67 doi 10.1016/j.mrrev.2016.03.006.

12. Sale JE, Lehmann AR, Woodgate R. Y-family DNA polymerases and their role in tolerance of cellular DNA damage. Nature reviews Molecular cell biology 2012;13(3):141-52 doi 10.1038/nrm3289.

13. Yu X-J, Yang M-J, Zhou B, Wang G-Z, Huang Y-C, Wu L-C, et al. Characterization of Somatic Mutations in Air Pollution-Related Lung Cancer. EBioMedicine 2015;2(6):583-90 doi 10.1016/j.ebiom.2015.04.003.

14. Fujimoto A, Furuta M, Totoki Y, Tsunoda T, Kato M, Shiraishi Y, et al. Whole-genome mutational landscape and characterization of noncoding and structural mutations in liver cancer. Nature Genetics 2016;48:500 doi 10.1038/ng.3547.

15. Totoki Y, Tatsuno K, Covington KR, Ueda H, Creighton CJ, Kato M, et al. Trans-ancestry mutational landscape of hepatocellular carcinoma genomes. Nature Genetics 2014;46:1267 doi 10.1038/ng.3126.

16. Frank A, Seitz HK, Bartsch H, Frank N, Nair J. Immunohistochemical detection of 1,N6-ethenodeoxyadenosine in nuclei of human liver affected by diseases predisposing to hepato-carcinogenesis. Carcinogenesis 2004;25(6):1027-31 doi 10.1093/carcin/bgh089.

17. Supek F, Lehner B. Clustered Mutation Signatures Reveal that Error-Prone DNA Repair Targets Mutations to Active Genes. Cell 2017;170(3):534-47.e23 doi 10.1016/j.cell.2017.07.003.

18. Schulze K, Imbeaud S, Letouzé E, Alexandrov LB, Calderaro J, Rebouissou S, et al. Exome sequencing of hepatocellular carcinomas identifies new mutational signatures and potential therapeutic targets. Nature Genetics 2015;47:505 doi 10.1038/ng.3252.

19. Alexandrov LB, Nik-Zainal S, Wedge DC, Aparicio SAJR, Behjati S, Biankin AV, et al. Signatures of mutational processes in human cancer. Nature 2013;500(7463):415-21 doi 10.1038/nature12477.

20. Attaluri S, Bonala RR, Yang I-Y, Lukin MA, Wen Y, Grollman AP, et al. DNA adducts of aristolochic acid II: total synthesis and site-specific mutagenesis studies in mammalian cells. Nucleic Acids Research 2010;38(1):339-52 doi 10.1093/nar/gkp815.

21. Scelo G, Riazalhosseini Y, Greger L, Letourneau L, Gonzalez-Porta M, Wozniak MB, et al. Variation in genomic landscape of clear cell renal cell carcinoma across Europe. Nature Communications 2014;5:5135 doi 10.1038/ncomms6135.

22. Hashimoto K, Bonala R, Johnson F, Grollman AP, Moriya M. Y-family DNA polymerase-independent gap-filling translesion synthesis across aristolochic acid-derived adenine adducts in mouse cells. DNA Repair 2016;46:55-60 doi 10.1016/j.dnarep.2016.07.003.

23. Jeon Y, Bekiranov S, Karnani N, Kapranov P, Ghosh S, MacAlpine D, et al. Temporal profile of replication of human chromosomes. Proceedings of the National Academy of Sciences of the United States of America 2005;102(18):6419-24 doi 10.1073/pnas.0405088102.

24. Gnarra JR, Tory K, Weng Y, Schmidt L, Wei MH, Li H, et al. Mutations of the VHL tumour suppressor gene in renal carcinoma. Nature Genetics 1994;7:85 doi 10.1038/ng0594-85.

25. Varela I, Tarpey P, Raine K, Huang D, Ong CK, Stephens P, et al. Exome sequencing identifies frequent mutation of the SWI/SNF complex gene PBRM1 in renal carcinoma. Nature 2011;469(7331):539-42 doi 10.1038/nature09639.

26. Dalgliesh GL, Furge K, Greenman C, Chen L, Bignell G, Butler A, et al. Systematic sequencing of renal carcinoma reveals inactivation of histone modifying genes. Nature 2010;463(7279):360-3 doi 10.1038/nature08672.

27. Hoang ML, Chen CH, Sidorenko VS, He J, Dickman KG, Yun BH, et al. Mutational signature of aristolochic acid exposure as revealed by whole-exome sequencing. Science translational medicine 2013;5(197):197ra02 doi 10.1126/scitranslmed.3006200.

28. Hamblin TJ, Davis Z, Gardiner A, Oscier DG, Stevenson FK. Unmutated Ig VH Genes Are Associated With a More Aggressive Form of Chronic Lymphocytic Leukemia. Blood 1999;94(6):1848-54.

29. Chan K, Roberts SA, Klimczak LJ, Sterling JF, Saini N, Malc EP, et al. An APOBEC3A hypermutation signature is distinguishable from the signature of background mutagenesis by APOBEC3B in human cancers. Nature Genetics 2015;47:1067 doi 10.1038/ng.3378.

30. Dirican E, Akkiprik M, Özer A. Mutation distributions and clinical correlations of PIK3CA gene mutations in breast cancer. Tumor Biology 2016;37(6):7033-45 doi 10.1007/s13277-016-4924-2.

31. Nik-Zainal S, Alexandrov LB, Wedge DC, Van Loo P, Greenman CD, Raine K, et al. Mutational processes molding the genomes of 21 breast cancers. Cell 2012;149(5):979-93 doi 10.1016/j.cell.2012.04.024.

32. Nik-Zainal S, Wedge DC, Alexandrov LB, Petljak M, Butler AP, Bolli N, et al. Association of a germline copy number polymorphism of APOBEC3A and APOBEC3B with burden of putative APOBEC-dependent mutations in breast cancer. Nature Genetics 2014;46:487 doi 10.1038/ng.2955.

33. Howard BD, Tessman I. Identification of the altered bases in mutated single-stranded DNA: II. In vivo mutagenesis by 5-bromodeoxyuridine and 2-aminopurine. Journal of Molecular Biology 1964;9(2):364-71 doi 10.1016/S0022-2836(64)80213-8.

34. Rusch HP, Baumann CA. Tumor Production in Mice with Ultraviolet Irradiation. The American Journal of Cancer 1939;35(1):55-62 doi 10.1158/ajc.1939.55.

35. Brash DE. UV Signature Mutations. Photochemistry and Photobiology 2015;91(1):15-26 doi 10.1111/php.12377.

36. Shlien A, Campbell BB, de Borja R, Alexandrov LB, Merico D, Wedge D, et al. Combined hereditary and somatic mutations of replication error repair genes result in rapid onset of ultra-hypermutated cancers. Nature Genetics 2015;47:257 doi 10.1038/ng.3202.

37. Mertz TM, Sharma S, Chabes A, Shcherbakova PV. Colon cancer-associated mutator DNA polymerase δ variant causes expansion of dNTP pools increasing its own infidelity. Proceedings of the National Academy of Sciences 2015;112(19):E2467-E76 doi 10.1073/pnas.1422934112.

38. Supek F, Lehner B. Differential DNA mismatch repair underlies mutation rate variation across the human genome. Nature 2015;521(7550):81-4 doi 10.1038/nature14173.

39. Ferreira AM, Tuominen I, Dijk-Bos K, Sanjabi B, Sluis T, Zee AG, et al. High Frequency of RPL22 Mutations in Microsatellite-Unstable Colorectal and Endometrial Tumors. Human Mutation 2014;35(12):1442-5 doi doi:10.1002/humu.22686.

40. Maruvka YE, Mouw KW, Karlic R, Parasuraman P, Kamburov A, Polak P, et al. Analysis of somatic microsatellite indels identifies driver events in human tumors. Nature Biotechnology 2017;35:951 doi 10.1038/nbt.3966.

41. Dulak AM, Stojanov P, Peng S, Lawrence MS, Fox C, Stewart C, et al. Exome and whole genome sequencing of esophageal adenocarcinoma identifies recurrent driver events and mutational complexity. Nature genetics 2013;45(5):478-86 doi 10.1038/ng.2591.

42. Secrier M, Li X, de Silva N, Eldridge MD, Contino G, Bornschein J, et al. Mutational signatures in esophageal adenocarcinoma define etiologically distinct subgroups with therapeutic relevance. Nature Genetics 2016;48:1131 doi 10.1038/ng.3659.

43. Dvorak K, Payne CM, Chavarria M, Ramsey L, Dvorakova B, Bernstein H, et al. Bile acids in combination with low pH induce oxidative stress and oxidative DNA damage: relevance to the pathogenesis of Barrett's oesophagus. Gut 2007;56(6):763-71 doi 10.1136/gut.2006.103697.

44. Tomkova M, Tomek J, Kriaucionis S, Schuster-Böckler B. Mutational signature distribution varies with DNA replication timing and strand asymmetry. Genome Biology 2018;19(1):129 doi 10.1186/s13059-018-1509-y.

45. Kamiya H. Mutations Induced by Oxidized DNA Precursors and Their Prevention by Nucleotide Pool Sanitization Enzymes. Genes and Environment 2007;29(4):133-40 doi 10.3123/jemsge.29.133.

46. Stamatoyannopoulos JA, Adzhubei I, Thurman RE, Kryukov GV, Mirkin SM, Sunyaev SR. Human mutation rate associated with DNA replication timing. Nature Genetics 2009;41:393 doi 10.1038/ng.363.

47. Seplyarskiy VB, Bazykin GA, Soldatov RA. Polymerase $\zeta$ Activity Is Linked to Replication Timing in Humans: Evidence from Mutational Signatures. Molecular Biology and Evolution 2015;32(12):3158-72 doi 10.1093/molbev/msv184.

48. Zhang W, Hong R, Xue L, Ou Y, Liu X, Zhao Z, et al. Piccolo mediates EGFR signaling and acts as a prognostic biomarker in esophageal squamous cell carcinoma. Oncogene 2017;36(27):3890-902 doi 10.1038/onc.2017.15.

49. Lee SH, Je EM, Yoo NJ, Lee SH. HMCN1, a cell polarity-related gene, is somatically mutated in gastric and colorectal cancers. Pathology & Oncology Research 2015;21(3):847-8 doi 10.1007/s12253-014-9809-3.

50. Chong IY, Cunningham D, Barber LJ, Campbell J, Chen L, Kozarewa I, et al. The genomic landscape of oesophagogastric junctional adenocarcinoma. The Journal of Pathology 2013;231(3):301-10 doi 10.1002/path.4247.

51. Chang J, Tan W, Ling Z, Xi R, Shao M, Chen M, et al. Genomic analysis of oesophageal squamous-cell carcinoma identifies alcohol drinking-related mutation signature and genomic alterations. Nature Communications 2017;8:15290 doi 10.1038/ncomms15290

52. Zanotti KJ, Gearhart PJ. Antibody diversification caused by disrupted mismatch repair and promiscuous DNA polymerases. DNA Repair 2016;38:110-6 doi dx.doi.org/10.1016/j.dnarep.2015.11.011.

53. Williams NL, Wang P, Wu J, Wang Y. In Vitro Lesion Bypass Studies of O4-Alkylthymidines with Human DNA Polymerase η. Chemical Research in Toxicology 2016;29(4):669-75 doi 10.1021/acs.chemrestox.5b00509.

54. Tarte K, Jourdan M, Veyrune JL, Berberich I, Fiol G, Redal N, et al. The Bcl-2 family member Bfl-1/A1 is strongly repressed in normal and malignant plasma cells but is a potent anti-apoptotic factor for myeloma cells. British Journal of Haematology 2004;125(3):373-82 doi 10.1111/j.1365-2141.2004.04908.x.

55. Ding L-W, Sun Q-Y, Tan K-T, Chien W, Thippeswamy AM, Eng Juh Yeoh A, et al. Mutational Landscape of Pediatric Acute Lymphoblastic Leukemia. Cancer Research 2017;77(2):390-400 doi 10.1158/0008-5472.can-16-1303.

56. Notta F, Chan-Seng-Yue M, Lemire M, Li Y, Wilson GW, Connor AA, et al. A renewed model of pancreatic cancer evolution based on genomic rearrangement patterns. Nature 2016;538(7625):378-82 doi 10.1038/nature19823.

All references cited herein are incorporated herein by reference in their entirety and for all purposes to the same extent as if each individual publication or patent or patent application was specifically and individually indicated to be incorporated by reference in its entirety.

The specific embodiments described herein are offered by way of example, not by way of limitation. Any sub-titles herein are included for convenience only, and are not to be construed as limiting the disclosure in any way.

## Claims

1.  A computer-implemented method for classifying a test sample obtained from a tumour of a test subject, comprising:

(a) providing a whole genome sequence of the tumour and of a non-tumour sample from the test subject;
(b) analysing the genome sequence of the tumour to compute at least five general features and at least five recurrence features selected from the general and recurrence features shown in Table SF2-1;
(c) providing at least five reference clusters that have been obtained by:

(i) analysing a training data set comprising tumour genomes of at least 1000 subjects to compute said at least five general features and at least five recurrence features defined in step (b) for each of said at least 1000 subjects;
(ii) performing principal component analysis (PCA) using the features of step (i) as input, optionally after scaling and/or centring the features to account for their different scales, to obtain a plurality of principal components that together account for at least 50% of the variance of the training data set; and
(iii) performing hierarchical clustering using said plurality of principal components to divide the samples of the training data set into at least five reference clusters, each reference cluster having a centre that is defined by computing the mean of the plurality of principal components of all samples in that cluster;

(d) determining the Euclidean distance between said plurality of principal components of the test sample and the centre of each of said reference clusters; and
(e) classifying the tumour sample obtained from the test subject as belonging to the reference cluster to which it is found to be nearest in step (d).

2. The method of claim 1, wherein said at least five general features comprise all 29 general features shown in Table SF2-1 and wherein said at least five recurrence features comprise all 13 recurrence features shown in Table SF2-1.

3. The method of claim 1 or claim 2, wherein mutations are considered recurrent if they are recurrent in the Pan-Cancer Analysis of Whole Genomes (PCAWG) consortium cohort.

4. The method according to any one of the preceding claims, wherein said training data set comprises 2583 cancer genomes of the PCAWG consortium cohort.

5. The method of any one of the preceding claims, wherein said plurality of principal components comprises the first 18 principal components.

6. The method of any one of the preceding claims, wherein the distance from the test sample to the closest cluster centre in step (d) is found to be within 0.5 of the distance to the next closest cluster centre, and wherein step (e) further comprises a step of adding further statistics and/or metadata to confirm the classification.

7. The method of any one of the preceding claims, wherein said at least five reference clusters comprise 16 reference clusters.

8. The method of claim 7, wherein said 16 reference clusters are defined by the means of the first 18 principal components as set forth in Table 2 or Table 3.

9. The method of claim 8, wherein the test sample is classified as belonging to one of the following clusters:

Cluster A;
Cluster B;
Cluster C;
Cluster E;
Cluster G;
Cluster J;
Cluster F;
Cluster H;
Cluster I;
Cluster L; and
Cluster O.

10. A method for classifying a test sample obtained from a tumour of a test subject, comprising:

sequencing DNA obtained from the test sample to provide a whole genome sequence of the tumour; and performing the method of any one of claims 1 to 9 using the whole genome sequence thus obtained.

**11.** The method of claim 10, wherein said sequencing comprises extracting and/or amplifying DNA from the sample.

**12.** A system for classifying a test sample obtained from a tumour of a test subject, comprising:

at least one processor; and
at least one non-transitory computer readable medium containing instructions that, when executed by the at least one processor, cause the at least one processor to perform operations comprising:

(a) receiving sequence data representing the whole genome sequence of the tumour and of a non-tumour sample from the test subject;
(b) analysing the sequence data to compute the 29 general features shown in Table SF2-1 and the 13 recurrence features shown in Table SF2-1;
(c) receiving principal components data defining the 16 reference clusters as set forth in Table 2 or Table 3;
(d) determining the Euclidean distance between the first 18 principal components of the test sample and the centre of each of said reference clusters; and
(e) classifying the tumour sample obtained from the test subject as belonging to the reference cluster to which it is found to be nearest in step (d).

**13.** The system of claim 12, wherein the system is for use in a method according to any one of claims 1 to 9.

**14.** A non-transitory computer readable medium for classifying a test sample obtained from a tumour of a test subject, comprising instructions that, when executed by at least one processor, cause the at least one processor to perform operations comprising:

(a) receiving sequence data representing the whole genome sequence of the tumour and of a non-tumour sample from the test subject;
(b) analysing the sequence data to compute the 29 general features shown in Table SF2-1 and the 13 recurrence features shown in Table SF2-1;
(c) receiving principal components data defining the 16 reference clusters as set forth in Table 2 or Table 3;
(d) determining the Euclidean distance between the first 18 principal components of the test sample and the centre of each of said reference clusters; and
(e) classifying the tumour sample obtained from the test subject as belonging to the reference cluster to which it is found to be nearest in step (d).

**15.** The medium of claim 14, wherein the medium is for use in a method according to any one of claims 1 to 9.

**16.** A method for predicting that a tumour of a patient is microsatellite instable (MSI), comprising: performing the method of claim 8, wherein the test sample is a sample that has been obtained from the tumour of the patient, and wherein the sample is classified as belonging to cluster J.

**17.** A method for predicting whether a tumour-burdened patient will benefit from immunotherapy, comprising: performing the method of claim 8, wherein the test sample is a sample that has been obtained from the tumour of the patient, and wherein the sample is classified as belonging to cluster H or cluster L.

**18.** The method of claim 16 or claim 17, wherein the method further comprises:

selecting the patient for immunotherapy to treat said tumour; and/or
administering a therapeutically effective amount of an immunotherapy to the patient to treat said tumour.

**19.** A method for prognosis of a patient having chronic lymphocytic leukaemia (Lymph-CLL), comprising: performing the method of claim 8, wherein the test sample is a sample that has been obtained from the tumour of the Lymph-CLL patient, and wherein:

if the sample is classified as belonging to cluster M, the patient is predicted to have a longer survival time;
if the sample is classified as belonging to cluster D, the patient is predicted to have a shorter survival time.

20. A method for predicting the primary origin of a metastatic tumour in a subject, comprising performing the method of claim 8, wherein the test sample is a sample that has been obtained from the metastatic tumour of the patient, and wherein:

if the sample is classified as belonging to cluster A, the primary origin of the metastatic tumour is predicted to be lung;

if the sample is classified as belonging to cluster B, the primary origin of the metastatic tumour is predicted to be liver;

if the sample is classified as belonging to cluster C, the primary origin of the metastatic tumour is predicted to be kidney;

if the sample is classified as belonging to cluster G, the primary origin of the metastatic tumour is predicted to be skin; and

optionally, if the sample is classified as belonging to cluster H, the primary origin of the metastatic tumour may be predicted to be colorectal or uterus adenocarcinoma.

21. A method for predicting whether a tumour-burdened patient will benefit from apolipoprotein B mRNA-editing enzyme, catalytic polypeptide-like (APOBEC)-targeting therapy, comprising: performing the method of claim 8, wherein the test sample is a sample that has been obtained from the tumour of the patient, and wherein the sample is classified as belonging to cluster A or cluster E.

Figure 1

Table SF5-1: Cluster-specific overview of total and recurrent SSMs per subtype

Figure 2

| Cluster | Total number (median) | | | | Number recurrent (percentage) | | | | | | | |
|---|---|---|---|---|---|---|---|---|---|---|---|---|
| | | | | | 1 bp A/T deletion | | 1 bp C/G deletion | | 1 bp A/T insertion | | 1 bp C/G insertion | |
| | 1 bp A/T del. | 1 bp C/G del. | 1 bp A/T ins. | 1 bp C/G ins. | within cluster | entire cohort | within cluster | entire cohort | within cluster | entire cohort | within cluster | entire cohort |
| A | 21,751 (307) | 50,154 (659) | 24,171 (336.5) | 4,147 (55) | 5 (<0.1%) | 1,541 (7.1%) | 21 (<0.1%) | 317 (0.6%) | 31 (0.1%) | 2,082 (8.6%) | 0 (0%) | 102 (2.5%) |
| B | 39,653 (119.5) | 37,960 (111.5) | 52,075 (151.5) | 4,797 (14) | 44 (0.1%) | 2,145 (5.4%) | 31 (0.1%) | 254 (0.7%) | 461 (0.9%) | 7,991 (15.3%) | 21 (0.4%) | 313 (6.5%) |
| C | 40,632 (185) | 18,128 (88) | 15,411 (74) | 2,387 (11) | 6 (<0.1%) | 644 (1.6%) | 4 (<0.1%) | 65 (0.4%) | 9 (0.1%) | 637 (4.1%) | 4 (0.2%) | 32 (1.3%) |
| D | 34,652 (47) | 22,492 (30) | 24,758 (34.5) | 3,455 (4) | 81 (0.2%) | 3,663 (10.6%) | 27 (0.1%) | 236 (1.0%) | 111 (0.4%) | 4,025 (16.3%) | 13 (0.4%) | 177 (5.1%) |
| E | 8,825 (65) | 5,922 (44) | 11,037 (96.5) | 1,164 (10) | 18 (0.2%) | 1,833 (20.8%) | 5 (0.1%) | 100 (1.7%) | 23 (0.2%) | 2,052 (18.6%) | 1 (0.1%) | 119 (10.2%) |
| F | 3,966 (30) | 2,125 (19) | 4,860 (35) | 405 (4) | 4 (0.1%) | 384 (9.7%) | 2 (0.1%) | 40 (1.9%) | 4 (0.1%) | 858 (17.7%) | 5 (1.2%) | 139 (34.3%) |
| G | 26,260 (227) | 8,224 (70) | 11,473 (104) | 2,457 (20) | 26 (0.1%) | 1,010 (3.0%) | 34 (0.4%) | 112 (1.4%) | 13 (0.1%) | 1,217 (10.6%) | 5 (0.2%) | 111 (4.5%) |
| H | 30,262 (2,059) | 2,994 (232) | 50,704 (6,078) | 4,119 (406.5) | 211 (0.7%) | 6,044 (20.0%) | 11 (0.4%) | 119 (4.0%) | 348 (0.7%) | 6,122 (12.1%) | 5 (0.1%) | 84 (2.0%) |
| I | 997 (1) | 252 (0.5) | 4,887 (1.5) | 3,744 (1) | 0 (0%) | 280 (28.1%) | 0 (0%) | 8 (3.2%) | 0 (0%) | 389 (8.0%) | 0 (0%) | 18 (0.5%) |
| J | 431,323 (19,035) | 43,387 (1,362) | 172,855 (9,367) | 19,070 (1,245) | 74,137 (17.2%) | 107,563 (24.9%) | 2,588 (6.0%) | 3,783 (8.7%) | 7,731 (4.5%) | 29,421 (17.0%) | 837 (4.4%) | 2,131 (11.2%) |
| K | 34,767 (53) | 17,859 (27) | 59,677 (92) | 5,097 (8) | 220 (0.6%) | 5,883 (16.9%) | 35 (0.2%) | 370 (2.1%) | 837 (1.4%) | 11,017 (18.5%) | 46 (0.9%) | 517 (10.1%) |
| L | 58,340 (522.5) | 8,270 (77.5) | 44,788 (434) | 10,421 (72.5) | 2,409 (4.1%) | 19,939 (34.2%) | 50 (0.6%) | 399 (4.8%) | 556 (1.2%) | 9,069 (20.2%) | 46 (0.4%) | 539 (5.2%) |
| M | 51,175 (156.5) | 8,702 (30) | 47,209 (119) | 4,167 (10.5) | 1,710 (3.3%) | 17,947 (35.1%) | 24 (0.3%) | 350 (4.0%) | 567 (1.2%) | 8,933 (18.9%) | 41 (1.0%) | 495 (11.9%) |
| N | 72,981 (129) | 13,914 (35) | 96,438 (236) | 7,323 (16.0) | 3,421 (4.7%) | 26,216 (35.9%) | 102 (0.7%) | 854 (6.1%) | 2,744 (2.8%) | 19,724 (20.5%) | 165 (2.3%) | 1,010 (13.8%) |
| O | 916 (2) | 477 (2) | 1,309 (2) | 132 (0) | 6 (0.7%) | 93 (10.2%) | 1 (0.2%) | 20 (4.2%) | 10 (0.8%) | 275 (21.0%) | 2 (1.5%) | 25 (18.9%) |
| P | 26 (3) | 13 (1) | 23 (2) | 3 (0) | 0 (0%) | 7 (26.9%) | 0 (0%) | 11 (84.6%) | 0 (0%) | 6 (26.1%) | 0 (0%) | 0 (0%) |
| overall | 780,494 (88) | 238,272 (40) | 570,960 (97) | 70,067 (10) | - | 119,160 (15.3%) | - | 4,437 (1.9%) | - | 53,103 (9.3%) | - | 2,991 (4.3%) |

Table SF5-2: Cluster-specific overview of total and recurrent 1 bp SIMs per subtype

| Cluster | MEDIAN | | | | | | | | |
|---|---|---|---|---|---|---|---|---|---|
| | Perc. in late-replicating regions (ratio high vs. early) | | Percentage in CDS | | Number of genes mutated | | Perc. of synonymous SSMs from total number in CDS | Number of predicted drivers | Number of mutational signatures |
| | SSMs | SIMs | SSMs | SIMs | SSMs | SIMs | | | |
| A | 67.9% (2.1x) | 67.0% (2.0x) | 0.8% | 0.8% | 246,5 | 12 | 23.9% | 7 | 9 |
| B | 63.3% (1.7x) | 57.5% (1.4x) | 0.8% | 1.0% | 64 | 5 | 24.4% | 3 | 10 |
| C | 53.9% (1.2x) | 47.5% (0.9x) | 1.2% | 1.2% | 42 | 6 | 23.5% | 2 | 10 |
| D | 58.5% (1.4x) | 56.2% (1.3x) | 1.1% | 1.0% | 28 | 2 | 24.1% | 4 | 11 |
| E | 52.8% (1.1x) | 56.8% (1.3x) | 1.2% | 0.9% | 94 | 3 | 25.6% | 6 | 12 |
| F | 59.2% (1.5x) | 55.6% (1.2x) | 1.1% | 1.0% | 19 | 1 | 23.9% | 2 | 10 |
| G | 65.1% (1.9x) | 59.3% (1.5x) | 0.8% | 0.8% | 410 | 5 | 33.6% | 4.5 | 11 |
| H | 60.2% (1.5x) | 66.7% (2.0x) | 0.6% | 0.2% | 3,223 | 13.5 | 18.7% | 15 | 9.5 |
| I | 55.6% (1.3x) | 49.4% (1.0x) | 1.2% | 0% | 2 | 0 | 22.0% | 1 | 6 |
| J | 52.8% (1.1x) | 51.3% (1.1x) | 1.3% | 0.5% | 381 | 104 | 28.3% | 8 | 10 |
| K | 59.8% (1.5x) | 58.1% (1.4x) | 1.1% | 0.8% | 29 | 2 | 24.7% | 4 | 10 |
| L | 75.2% (3.0x) | 67.2% (2.1x) | 0.7% | 0.5% | 123 | 6 | 25.8% | 5 | 14 |
| M | 66.9% (2.0x) | 67.5% (2.1x) | 1.1% | 0.6% | 41,5 | 3 | 25.6% | 5 | 11 |
| N | 62.8% (1.7x) | 64.4% (1.8x) | 1.2% | 0.6% | 43 | 3 | 25.5% | 5 | 10 |
| O | 58.2% (1.4x) | 52.9% (1.1x) | 1.0% | 0% | 3 | 0 | 20.7% | 1 | 7 |
| P | 58.8% (1.4x) | 55.6% (1.2x) | 0.9% | 0% | 1 | 0 | 45.0% | 1 | 5 |
| overall | 61.2% (1.6x) | 58.6% (1.4x) | 1.1% | 0.8% | 42 | 3 | 25.0% | 4 | 10 |

Table SF5-3: Overview of the median values across samples for the various layers of annotation

Figure 3

| Cluster | | | Top 3 predicted drivers | | | | | |
|---|---|---|---|---|---|---|---|---|
| A | | driver | *TP53* | *CDKN2A* | *NOTCH1* | | | |
| | | perc. of samples | 73.5% | 35.3% | 22.1% | | | |
| B | | driver | *TP53* | *CTNNB1* | *ARID1A* | | | |
| | | perc. of samples | 31.2% | 23.5% | 17.6% | | | |
| C | | driver | *VHL* | *PBRM1* | *SETD2* | | | |
| | | perc. of samples | 42.6% | 28.2% | 11.3% | | | |
| D | | driver | *TP53* | *PTEN* | *19p13.3a* | | | |
| | | perc. of samples | 40% | 12.4% | 12.2% | | | |
| E | | driver | *TP53* | *PIK3CA* | *CDKN2A,* | *TERT* | | |
| | | perc. of samples | 52% | 24.5% | 18.4% | | | |
| F | | driver | *TP53* | *PTEN* | *ERG* | | | |
| | | perc. of samples | 23.2% | 16.8% | 12.6% | | | |
| G | | driver | *TERT* | *BRAF* | *CDKN2A* | | | |
| | | perc. of samples | 69% | 56.3% | 50.6% | | | |
| H | | driver | *PIK3CA, POLE* | *APC KRAS* | *ATM,* | *CTNNB1* | | |
| | | perc. of samples | 100% | 87.5% | 62.5% | | | |
| I | | driver | *BRAF* | *TP53* | *APC* | | | |
| | | perc. of samples | 50% | 25% | 18.8% | | | |
| J | | driver | *ARID1A, RPL22* | *ACVR2A* | *PIK3CA,* | *PTEN,* | *TP53* | |
| | | perc. of samples | 47.1% | 41.2% | 35.3% | | | |
| K | | driver | *TP53* | *CDKN2A* | *PTEN* | | | |
| | | perc. of samples | 33.7% | 15.7% | 13% | | | |
| L | | driver | *TP53* | *CDKN2A* | *ARID1A,* | *SMAD4* | | |
| | | perc. of samples | 75% | 31.7% | 18.3% | | | |
| M | | driver | *TP53* | *BCL2* | *CREBBP* | | | |
| | | perc. of samples | 29.3% | 23.4% | 19% | | | |
| N | | driver | *TP53* | *KRAS* | *CDKN2A* | | | |
| | | perc. of samples | 56.3% | 51.8% | 43.7% | | | |
| O | | driver | *BRAF* | *ERG* | *CDKN1B* | | | |
| | | perc. of samples | 32.6% | 18.6% | 11.6% | | | |
| P | | driver | *BRAF* | *ACO1, APTX, FANCG, LDB1, POLE, PTCH1* | | | | | |
| | | perc. of samples | 66.7% | 11.1% | | | | |
| overall | | driver | *TP53* | *CDKN2A* | *ARID1A* | | | |
| | | perc. of samples | 36.9% | 18.4% | 12.2% | | | |

**Table SF5-4: Top 3 predicted drivers per cluster**

# Figure 4

| | | | | | | | | | | |
|---|---|---|---|---|---|---|---|---|---|---|
| A | signature | SBS5 | SBS4 | SBS1 | DBS2 | DBS11 | DBS6 | ID3 | ID1 | ID2 |
| | perc. of samples | 94.1% | 89.7% | 72.1% | 92.6% | 7.4% | 7.4% | 97.1% | 86.8% | 55.9% |
| | median contribution | 41.7% | 48.0% | 0.6% | 100% | 45.8% | 30.6% | 71.9% | 11.2% | 8.0% |
| B | signature | SBS5 | SBS1 | SBS12 | DBS2 | DBS4 | DBS5 | ID1 | ID5 | ID3 |
| | perc. of samples | 100% | 67.6% | 59.3% | 96.9% | 67.0% | 8.3% | 99.7% | 96.0% | 94.4% |
| | median contribution | 66.9% | 1.0% | 19.4% | 61.0% | 41.2% | 23.7% | 21.0% | 32.6% | 25.4% |
| C | signature | SBS1 | SBS5 | SBS40 | DBS2 | DBS4 | DBS9 | ID8 | ID5 | ID9 |
| | perc. of samples | 99.5% | 94.4% | 87.7% | 88.7% | 68.2% | 56.9% | 96.4% | 95.9% | 60.0% |
| | median contribution | 5.4% | 17.5% | 74.8% | 20.8% | 23.9% | 62.2% | 16.0% | 60.3% | 10.7% |
| D | signature | SBS1 | SBS5 | SBS40 | DBS2 | DBS4 | DBS6 | ID1 | ID9 | ID8 |
| | perc. of samples | 99.8% | 97.2% | 46.6% | 68.3% | 63.5% | 37.1% | 91.4% | 70.9% | 69.1% |
| | median contribution | 8.7% | 32.8% | 47.0% | 26.1% | 29.7% | 30.0% | 13.6% | 20.7% | 21.6% |
| E | signature | SBS5 | SBS1 | SBS13 | DBS11 | DBS2 | DBS4 | ID1 | ID2 | ID8 |
| | perc. of samples | 100% | 100% | 99.0% | 75.5% | 70.4% | 62.2% | 100% | 79.6% | 70.4% |
| | median contribution | 28.3% | 4.9% | 28.7% | 50.0% | 15.5% | 23.8% | 24.6% | 12.4% | 21.4% |
| F | signature | SBS5 | SBS1 | SBS40 | DBS2 | DBS4 | DBS11 | ID1 | ID2 | ID8 |
| | perc. of samples | 100% | 100% | 41.1% | 55.8% | 47.4% | 28.4% | 96.8% | 70.5% | 66.3% |
| | median contribution | 51.2% | 12.6% | 52.3% | 35.3% | 41.7% | 46.2% | 26.1% | 9.0% | 16.5% |
| G | signature | SBS7a | SBS7b | SBS7d | DBS1 | DBS5 | DBS9 | ID1 | ID13 | ID8 |
| | perc. of samples | 90.8% | 90.8% | 90.8% | 92.0% | 1.1% | 1.1% | 93.1% | 87.4% | 87.4% |
| | median contribution | 68.4% | 19.3% | 3.1% | 100% | 100% | 100% | 10.7% | 31.6% | 22.4% |
| H | signature | SBS10a | SBS28 | SBS10b | DBS3 | DBS10 | DBS4 | ID1 | ID2 | ID5 |
| | perc. of samples | 100% | 100% | 100% | 87.5% | 50.0% | 37.5% | 100% | 100% | 12.5% |
| | median contribution | 46.8% | 20.1% | 13.3% | 67.7% | 44.6% | 9.2% | 69.8% | 25.7% | 30.4% |
| I | signature | SBS1 | SBS5 | SBS40 | DBS7 | DBS4 | DBS2 | ID2 | ID1 | ID14 |
| | perc. of samples | 100% | 81.2% | 56.2% | 31.2% | 31.2% | 25.0% | 56.2% | 50.0% | 18.8% |
| | median contribution | 23.0% | 52.3% | 57.2% | 86.2% | 6.9% | 10.9% | 21.5% | 17.4% | 72.0% |
| J | signature | SBS5 | SBS1 | SBS44 | DBS9 | DBS2 | DBS7 | ID2 | ID1 | - |
| | perc. of samples | 100% | 100% | 52.9% | 76.5% | 76.5% | 64.7% | 100% | 94.1% | - |
| | median contribution | 33.3% | 5.6% | 40.0% | 35.7% | 9.5% | 38.8% | 73.6% | 26.5% | - |
| K | signature | SBS1 | SBS5 | SBS40 | DBS2 | DBS4 | DBS6 | ID1 | ID2 | ID8 |
| | perc. of samples | 100% | 99.6% | 47.3% | 66.1% | 59.4% | 33.3% | 99.8% | 78.9% | 57.1% |
| | median contribution | 15.0% | 38.4% | 50.1% | 26.1% | 31.8% | 29.1% | 34.9% | 11.1% | 17.6% |
| L | signature | SBS1 | SBS17b | SBS17a | DBS4 | DBS2 | DBS6 | ID2 | ID1 | ID5 |
| | perc. of samples | 99.0% | 95.2% | 95.2% | 90.4% | 89.4% | 65.4% | 98.1% | 96.2% | 53.8% |
| | median contribution | 6.4% | 23.7% | 12.1% | 19.9% | 13.1% | 14.2% | 31.8% | 30.1% | 32.8% |
| M | signature | SBS1 | SBS5 | SBS40 | DBS4 | DBS9 | DBS11 | ID2 | ID1 | ID5 |
| | perc. of samples | 100% | 99.5% | 64.1% | 73.4% | 68.5% | 66.8% | 99.5% | 98.9% | 32.1% |
| | median contribution | 8.5% | 33.6% | 42.5% | 15.4% | 36.2% | 23.5% | 36.8% | 36.8% | 36.5% |
| N | signature | SBS5 | SBS1 | SBS40 | DBS4 | DBS2 | DBS9 | ID1 | ID2 | ID8 |
| | perc. of samples | 100% | 99.7% | 52.7% | 78.1% | 65.6% | 57.6% | 100% | 93.9% | 22.8% |
| | median contribution | 29.6% | 20.8% | 44.9% | 25.0% | 20.6% | 36.4% | 52.6% | 28.7% | 25.0% |
| O | signature | SBS5 | SBS1 | SBS40 | DBS2 | DBS9 | DBS4 | ID1 | ID5 | ID2 |
| | perc. of samples | 100% | 100% | 53.5% | 32.6% | 23.3% | 14.0% | 62.8% | 55.8% | 51.2% |
| | median contribution | 42.4% | 16.3% | 43.5% | 18.8% | 71.3% | 38.8% | 24.2% | 34.7% | 7.7% |
| P | signature | SBS5 | SBS1 | SBS40 | DBS2 | DBS9 | - | ID1 | ID5 | ID2 |
| | perc. of samples | 100% | 100% | 55.6% | 11.1% | 11.1% | - | 77.8% | 66.7% | 66.7% |
| | median contribution | 39.0% | 25.7% | 47.7% | 100% | 100% | - | 25.0% | 65.2% | 10.8% |
| overall | signature | SBS5 | SBS1 | SBS40 | DBS2 | DBS4 | DBS9 | ID1 | ID2 | ID8 |
| | perc. of samples | 97.8% | 93.7% | 43.9% | 69.3% | 61.4% | 34.3% | 93.1% | 69.0% | 56.8% |
| | median contribution | 35.6% | 9.7% | 48.6% | 27.0% | 28.6% | 41.2% | 25.1% | 13.4% | 17.5% |

Table SF5-5: Top 3 mutational signatures of type SBS, DBS and ID per cluster

# Figure 5

Table 1: Key characteristics of the 16 clusters

| Cluster | Main tumour type(s) | Median number of SSMs | SIMs | Num. of samples | Key characteristic(s) | Association with overall recurrence SSMs | SIMs | Possible causative agent or mechanism |
|---|---|---|---|---|---|---|---|---|
| A | Lung-SCC | 44,910 | 1,634 | 68 | • high percentage of C>A SSMs • high percentage of 1 bp C/G deletions | negative | negative | - tobacco smoke - increased activity of cytidine deaminases |
| B | Liver-HCC | 11,046.5 | 575 | 324 | • high percentage of T>C SSMs | negative | negative | alcohol intake |
| C | Kidney-RCC | 4,491 | 576 | 195 | • high percentage of 1 bp A/T insertions in no or short homopolymer context | negative | negative | 10 samples: exposure to aristolochic acid |
| D | Ovary-AdenoCA, Breast-AdenoCA, Lymph-CLL, Panc-Endocrine, Prost-AdenoCA | 3,484.5 | 189.5 | 502 | • low percentage of 1 bp A/T insertions • high percentage of 1 bp C/G deletions | negative | negative | unknown |
| E | Breast-AdenoCA, Head-SCC, Bladder-TCC | 10,426 | 362.5 | 98 | • high percentage of C>G SSMs | negative | no | increased activity of cytidine deaminases |
| F | Prost-AdenoCA, CNS-Medullo, Panc-Endocrine, Breast-AdenoCA, Thy-AdenoCA | 2,389 | 144 | 95 | • high percentage of 1 bp C/G insertions in a long homopolymer context • high percentage of recurrent 1 bp C/G insertions | no | no | unknown |
| G | Skin-Melanoma | 89,002 | 647 | 87 | • high percentage of C>T SSMs • high percentage of recurrent SSMs • high percentage of recurrent C>T SSMs | positive | negative | UV-light exposure |
| H | ColoRect-AdenoCA | 822,314 | 9,168 | 8 | • high number of SSMs | positive | no | deregulated activity of polymerase ε |
| I | CNS-PiloAstro | 125 | 5 | 16 | • high percentage of 1 bp C/G insertions | no | no | unknown[1] |
| J | Uterus-AdenoCA, Stomach-AdenoCA, ColoRect-AdenoCA | 55,789 | 30,228 | 17 | • high number of SIMs • high percentage of mutations of type SIM • high percentage of 1 bp C/G deletions in a midsize homopolymer context | no | positive | microsatellite instability |
| K | Prost-AdenoCA, CNS-Medullo, Breast-AdenoCA, Panc-AdenoCA, | 3,555.5 | 281.5 | 522 | • high percentage of 1 bp A/T insertions | no | positive | unknown |
| L | Eso-AdenoCA | 24,906 | 1,446.5 | 104 | • high percentage of T>G SSMs • high percentage of recurrent T>G SSMs | positive | positive | gastric acid (reflux) |
| M | Lymph-BNHL | 7,065 | 416.5 | 184 | • high percentage of recurrent 1 bp A/T deletions • high percentage of 1 bp A/T deletions in a long homopolymer context • high percentage recurrent C>G SSMs | positive | positive | hypermutation of the immunoglobulin genes |
| N | Panc-AdenoCA, CNS-Medullo | 4,993 | 542 | 311 | • high percentage of 1 bp A/T deletions in a long homopolymer context • high percentage of recurrent 1 bp A/T deletions • high percentage of recurrent mutations of type SIM | positive | positive | unknown |
| O | Prost-AdenoCA, CNS-PiloAstro | 182 | 11 | 43 | • high percentage of recurrent T>C, C>G & T>A SSMs | positive | positive | unknown[1] |
| P | CNS-PiloAstro | 118 | 13 | 9 | • high percentage of recurrent 1 bp C/G deletions • high percentage of 1 bp A/T deletions in a long homopolymer context | positive | positive | unknown[1] |

Figure 6

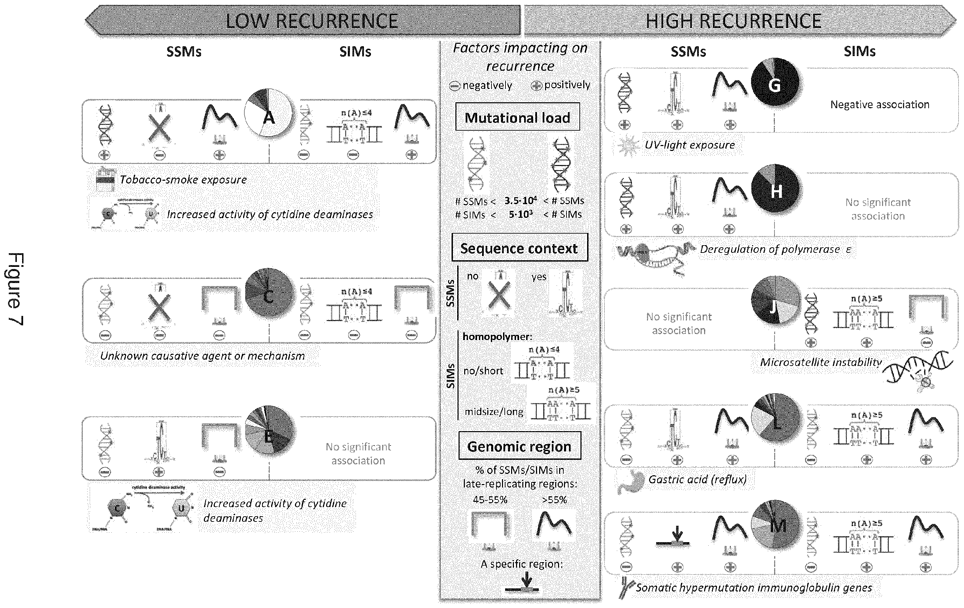

Figure 7

EP 3 739 065 A1

Figure 8

48

Hazard ratio

ckust_char    D (N=42)    reference

M (N=36)    0.26 (0.1 – 0.66)    0.004 **

# Events: 25; Global p-value (Log-Rank): < .001
AIC: 180.89; Concordance Index: 0.65

0.1    0.2    0.3    0.4    0.5    0.6

Figure 8
(cont.)

Figure 9

Europäisches
Patentamt

European
Patent Office

Office européen
des brevets

## EUROPEAN SEARCH REPORT

Application Number

EP 19 38 2385

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SERENA NIK-ZAINAL ET AL: "Landscape of somatic mutations in 560 breast cancer whole-genome sequences", NATURE, vol. 534, no. 7605, 2 May 2016 (2016-05-02), pages 47-54, XP055379838, London ISSN: 0028-0836, DOI: 10.1038/nature17676 * Methods in supplemental material * | 1-21 | INV. C12Q1/6869 C12Q1/6886 G16B20/20 G16B40/00 |
| A | LI TAI FANG ET AL: "An ensemble approach to accurately detect somatic mutations using SomaticSeq", GENOME BIOLOGY, vol. 16, no. 1, 17 September 2015 (2015-09-17), XP055531382, DOI: 10.1186/s13059-015-0758-2 * abstract * | 1-21 | |
| A | LUDMIL?B. ALEXANDROV ET AL: "Deciphering Signatures of Mutational Processes Operative in Human Cancer", CELL REPORTS, vol. 3, no. 1, 1 January 2013 (2013-01-01), pages 246-259, XP055391929, US ISSN: 2211-1247, DOI: 10.1016/j.celrep.2012.12.008 * figure 1 * | 1-21 | TECHNICAL FIELDS SEARCHED (IPC) G06F C12Q G16B |

-/--

The present search report has been drawn up for all claims

| Place of search | Date of completion of the search | Examiner |
|---|---|---|
| Munich | 21 February 2020 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 19 38 2385

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KORNELIUS SCHULZE ET AL: "Exome sequencing of hepatocellular carcinomas identifies new mutational signatures and potential therapeutic targets", NATURE GENETICS., vol. 47, no. 5, 30 March 2015 (2015-03-30), pages 505-511, XP055392299, NEW YORK, US ISSN: 1061-4036, DOI: 10.1038/ng.3252 * pages 8-10 * | 1-21 | |
| A | AINSCOUGH BENJAMIN J ET AL: "A deep learning approach to automate refinement of somatic variant calling from cancer sequencing data", NATURE GENETICS, NATURE PUBLISHING GROUP, NEW YORK, US, vol. 50, no. 12, 5 November 2018 (2018-11-05), pages 1735-1743, XP036900727, ISSN: 1061-4036, DOI: 10.1038/S41588-018-0257-Y [retrieved on 2018-11-05] * abstract * | 1-21 | TECHNICAL FIELDS SEARCHED (IPC) |

The present search report has been drawn up for all claims

| Place of search | Date of completion of the search | Examiner |
|---|---|---|
| Munich | 21 February 2020 | Hennard, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Ann Oncol.,* March 2018, vol. 29 (3), 563-572
- *Science Advances,* 07 October 2016, vol. 2 (10), e1601737
- *Mol Ther Oncolytics,* 21 December 2018, vol. 11, 1-13
- *Cell,* 2018, vol. 175 (4), 1074-87.e18
- *Nature,* 2016, vol. 532 (7598), 264-7
- **FREDRIKSSON NJ ; NY L ; NILSSON JA ; LARS-SON E.** Systematic analysis of noncoding somatic mutations and gene expression alterations across 14 tumor types. *Nat Genet,* 2014, vol. 46, 1258
- **OSTROW SL ; BARSHIR R ; DEGREGORI J ; YEGER-LOTEM E ; HERSHBERG R.** Cancer Evolution Is Associated with Pervasive Positive Selection on Globally Expressed Genes. *PLoS Genet,* 2014, vol. 10 (3), e1004239
- **SABARINATHAN R ; PICH O ; MARTINCORENA I ; RUBIO-PEREZ C ; JUUL M ; WALA J et al.** *The whole-genome panorama of cancer drivers. bioRxiv,* 2017
- **TOMASETTI C ; MARCHIONNI L ; NOWAK MA ; PARMIGIANI G ; VOGELSTEIN B.** Only three driver gene mutations are required for the development of lung and colorectal cancers. *Proc Natl Acad Sci USA,* 2015, vol. 112 (1), 118-23
- **MARTINCORENA I ; FOWLER JC ; WABIK A ; LAWSON ARJ ; ABASCAL F ; HALL MWJ et al.** Somatic mutant clones colonize the human esophagus with age. *Science,* 2018, vol. 362 (6417), 911-7
- **CICCARELLI FD.** Mutations differ in normal and cancer cells of the oesophagus. *Nature,* 2019, vol. 565 (7739), 301-3
- **WOO YH ; LI W-H.** DNA replication timing and selection shape the landscape of nucleotide variation in cancer genomes. *Nat Commun,* 2012, vol. 3, 1004
- **POLAK P ; KARLIC R ; KOREN A ; THURMAN R ; SANDSTROM R ; LAWRENCE MS et al.** Cell-of-origin chromatin organization shapes the mutational landscape of cancer. *Nature,* 2015, vol. 518 (7539), 360-4
- **ALEXANDROV LB ; NIK-ZAINAL S ; WEDGE DC ; APARICIO SAJR ; BEHJATI S ; BIANKIN AV et al.** Signatures of mutational processes in human cancer. *Nature,* 2013, vol. 500 (7463), 415-21
- **CAMPBELL PJ ; GETZ G ; STUART JM ; KORBEL JO ; STEIN LD.** *Pan-cancer analysis of whole genomes. bioRxiv,* 2017

- **DING L ; BAILEY MH ; PORTA-PARDO E ; THORSSON V ; COLAPRICO A ; BERTRAND D et al.** Perspective on Oncogenic Processes at the End of the Beginning of Cancer Genomics. *Cell,* 2018, vol. 173 (2), 305-20.e10
- **LÊ S ; JOSSE J ; HUSSON F.** FactoMineR: An R Package for Multivariate Analysis. *Journal of Statistical Software,* 2008, vol. 25 (1), 18
- **HARROW J ; FRANKISH A ; GONZALEZ JM ; TAPANARI E ; DIEKHANS M ; KOKOCINSKI F et al.** GENCODE: the reference human genome annotation for The ENCODE Project. *Genome research,* 2012, vol. 22 (9), 1760-74
- **RAMOS AH ; LICHTENSTEIN L ; GUPTA M ; LAWRENCE MS ; PUGH TJ ; SAKSENA G et al.** Oncotator: Cancer Variant Annotation Tool. *Hum Mutat,* 2015, vol. 36 (4), E2423-E9
- **RHEINBAY E ; NIELSEN MM ; ABASCAL F ; TIAO G ; HORNSHØJ H ; HESS JM et al.** *Discovery and characterization of coding and non-coding driver mutations in more than 2,500 whole cancer genomes. bioRxiv,* 2017
- **HANSEN RS ; THOMAS S ; SANDSTROM R ; CANFIELD TK ; THURMAN RE ; WEAVER M et al.** Sequencing newly replicated DNA reveals widespread plasticity in human replication timing. *Proc Natl Acad Sci USA,* 2010, vol. 107 (1), 139-44
- **ALEXANDROV L ; KIM J ; HARADHVALA NJ ; HUANG MN ; NG AWT ; BOOT A et al.** *The Repertoire of Mutational Signatures in Human Cancer. bioRxiv,* 2018, 322859
- **GUT IG ; WOOD PD ; REDMOND RW.** Interaction of Triplet Photosensitizers with Nucleotides and DNA in Aqueous Solution at Room Temperature. *J Am Chem Soc,* 1996, vol. 118 (10), 2366-73
- **FREDRIKSSON NJ ; ELLIOTT K ; FILGES S ; VAN DEN EYNDEN J ; STÅHLBERG A ; LARSSON E.** Recurrent promoter mutations in melanoma are defined by an extended context-specific mutational signature. *PLoS Genet,* 2017, vol. 13 (5), e1006773
- **SABARINATHAN R ; MULARONI L ; DEU-PONS J ; GONZALEZ-PEREZ A ; LÓPEZ-BIGAS N.** Nucleotide excision repair is impaired by binding of transcription factors to DNA. *Nature,* 2016, vol. 532 (7598), 264-7
- **ELLEGREN H.** Microsatellites: simple sequences with complex evolution. *Nat Rev Genet,* 2004, vol. 5, 435

- **SUN JX ; HELGASON A ; MASSON G ; EBENE-SERSDÓTTIR SS ; LI H ; MALLICK S et al.** A direct characterization of human mutation based on microsatellites. *Nat Genet,* 2012, vol. 44, 1161
- **SUPEK F ; LEHNER B.** Differential DNA mismatch repair underlies mutation rate variation across the human genome. *Nature,* 2015, vol. 521 (7550), 81-4
- **YANG Y ; STERLING J ; STORICI F ; RESNICK MA ; GORDENIN DA.** Hypermutability of Damaged Single-Strand DNA Formed at Double-Strand Breaks and Uncapped Telomeres in Yeast Saccharomyces cerevisiae. *PLoS Genet,* 2008, vol. 4 (11), e1000264
- **TOMKOVA M ; TOMEK J ; KRIAUCIONIS S ; SCHUSTER-BÖCKLER B.** Mutational signature distribution varies with DNA replication timing and strand asymmetry. *Genome Biology,* 2018, vol. 19 (1), 129
- **KAMIYA H.** Mutations Induced by Oxidized DNA Precursors and Their Prevention by Nucleotide Pool Sanitization Enzymes. *Genes and Environment,* 2007, vol. 29 (4), 133-40
- **SEPLYARSKIY VB ; BAZYKIN GA ; SOLDATOV RA.** Polymerase $\zeta$ Activity Is Linked to Replication Timing in Humans: Evidence from Mutational Signatures. *Molecular Biology and Evolution,* 2015, vol. 32 (12), 3158-72
- **HAMBLIN TJ ; DAVIS Z ; GARDINER A ; OSCIER DG ; STEVENSON FK.** Unmutated Ig VH Genes Are Associated With a More Aggressive Form of Chronic Lymphocytic Leukemia. *Blood,* 1999, vol. 94 (6), 1848-54
- **PFEIFER GP ; DENISSENKO MF ; OLIVIER M ; TRETYAKOVA N ; HECHT SS ; HAINAUT P.** Tobacco smoke carcinogens, DNA damage and p53 mutations in smoking-associated cancers. *Oncogene,* 2002, vol. 21, 7435
- **CHAN K ; ROBERTS SA ; KLIMCZAK LJ ; STERLING JF ; SAINI N ; MALC EP et al.** An APOBEC3A hypermutation signature is distinguishable from the signature of background mutagenesis by APOBEC3B in human cancers. *Nat Genet,* 2015, vol. 47, 1067
- **LANG GI ; MURRAY AW.** Mutation Rates across Budding Yeast Chromosome VI Are Correlated with Replication Timing. *Genome Biology and Evolution,* 2011, vol. 3, 799-811
- **XIAO Y ; FREEMAN GJ.** The Microsatellite Instable (MSI) Subset of Colorectal Cancer is a particularly good candidate for checkpoint blockade immunotherapy. *Cancer discov,* 2015, vol. 5 (1), 16-8
- **SARIDAKI Z ; SOUGLAKOS J ; GEORGOULIAS V.** Prognostic and predictive significance of MSI in stages II/III colon cancer. *World J Gastroenterol,* 2014, vol. 20 (22), 6809-14

- **NIU B ; YE K ; ZHANG Q ; LU C ; XIE M ; MCLELLAN MD et al.** MSIsensor: microsatellite instability detection using paired tumor-normal sequence data. *Bioinformatics,* 2014, vol. 30 (7), 1015-6
- **UMAR A ; BOLAND CR ; TERDIMAN JP ; SYNGAL S ; CHAPELLE ADL ; RUSCHOFF J et al.** Revised Bethesda Guidelines for Hereditary Nonpolyposis Colorectal Cancer (Lynch Syndrome) and Microsatellite Instability. *J Natl Cancer Inst,* 2004, vol. 96 (4), 261-8
- **SCHLESNER M ; EILS R.** Hypermutation takes the driver's seat. *Genome Med,* 2015, vol. 7 (1), 31
- **HEONG V ; NGOI N ; TAN DSP.** Update on immune checkpoint inhibitors in gynecological cancers. *J Gynecol Oncol,* 2017, vol. 28 (2), e20
- **PUENTE XS ; BEA S ; VALDES-MAS R ; VILLAMOR N ; GUTIERREZ-ABRIL J ; MARTIN-SUBERO JI et al.** Non-coding recurrent mutations in chronic lymphocytic leukaemia. *Nature,* 2015, vol. 526 (7574), 519-24
- **SECRIER M ; LI X ; DE SILVA N ; ELDRIDGE MD ; CONTINO G ; BORNSCHEIN J et al.** Mutational signatures in esophageal adenocarcinoma define etiologically distinct subgroups with therapeutic relevance. *Nat Genet,* 2016, vol. 48, 1131
- **WAGIH O.** ggseqlogo: a versatile R package for drawing sequence logos. *Bioinformatics,* 2017, vol. 33 (22), 3645-7
- **FAN Y ; XI L ; HUGHES DST ; ZHANG J ; ZHANG J ; FUTREAL PA et al.** MuSE: accounting for tumor heterogeneity using a sample-specific error model improves sensitivity and specificity in mutation calling from sequencing data. *Genome Biol.,* 2016, vol. 17 (1), 178
- **MONCUNILL V ; GONZALEZ S ; BEA S ; AN-DRIEUX LO ; SALAVERRIA I ; ROYO C et al.** Comprehensive characterization of complex structural variations in cancer by directly comparing genome sequence reads. *Nat Biotechnol.,* 2014, vol. 32, 1106
- **COSTELLO M ; PUGH TJ ; FENNELL TJ ; STEWART C ; LICHTENSTEIN L ; MELDRIM JC et al.** Discovery and characterization of artifactual mutations in deep coverage targeted capture sequencing data due to oxidative DNA damage during sample preparation. *Nucleic Acids Res.,* 2013, vol. 41 (6
- **WHALLEY JP ; BUCHHALTER I ; RHEINBAY E ; RAINE KM ; KLEINHEINZ K ; STOBBE MD et al.** *Framework For Quality Assessment Of Whole Genome, Cancer Sequences. bioRxiv,* 2017
- **SCHNEIDER TD ; STORMO GD ; GOLD L ; EHRENFEUCHT A.** Information content of binding sites on nucleotide sequences. *J Mol Biol.,* 1986, vol. 188 (3), 415-31
- **KULLBACK S ; LEIBLER RA.** *On Information and Sufficiency. Ann Math Statist,* 1951, vol. 22 (1), 79-86

- **PICH O ; MUIÑOS F ; SABARINATHAN R ; REYES-SALAZAR I ; GONZALEZ-PEREZ A ; LOPEZ-BIGAS N.** Somatic and Germline Mutation Periodicity Follow the Orientation of the DNA Minor Groove around Nucleosomes. *Cell,* 2018, vol. 175 (4), 1074-87.e18
- **SEGAL E ; FONDUFE-MITTENDORF Y ; CHEN L ; THÅSTRÖM A ; FIELD Y ; MOORE IK et al.** A genomic code for nucleosome positioning. *Nature,* 2006, vol. 442 (7104), 772-8
- **WASZAK SM et al.** *Germline determinants of the somatic mutation landscape in 2,642 cancer genomes. bioRxiv,* 2017
- **NIU B et al.** MSIsensor: microsatellite instability detection using paired tumor-normal sequence data. *Bioinformatics,* 2014, vol. 30 (7), 1015-6
- **PUENTE XS et al.** Non-coding recurrent mutations in chronic lymphocytic leukaemia. *Nature,* 2015, vol. 526 (7574), 519-24
- **WASZAK SM ; TIAO G ; ZHU B ; RAUSCH T ; MUYAS F ; RODRIGUEZ-MARTIN B et al.** *Germline determinants of the somatic mutation landscape in 2,642 cancer genomes. bioRxiv,* 2017
- **AVKIN S ; LIVNEH Z.** Efficiency, specificity and DNA polymerase-dependence of translesion replication across the oxidative DNA lesion 8-oxoguanine in human cells. *Mutation Research/Fundamental and Molecular Mechanisms of Mutagenesis,* 2002, vol. 510 (1-2), 81-90
- **LIU B ; XUE Q ; TANG Y ; CAO J ; GUENGERICH FP ; ZHANG H.** Mechanisms of mutagenesis: DNA replication in the presence of DNA damage. *Mutation Research/Reviews in Mutation Research,* 2016, vol. 768, 53-67
- **SALE JE ; LEHMANN AR ; WOODGATE R.** Y-family DNA polymerases and their role in tolerance of cellular DNA damage. *Nature reviews Molecular cell biology,* 2012, vol. 13 (3), 141-52
- **YU X-J ; YANG M-J ; ZHOU B ; WANG G-Z ; HUANG Y-C ; WU L-C et al.** Characterization of Somatic Mutations in Air Pollution-Related Lung Cancer. *EBioMedicine,* 2015, vol. 2 (6), 583-90
- **FUJIMOTO A ; FURUTA M ; TOTOKI Y ; TSUNODA T ; KATO M ; SHIRAISHI Y et al.** Whole-genome mutational landscape and characterization of noncoding and structural mutations in liver cancer. *Nature Genetics,* 2016, vol. 48, 500
- **TOTOKI Y ; TATSUNO K ; COVINGTON KR ; UEDA H ; CREIGHTON CJ ; KATO M et al.** Trans-ancestry mutational landscape of hepatocellular carcinoma genomes. *Nature Genetics,* 2014, vol. 46, 1267
- **FRANK A ; SEITZ HK ; BARTSCH H ; FRANK N ; NAIR J.** Immunohistochemical detection of 1,N6-ethenodeoxyadenosine in nuclei of human liver affected by diseases predisposing to hepato-carcinogenesis. *Carcinogenesis,* 2004, vol. 25 (6), 1027-31
- **SUPEK F ; LEHNER B.** Clustered Mutation Signatures Reveal that Error-Prone DNA Repair Targets Mutations to Active Genes. *Cell,* 2017, vol. 170 (3), 534-47.e23
- **SCHULZE K ; IMBEAUD S ; LETOUZÉ E ; ALEXANDROV LB ; CALDERARO J ; REBOUISSOU S et al.** Exome sequencing of hepatocellular carcinomas identifies new mutational signatures and potential therapeutic targets. *Nature Genetics,* 2015, vol. 47, 505
- **ALEXANDROV LB ; ZAINAL S ; WEDGE DC ; APARICIO SAJR ; BEHJATI S ; BIANKIN AV et al.** Signatures of mutational processes in human cancer. *Nature,* 2013, vol. 500 (7463), 415-21
- **ATTALURI S ; BONALA RR ; YANG I-Y ; LUKIN MA ; WEN Y ; GROLLMAN AP et al.** DNA adducts of aristolochic acid II: total synthesis and site-specific mutagenesis studies in mammalian cells. *Nucleic Acids Research,* 2010, vol. 38 (1), 339-52
- **SCELO G ; RIAZALHOSSEINI Y ; GREGER L ; LETOURNEAU L ; GONZALEZ-PORTA M ; WOZNIAK MB et al.** Variation in genomic landscape of clear cell renal cell carcinoma across Europe. *Nature Communications,* 2014, vol. 5, 5135
- **HASHIMOTO K ; BONALA R ; JOHNSON F ; GROLLMAN AP ; MORIYA M.** Y-family DNA polymerase-independent gap-filling translesion synthesis across aristolochic acid-derived adenine adducts in mouse cells. *DNA Repair,* 2016, vol. 46, 55-60
- **JEON Y ; BEKIRANOV S ; KARNANI N ; KAPRANOV P ; GHOSH S ; MACALPINE D et al.** Temporal profile of replication of human chromosomes. *Proceedings of the National Academy of Sciences of the United States of America,* 2005, vol. 102 (18), 6419-24
- **GNARRA JR ; TORY K ; WENG Y ; SCHMIDT L ; WEI MH ; LI H et al.** Mutations of the VHL tumour suppressor gene in renal carcinoma. *Nature Genetics,* 1994, vol. 7, 85
- **VARELA I ; TARPEY P ; RAINE K ; HUANG D ; ONG CK ; STEPHENS P et al.** Exome sequencing identifies frequent mutation of the SWI/SNF complex gene PBRM1 in renal carcinoma. *Nature,* 2011, vol. 469 (7331), 539-42
- **DALGLIESH GL ; FURGE K ; GREENMAN C ; CHEN L ; BIGNELL G ; BUTLER A et al.** Systematic sequencing of renal carcinoma reveals inactivation of histone modifying genes. *Nature,* 2010, vol. 463 (7279), 360-3
- **HOANG ML ; CHEN CH ; SIDORENKO VS ; HE J ; DICKMAN KG ; YUN BH et al.** Mutational signature of aristolochic acid exposure as revealed by whole-exome sequencing. *Science translational medicine,* 2013, vol. 5 (197), 197ra02

- **CHAN K ; ROBERTS SA ; KLIMCZAK LJ ; STERLING JF ; SAINI N ; MALC EP et al.** An APOBEC3A hypermutation signature is distinguishable from the signature of background mutagenesis by APOBEC3B in human cancers. *Nature Genetics,* 2015, vol. 47, 1067
- **DIRICAN E ; AKKIPRIK M ; ÖZER A.** Mutation distributions and clinical correlations of PIK3CA gene mutations in breast cancer. *Tumor Biology,* 2016, vol. 37 (6), 7033-45
- **NIK-ZAINAL S ; ALEXANDROV LB ; WEDGE DC ; VAN LOO P ; GREENMAN CD ; RAINE K et al.** Mutational processes molding the genomes of 21 breast cancers. *Cell,* 2012, vol. 149 (5), 979-93
- **NIK-ZAINAL S ; WEDGE DC ; ALEXANDROV LB ; PETLJAK M ; BUTLER AP ; BOLLI N et al.** Association of a germline copy number polymorphism of APOBEC3A and APOBEC3B with burden of putative APOBEC-dependent mutations in breast cancer. *Nature Genetics,* 2014, vol. 46, 487
- **HOWARD BD ; TESSMAN I.** Identification of the altered bases in mutated single-stranded DNA: II. In vivo mutagenesis by 5-bromodeoxyuridine and 2-aminopurine. *Journal of Molecular Biology,* 1964, vol. 9 (2), 364-71
- **RUSCH HP ; BAUMANN CA.** Tumor Production in Mice with Ultraviolet Irradiation. *The American Journal of Cancer,* 1939, vol. 35 (1), 55-62
- **BRASH DE.** UV Signature Mutations. *Photochemistry and Photobiology,* 2015, vol. 91 (1), 15-26
- **SHLIEN A ; CAMPBELL BB ; DE BORJA R ; ALEXANDROV LB ; MERICO D ; WEDGE D et al.** Combined hereditary and somatic mutations of replication error repair genes result in rapid onset of ultra-hypermutated cancers. *Nature Genetics,* 2015, vol. 47, 257
- **MERTZ TM ; SHARMA S ; CHABES A.** Shcherbakova PV. Colon cancer-associated mutator DNA polymerase $\delta$ variant causes expansion of dNTP pools increasing its own infidelity. *Proceedings of the National Academy of Sciences,* 2015, vol. 112 (19), E2467-E76
- **SUPEK F ; LEHNER B.** Differential DNA mismatch repair underlies mutation rate variation across the human genome. *Nature,* 2015, vol. 521 (7550), 81-4
- **FERREIRA AM ; TUOMINEN I ; DIJK-BOS K ; SANJABI B ; SLUIS T ; ZEE AG et al.** High Frequency of RPL22 Mutations in Microsatellite-Unstable Colorectal and Endometrial Tumors. *Human Mutation,* 2014, vol. 35 (12), 1442-5
- **MARUVKA YE ; MOUW KW ; KARLIC R ; PARASURAMAN P ; KAMBUROV A ; POLAK P et al.** Analysis of somatic microsatellite indels identifies driver events in human tumors. *Nature Biotechnology,* 2017, vol. 35, 951
- **DULAK AM ; STOJANOV P ; PENG S ; LAWRENCE MS ; FOX C ; STEWART C et al.** Exome and whole genome sequencing of esophageal adenocarcinoma identifies recurrent driver events and mutational complexity. *Nature genetics,* 2013, vol. 45 (5), 478-86
- **SECRIER M ; LI X ; DE SILVA N ; ELDRIDGE MD ; CONTINO G ; BORNSCHEIN J et al.** Mutational signatures in esophageal adenocarcinoma define etiologically distinct subgroups with therapeutic relevance. *Nature Genetics,* 2016, vol. 48, 1131
- **DVORAK K ; PAYNE CM ; CHAVARRIA M ; RAMSEY L ; DVORAKOVA B ; BERNSTEIN H et al.** Bile acids in combination with low pH induce oxidative stress and oxidative DNA damage: relevance to the pathogenesis of Barrett's oesophagus. *Gut,* 2007, vol. 56 (6), 763-71
- **STAMATOYANNOPOULOS JA ; ADZHUBEI I ; THURMAN RE ; KRYUKOV GV ; MIRKIN SM ; SUNYAEV SR.** Human mutation rate associated with DNA replication timing. *Nature Genetics,* 2009, vol. 41, 393
- **ZHANG W ; HONG R ; XUE L ; OU Y ; LIU X ; ZHAO Z et al.** Piccolo mediates EGFR signaling and acts as a prognostic biomarker in esophageal squamous cell carcinoma. *Oncogene,* 2017, vol. 36 (27), 3890-902
- **LEE SH ; JE EM ; YOO NJ ; LEE SH.** HMCN1, a cell polarity-related gene, is somatically mutated in gastric and colorectal cancers. *Pathology & Oncology Research,* 2015, vol. 21 (3), 847-8
- **CHONG IY ; CUNNINGHAM D ; BARBER LJ ; CAMPBELL J ; CHEN L ; KOZAREWA I et al.** The genomic landscape of oesophagogastric junctional adenocarcinoma. *The Journal of Pathology,* 2013, vol. 231 (3), 301-10
- **CHANG J ; TAN W ; LING Z ; XI R ; SHAO M ; CHEN M et al.** Genomic analysis of oesophageal squamous-cell carcinoma identifies alcohol drinking-related mutation signature and genomic alterations. *Nature Communications,* 2017, vol. 8, 15290
- **ZANOTTI KJ ; GEARHART PJ.** Antibody diversification caused by disrupted mismatch repair and promiscuous DNA polymerases. *DNA Repair,* 2016, vol. 38, 110-6
- **WILLIAMS NL ; WANG P ; WU J ; WANG Y.** In Vitro Lesion Bypass Studies of O4-Alkylthymidines with Human DNA Polymerase $\eta$. *Chemical Research in Toxicology,* 2016, vol. 29 (4), 669-75
- **TARTE K ; JOURDAN M ; VEYRUNE JL ; BERBERICH I ; FIOL G ; REDAL N et al.** The Bcl-2 family member Bfl-1/A1 is strongly repressed in normal and malignant plasma cells but is a potent anti-apoptotic factor for myeloma cells. *British Journal of Haematology,* 2004, vol. 125 (3), 373-82

- **DING L-W ; SUN Q-Y ; TAN K-T ; CHIEN W ; THIP-PESWAMY AM ; ENG JUH YEOH A et al.** Mutational Landscape of Pediatric Acute Lymphoblastic Leukemia. *Cancer Research,* 2017, vol. 77 (2), 390-400

- **NOTTA F ; CHAN-SENG-YUE M ; LEMIRE M ; LI Y ; WILSON GW ; CONNOR AA et al.** A renewed model of pancreatic cancer evolution based on genomic rearrangement patterns. *Nature,* 2016, vol. 538 (7625), 378-82